# EUROPEAN PATENT SPECIFICATION

(11) **EP 4 041 341 B1**
(45) Date of publication and mention of the grant of the patent: **30.09.2026**
(21) Application number: 20797270.4
(22) Date of filing: 01.10.2020
(51) Int. Cl.: A61M 5/142, A61M 39/22, A61M 5/168, A61M 5/172, A61M 5/145, A61M 39/26

(54) **NEEDLE INSERTION MECHANISM FOR DRUG DELIVERY DEVICE**
NADELEINFÜHRMECHANISMUS FÜR EINE ARZNEIMITTELABGABEVORRICHTUNG
MÉCANISME D'INSERTION D'AIGUILLE POUR DISPOSITIF D'ADMINISTRATION DE MÉDICAMENT

(30) Priority: 08.10.2019 US 201962912552 P; 08.07.2020 US 202063049337 P
(43) Date of publication of application: 17.08.2022
(73) Proprietor: Amgen Inc., Thousand Oaks, CA 91320-1799 (US)
(72) Inventor: PIRBODAGHI, Tohid, Thousand Oaks, CA 91320-1799 (US); TAMSKY, Joshua, Thousand Oaks, CA 91320-1799 (US); FAUCHER, Paul Daniel, Thousand Oaks, CA 91320-1799 (US); LIND, Jeff, Thousand Oaks, CA 91320-1799 (US); GIBSON, Scott Robert, Thousand Oaks, CA 91320-1799 (US); MOBERG, Sheldon B., Thousand Oaks, CA 91320-1799 (US); AKBARI, Samin, Thousand Oaks, CA 91320-1799 (US)
(74) Representative: J A Kemp LLP
(86) International application number: PCT/US2020/053788
(87) International publication number: WO 2021/071740

(56) References cited:
- EP-A1- 3 354 303
- WO-A1-2016/100781
- WO-A1-2019/070472
- WO-A1-2019/149328
- WO-A1-2019/173785
- US-A1- 2019 117 897

## Description

### FIELD OF DISCLOSURE

The present disclosure generally relates to drug delivery devices and, more particularly, to drug delivery devices having multi-stage actuation assemblies to assist in drug flow.

### BACKGROUND

Drug delivery devices, such as injectors, are used to deliver liquid drugs to a patient. Upon activation, a drug delivery device may expel a drug stored within an internal reservoir of a primary container through a needle, cannula, or other delivery member into the patient. Some drug delivery devices may be temporarily attached to a patient to deliver a drug via an injection needle or some other means over an extended period of time. The drug delivery device may be adhesively attached to the tissue of the patient's abdomen, thigh, arm, or some other portion of the patient's body. EP3354303A1, WO2019/173785A1, WO2019/149328A1, WO2016/100781A1, US2019/117897A1, and WO2019/070472A1 disclose examples of drug delivery devices and components thereof.

Occlusions of the fluid path may occur, and clots may form within the fluid path of the drug delivery device. The coagulated material may prevent the drug from being delivered when the pressure required to push the medication through the clot (or to alternatively displace the clot) exceeds the drive force capability of the device. Accordingly, the drug delivery device may stall, which can adversely impact delivery of the drug to the user, particularly with respect to delayed delivery devices. Delayed delivery devices may enhance therapeutic efficacy of certain drugs while preventing adverse side effects. Such devices may first be activated by a healthcare professional, thereby causing a needle and/or a cannula to be inserted into a patient's tissue, but may not administer the drug until after a predetermined delay.

As described in more detail below, the present disclosure sets forth systems for delivery devices embodying advantageous alternatives to existing systems, and that may address one or more of the challenges or needs mentioned herein, as well as provide other benefits and advantages.

### SUMMARY

In accordance with a first aspect there is provided a drug delivery device according to claim 1.

In some approaches, the needle insertion mechanism further includes a needle yoke and the cannula yoke. The needle yoke may be coupled with the actuation assembly and may include a needle coupling portion to receive a portion of the needle. Similarly, the cannula yoke may be coupled with the actuation assembly and may include a cannula coupling portion to receive a portion of the cannula. In some forms, the actuation assembly may include a scotch yoke assembly adapted to engage the needle yoke and/or the cannula yoke. The scotch yoke assembly may include a first spindle operably coupled with the needle yoke and the cannula yoke. The first spindle may be adapted to, in response to a needle insertion input, move at least one of the needle or the cannula to an extended position.

In some of these examples, the first spindle may include a needle insertion mechanism engagement portion having a drive pin. The needle insertion mechanism engagement portion may engage a portion of the needle yoke and/or the cannula yoke. Upon rotating the first spindle, the needle insertion mechanism engagement portion may urge the needle and/or the cannula to the extended position.

In some examples, the valve assembly may include a second spindle operably coupled with the needle yoke. In response to a valve input, the second spindle may move the needle yoke between the valve open position and the valve closed position. The second spindle may include a valve engagement portion in the form of a track. The valve engagement portion may receive a portion of the needle yoke. Upon rotating the second spindle, the valve engagement portion may urge the needle between the valve open position and the valve closed position.

In other examples, the second spindle may include a valve engagement portion in the form of a ramped surface. The valve engagement portion may receive a portion of the needle yoke. Upon rotating the second spindle, the valve engagement portion may urge the needle between the valve open position and the valve closed position.

In some examples, the needle insertion mechanism further includes a first escapement assembly and a second escapement assembly. The first escapement assembly includes a driving member, an urging member operably coupled with the driving member, and a pivotable member operably coupled with the urging member. The first escapement assembly is operably coupled with the first spindle. The second escapement assembly includes a driving member, an urging member operably coupled with the driving member, and a pivotable member operably coupled with the urging member. The second escapement assembly is operably coupled with the second spindle. In some examples, at least one of the driving members includes an electromechanical actuator that contracts upon receiving an electrical signal to compress the urging member, thereby selectively pivoting the pivotable member. In some forms, the electromechanical actuator may be in the form of a memory wire.

In some forms, the valve assembly may include a second spindle that is operably coupled with the first spindle. The second spindle may be adapted to, in response to a valve input, urge the first spindle to move the needle yoke between the valve open position and the valve closed position. The second spindle may include a second spindle gear member adapted to engage a first spindle gear member operably coupled with the first spindle. Upon rotating the second spindle, the second spindle gear member may urge the first spindle gear member, thereby rotating the first spindle to urge the needle between the valve open position and the valve closed position. In these examples, the needle insertion mechanism may further include first and second escapement assemblies operably coupled with the first and second spindles, respectively.

In other forms, the valve assembly may include a rack and pinion assembly operably coupled with the first spindle. The rack and pinion assembly may, in response to a valve input, urge the first spindle to move the needle yoke between the valve open position and the valve closed position. The rack and pinion assembly may include a pinion operably coupled with the first spindle and an actuation rack being movable in a linear direction. The valve actuation rack may have a plurality of stops formed thereon to engage the pinion. Upon linearly moving the actuation rack, at least one of the plurality of stops engages the pinion to cause the first spindle to rotate and urge the needle between the valve open position and the valve closed position. In these examples, the needle insertion mechanism may further include first and second escapement assemblies operably coupled with the first spindle and the rack and pinion assembly, respectively.

In other forms, the valve assembly may include a bi-stable spring assembly operably coupled with the needle yoke. The bi-stable spring assembly may, in response to a valve input, move the needle yoke between the valve open position and the valve closed position. In some examples, the bi-stable spring assembly may include a bi-stable spring operably coupled with the needle yoke and being movable between a first position and a second position and at least one pivotable body being operably coupled with the bi-stable spring and the needle yoke. Upon pivoting the pivotable body, the pivotable body urges the bi-stable spring between the first position and the second position, thereby moving the needle between the valve open position and the valve closed position. In these examples, the needle insertion mechanism may further include first and second escapement assemblies operably coupled with the first spindle and the bi-stable spring assembly, respectively.

In other forms, the first spindle may be adapted to, in response to a valve input, move the needle yoke between the valve open position and the valve closed position. More specifically, the first spindle may include at least one drive pin to engage the needle yoke to move at least one of the needle or the cannula to the extended position and a cam track adapted to engage the needle yoke to move the needle yoke between the valve open and the valve closed position. In these examples, the needle insertion mechanism may further include a first escapement assembly operably coupled with the first spindle to control operation thereof.

In some forms, the first spindle may include at least one drive pin that engages the needle yoke to move at least one of the needle or the cannula to the extended position, the valve open, and valve closed positions. The needle insertion mechanism may further include a first escapement assembly operably coupled with the first spindle to control operation thereof.

In other forms, the valve assembly may include a plunger displacement timing assembly operably coupled with the needle yoke. The plunger displacement timing assembly may, in response to a valve input, move the needle yoke between the valve open position and the valve closed position. The plunger displacement timing assembly may include a plunger including a tether member operably coupled therewith and a rotatable timing wheel operably coupled with the tether member and the needle yoke. Upon advancement of the plunger, the tether member causes the rotatable timing wheel to rotate, thereby moving the needle between the valve open position and the valve closed position. In these examples, the needle insertion mechanism may further include first and second escapement assemblies operably coupled with the first spindle and the plunger displacement timing assembly, respectively.

In other forms, the actuation assembly is in the form of a linear slide assembly adapted to slidingly engage the needle yoke and the cannula yoke. The linear slide assembly may include a linear slide member operably coupled with the needle yoke and a valve actuation cam. The linear slide member may, in response to a needle insertion input, move at least one of the needle or the cannula to an extended position. The valve actuation cam may, in response to a valve input, urge the linear slide member to move the needle yoke between the valve open position and the valve closed position. The linear slide member may further include a needle insertion mechanism engagement portion having a sliding track. The needle insertion mechanism engagement portion may receive a portion of the needle yoke. Upon linearly urging the linear slide member, the needle insertion mechanism engagement portion urges the needle and/or the cannula to an insertion position. In some examples, the valve actuation cam is operably coupled with the linear slide member. Upon rotating the valve actuation cam, the cam urges the linear slide member, thereby causing the needle insertion mechanism engagement portion to urge the needle between the valve open position and the valve closed position. In these examples, the linear slide assembly may further include first and second escapement assemblies operably coupled with the linear slide member and the valve actuation cam, respectively.

### BRIEF DESCRIPTION OF THE DRAWINGS

The above needs are at least partially met through provision of the needle insertion mechanism for a drug delivery device described in the following detailed description, particularly when studied in conjunction with the drawings, wherein:

The accompanying figures show embodiments according to the disclosure and are exemplary rather than limiting.
Fig. 1 illustrates a schematic representation of an example arrangement of a drug delivery device having a needle insertion mechanism in accordance with various embodiments;
Fig. 2 illustrates a schematic of an example needle insertion mechanism for the example drug delivery device of Fig. 1 in a valve closed position in accordance with various embodiments;
Fig. 3 illustrates a schematic of the example needle insertion mechanism of Fig. 2 in a valve open position in accordance with various embodiments;
Fig. 4 illustrates a first example needle insertion mechanism for the example drug delivery device of Figs. 1-3 having an integrated active valve in accordance with various embodiments;
Fig. 5 illustrates an example needle yoke for use with the example needle insertion mechanism of Fig. 4 in accordance with various embodiments;
Fig. 6 illustrates the example needle insertion mechanism of Figs. 4 and 5 in a storage (pre-activation) position in accordance with various embodiments;
Fig. 7 illustrates the example needle insertion mechanism of Figs. 4-6 upon activation in accordance with various embodiments;
Fig. 8 illustrates the example needle insertion mechanism of Figs. 4-7 in an extended position in accordance with various embodiments;
Fig. 9 illustrates the example needle insertion mechanism of Figs. 4-8 in a needle retracted, valve closed position in accordance with various embodiments;
Fig. 10 illustrates a side view of a portion of the example needle insertion mechanism of Figs. 4-9 in accordance with various embodiments;
Fig. 11 illustrates the example needle insertion mechanism of Figs. 4-10 during opening of the valve in accordance with various embodiments;
Fig. 12 illustrates the example needle insertion mechanism of Figs. 4-11 in the valve open position in accordance with various embodiments;
Fig. 13 illustrates the example needle insertion mechanism of Figs. 4-12 during closing of the valve in accordance with various embodiments;
Fig. 14 illustrates the example needle insertion mechanism of Figs. 4-13 in the valve closed position in accordance with various embodiments;
Fig. 15 illustrates a second example needle insertion mechanism for the example drug delivery device of Figs. 1-3 having an integrated, ramped active valve in accordance with various embodiments;
Fig. 16 illustrates a third example needle insertion mechanism for the example drug delivery device of Figs. 1-3 having a gear driven valve assembly in accordance with various embodiments;
Fig. 17 illustrates the example needle insertion mechanism of Fig. 16 in a storage (pre-activation) position in accordance with various embodiments;
Fig. 18 illustrates the example needle insertion mechanism of Figs. 16 and 17 upon activation in accordance with various embodiments;
Fig. 19 illustrates the example needle insertion mechanism of Figs. 16-18 in a transitory state in accordance with various embodiments;
Fig. 20 illustrates the example needle insertion mechanism of Figs. 16-19 in an extended position in accordance with various embodiments;
Fig. 21 illustrates the example needle insertion mechanism of Figs. 16-20 in a needle retracted, valve closed position in accordance with various embodiments;
Fig. 22 illustrates the example needle insertion mechanism of Figs. 16-21 in a valve opened position in accordance with various embodiments;
Fig. 23 illustrates the example needle insertion mechanism of Figs. 16-22 during closing of the valve in accordance with various embodiments;
Fig. 24 illustrates the example needle insertion mechanism of Fig. 16-23 in a valve closed position in accordance with various embodiments;
Fig. 25 illustrates the example needle insertion mechanism of Figs. 16-24 during opening of the valve assembly in accordance with various embodiments;
Fig. 26 illustrates a fourth example needle insertion mechanism for the example drug delivery device of Figs. 1-3 having a rack and pinion valve assembly in accordance with various embodiments;
Fig. 27 illustrates the example needle insertion mechanism of Fig. 26 upon activation in accordance with various embodiments;
Fig. 28 illustrates the example needle insertion mechanism of Figs. 26 and 27 in a transitory state in accordance with various embodiments;
Fig. 29 illustrates the example needle insertion mechanism of Figs. 26-28 in an extended position in accordance with various embodiments;
Fig. 30 illustrates the example needle insertion mechanism of Figs. 26-29 in in a needle retracted, valve closed position in accordance with various embodiments;
Fig. 31 illustrates the example needle insertion mechanism of Figs. 26-30 during opening of the valve in accordance with various embodiments;
Fig. 32 illustrates a side view of a portion of the example needle insertion mechanism of Figs. 26-31 upon valve opening in accordance with various embodiments;
Fig. 33 illustrates the example needle insertion mechanism of Figs. 26-32 during valve closing in accordance with various embodiments;
Fig. 34 illustrates a side view of a portion of the example needle insertion mechanism of Figs. 26-33 during valve closing in accordance with various embodiments;
Fig. 35 illustrates the example needle insertion mechanism of Figs. 26-34 in a valve closed position in accordance with various embodiments;
Fig. 36 illustrates a side view of a portion of the example needle insertion mechanism of Figs. 26-35 in the valve closed position in accordance with various embodiments;
Fig. 37 illustrates the example needle insertion mechanism of Figs. 26-36 during valve opening in accordance with various embodiments;
Fig. 38 illustrates a side view of a portion of the example needle insertion mechanism of Figs. 26-37 during valve opening accordance with various embodiments;
Fig. 39 illustrates a fifth example needle insertion mechanism for the example drug delivery device of Figs. 1-3 having a bi-stable spring mechanism valve assembly in accordance with various embodiments;
Fig. 40 illustrates a partial view of the example needle insertion mechanism of Fig. 39 in accordance with various embodiments;
Fig. 41 illustrates the example needle insertion mechanism of Fig. 39-40 upon activation in accordance with various embodiments;
Fig. 42 illustrates the example needle insertion mechanism of Figs. 39-41 in an extended position in accordance with various embodiments;
Fig. 43 illustrates the example needle insertion mechanism of Figs. 39-42 in a retracted, valve open position in accordance with various embodiments;
Fig. 44 illustrates the example needle insertion mechanism of Figs. 39-43 in a retracted, valve closed position in accordance with various embodiments;
Fig. 45 illustrates the example needle insertion mechanism of Figs. 39-44 in a retracted, valve open position in accordance with various embodiments;
Fig. 46 illustrates a sixth example needle insertion mechanism for the example drug delivery device of Figs. 1-3 having a single spindle valve assembly in accordance with various embodiments;
Fig. 47 illustrates a detail/partial view of the example spindle of the example needle insertion mechanism of Fig. 46 in accordance with various embodiments;
Fig. 48 illustrates an example needle yoke for use with the example needle insertion mechanism of Figs. 46 and 47 in accordance with various embodiments;
Fig. 49 illustrates a top view of the example needle insertion mechanism of Figs. 46-48 in accordance with various embodiments;
Fig. 50 illustrates a side view of the example needle insertion mechanism of Figs. 46-49 in accordance with various embodiments;
Fig. 51 illustrates a cross-sectional view of the example needle insertion mechanism of Figs. 46-50 in accordance with various embodiments;
Fig. 52 illustrates the example needle insertion mechanism of Figs. 46-51 upon activation in accordance with various embodiments;
Fig. 53 illustrates the example needle insertion mechanism of Figs. 46-52 during needle/cannula insertion in accordance with various embodiments;
Fig. 54 illustrates a cross-sectional view of the example needle insertion mechanism of Figs. 46-53 during needle/cannula insertion in accordance with various embodiments;
Fig. 55 illustrates a cross-sectional view of the example needle insertion mechanism of Figs. 46-54 during needle/cannula insertion in accordance with various embodiments;
Fig. 56 illustrates a cross-sectional view of the example needle insertion mechanism of Figs. 46-55 in an extended position in accordance with various embodiments;
Fig. 57 illustrates a cross-sectional view of the example needle insertion mechanism of Figs. 46-56 in a retracted, valve closed position in accordance with various embodiments;
Fig. 58 illustrates a cross-sectional view of the example needle insertion mechanism of Figs. 46-57 in a valve open position in accordance with various embodiments;
Fig. 59 illustrates a cross-sectional view of the example needle insertion mechanism of Figs. 46-58 during valve closing in accordance with various embodiments;
Fig. 60 illustrates a cross-sectional view of the example needle insertion mechanism of Figs. 46-59 in a valve closed position in accordance with various embodiments;
Fig. 61 illustrates a cross-sectional view of the example needle insertion mechanism of Figs. 46-60 during valve opening in accordance with various embodiments;
Fig. 62 illustrates a cross-sectional view of the example needle insertion mechanism of Figs. 46-61 in a valve open position in accordance with various embodiments;
Fig. 63 illustrates a cross-sectional view of the example needle insertion mechanism of Figs. 46-62 during valve closing in accordance with various embodiments;
Fig. 64 illustrates a cross-sectional view of the example needle insertion mechanism of Figs. 46-63 in a valve closed position in accordance with various embodiments;
Fig. 65 illustrates a cross-sectional view of the example needle insertion mechanism of Figs. 46-64 during valve opening in accordance with various embodiments;
Fig. 66 illustrates a cross-sectional view of the example needle insertion mechanism of Figs. 46-65 during valve opening in accordance with various embodiments;
Fig. 67 illustrates a cross-sectional view of the example needle insertion mechanism of Figs. 46-66 in a valve open position in accordance with various embodiments;
Fig. 68 illustrates a seventh example needle insertion mechanism for the example drug delivery device of Figs. 1-3 having a spring loaded spindle valve assembly in accordance with various embodiments;
Fig. 69 illustrates an eighth example needle insertion mechanism for the example drug delivery device of Figs. 1-3 having a rotational escapement valve assembly in accordance with various embodiments;
Fig. 70 illustrates the example needle insertion mechanism of Fig.69 upon insertion of the cannula and retraction of the needle yoke in accordance with various embodiments;
Fig. 71 illustrates the example needle insertion mechanism of Figs. 69 and 70 in a transitory position in accordance with various embodiments;
Fig. 72 illustrates a close up view of the example spindle of the example needle insertion mechanism of Figs.69-71 in a transitory position in accordance with various embodiments;
Fig. 73 illustrates a ninth example needle insertion mechanism for the example drug delivery device of Figs. 1-3 having a multi-rotational scotch yoke valve assembly in accordance with various embodiments;
Fig. 74 illustrates the example needle insertion mechanism of Fig. 73 upon activation in accordance with various embodiments;
Fig. 75 illustrates the example needle insertion mechanism of Figs. 73 and 74 in an extended position in accordance with various embodiments;
Fig. 76 illustrates the example needle insertion mechanism of Figs. 73-75 in a retracted, valve closed position in accordance with various embodiments;
Fig. 77 illustrates the example needle insertion mechanism of Figs. 73-76 in a first transitory position moving from a valve closed position to a valve open position in accordance with various embodiments;
Fig. 78 illustrates the example needle insertion mechanism of Figs. 73-77 in a valve open position in accordance with various embodiments;
Fig. 79 illustrates the example needle insertion mechanism of Figs. 73-78 in a first transitory position moving from a valve open to valve closed position in accordance with various embodiments;
Fig. 80 illustrates the example needle insertion mechanism of Figs. 73-79 in a second transitory position moving from a valve open to valve closed position in accordance with various embodiments;
Fig. 81 illustrates the example needle insertion mechanism of Figs. 73-80 in a valve closed state in accordance with various embodiments;
Fig. 82 illustrates a tenth example needle insertion mechanism for the example drug delivery device of Figs. 1-3 having a plunger displacement timing valve assembly in accordance with various embodiments;
Fig. 83 illustrates an example dosing timing wheel for use with the example needle insertion mechanism of Fig. 82 illustrating a valve closed position in accordance with various embodiments;
Fig. 84 illustrates the example dosing timing wheel for use with the example needle insertion mechanism of Figs. 82 and 83 illustrating a valve open position in accordance with various embodiments;
Fig. 85 illustrates an example valve actuation lifter for use with the example needle insertion mechanism of Figs. 82-84 in a lowered position in accordance with various embodiments;
Fig. 86 illustrates the example valve actuation lifter for use with the example needle insertion mechanism of Figs. 82-85 in a raised position in accordance with various embodiments;
Fig. 87 illustrates the example needle insertion mechanism of Figs. 82-86 upon activation in accordance with various embodiments;
Fig. 88 illustrates the example needle insertion mechanism of Figs. 82-87 in an insertion position in accordance with various embodiments;
Fig. 89 illustrates the example needle insertion mechanism of Figs. 82-88 in in a needle retracted, valve closed position in accordance with various embodiments;
Fig. 90 illustrates a partial cross-sectional view of the example needle insertion mechanism of Figs. 82-89 in accordance with various embodiments;
Fig. 91 illustrates the example needle insertion mechanism of Figs. 82-90 in a valve opened position in accordance with various embodiments;
Fig. 92 illustrates the example needle insertion mechanism of Figs. 82-91 during drug delivery in accordance with various embodiments;
Fig. 93 illustrates the example needle insertion mechanism of Figs. 82-92 nearing completion of drug delivery in accordance with various embodiments;
Fig. 94 illustrates the example needle insertion mechanism of Figs. 82-93 during transition to a valve closed position in accordance with various embodiments;
Fig. 95 illustrates an eleventh example needle insertion mechanism for the example drug delivery device of Figs. 1-3 having a linear slide valve assembly in accordance with various embodiments;
Fig. 96 illustrates an example linear slide and needle yoke engagement for the example needle insertion mechanism of Fig. 95 in accordance with various embodiments;
Fig. 97 illustrates the example needle insertion mechanism of Figs. 95 and 96 upon activation in accordance with various embodiments;
Fig. 98 illustrates the example needle insertion mechanism of Figs. 95-97 in an insertion position in accordance with various embodiments;
Fig. 99 illustrates the example needle insertion mechanism of Figs. 95-98 in in a needle retracted, valve closed position in accordance with various embodiments;
Fig. 100 illustrates the example needle insertion mechanism of Figs. 95-99 upon releasing a valve actuation cam in accordance with various embodiments;
Fig. 101 illustrates the example needle insertion mechanism of Figs. 95-100 during valve actuation in accordance with various embodiments;
Fig. 102 illustrates the example needle insertion mechanism of Figs. 95-101 in a valve open position in accordance with various embodiments;
Fig. 103 illustrates the example needle insertion mechanism of Figs. 95-102 in a valve closed position in accordance with various embodiments;
Fig. 104 illustrates the example needle insertion mechanism of Figs. 95-103 having an alternative cam track in accordance with various embodiments;
Fig. 105 illustrates a twelfth example needle insertion mechanism for the example drug delivery device of Figs. 1-3 having a spring-powered needle insertion mechanism and linear slide valve assembly in accordance with various embodiments;
Fig. 106 illustrates the example needle insertion mechanism of Fig. 105 during needle insertion in accordance with various embodiments;
Fig. 107 illustrates the example needle insertion mechanism of Figs. 105 and 106 in a valve closed position in accordance with various embodiments; and
Fig. 108 illustrates the example needle insertion mechanism of Figs. 95-107 in a valve open position in accordance with various embodiments.

Skilled artisans will appreciate that elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present invention. Also, common but well-understood elements that are useful or necessary in a commercially feasible embodiment are often not depicted in order to facilitate a less obstructed view of these various embodiments. It will further be appreciated that certain actions and/or steps may be described or depicted in a particular order of occurrence while those skilled in the art will understand that such specificity with respect to sequence is not actually required. It will also be understood that the terms and expressions used herein have the ordinary technical meaning as is accorded to such terms and expressions by persons skilled in the technical field as set forth above except where different specific meanings have otherwise been set forth herein.

### DETAILED DESCRIPTION

The present disclosure generally relates to a drive system for a drug delivery device that includes a needle insertion and retraction mechanism that incorporates an active valve assembly to enable time-controlled multi-dose drug delivery for various types of drug delivery devices such as, for example, on-body injectors, autoinjectors, and the like. The system may be actuated multiple times to enable drug dosing within extended periods while also functioning as a backflow prevention mechanism to prevent ingress of fluids (e.g., bodily fluids) into the drug delivery device. The valve is in fluid communication with the fluid flow connection and is movable between first and second positions. By incorporating the valve into the needle insertion mechanism, the needle insertion mechanism can selectively insert a needle and/or a cannula into the patient, close the valve to prevent the ingress and/or egress of fluids, after a pre-determined delay, open the valve to deliver the drug or medicament from the container via the fluid flow connection, and repeat these steps as needed. In some implementations, the design may also be used for single dose drug delivery.

The drug delivery devices described herein may have a delayed delivery, and as such, the needle and cannula may be inserted prior to drug delivery. Accordingly, upon insertion of the needle and/or the cannula, the needle insertion assembly moves the valve to a closed first position whereby fluid flow is restricted. At a later time, the needle insertion assembly urges the valve to a second position whereby fluid may flow through the needle and/or cannula. At yet a later time, the needle insertion assembly urges the valve to a closed position (which may be the same as the first position or a different position) and again to an open position (which may be the same as the second position or a different position). The valve is a fluid path element disposed in the fluid path that selectively restricts and permits fluid flow through the needle and/or cannula. In some examples, the cannula is inserted, yet prior to drug delivery, the valve design allows the remainder of the fluid flow path to remain sealed against the ingress of fluids (e.g., bodily fluids), thus reducing the likelihood of clogs or clots in the fluid path. Such a design is particularly advantageous for drug delivery devices having non-primed (air filled) fluid paths.

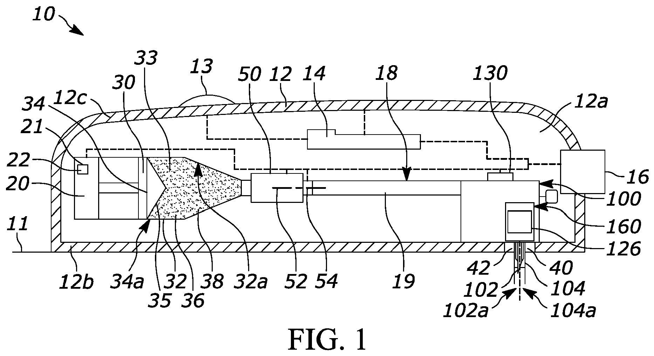

Referring to the Figures, a general drug delivery device 10 is provided that may include any number of aspects of the valve arrangement herein described. In some embodiments, including the one illustrated in Fig. 1, the drug delivery device 10 may be configured as a wearable drug delivery device, such as an on-body injector or an ambulatory infusion pump, that may be releasably coupled with a patient (e.g., to a patient's tissue 11 such as the patient's skin). In other embodiments, the drug delivery device 10 may be in the form of an autoinjector, a pen injector, or any other type of handheld devices including hybrids thereof. The drug delivery device 10 may be operated to subcutaneously or transdermally deliver a drug to a patient. The drug delivery device 10 may be configured to automatically deliver a fixed or a patient/operator-settable dose of a drug over a fixed and/or a patient/operator-settable period of time. The drug delivery device 10 may be intended for self-administration by the patient, and in some examples, may be used by a caregiver or a formally trained healthcare provider to administer an injection.

The drug delivery device 10 has a housing 12 that is releasably coupled with the patient's tissue 11 and that defines a shell and having an inner volume 12a, an activation mechanism 20, a container 30, a needle insertion mechanism 100, which incorporates an actuation assembly 130 and a valve assembly 160, each of which may be at least partially disposed within the housing 12. It is appreciated that the releasable coupling between the housing 12 and the patient's skin 11 can include any coupling or couplings that allow the drug delivery device 10 to be selectively secured to the patient, including the user holding the device 10 against the injection site, a suction force, an adhesive, or other means of holding the device 10 to the patient such as, for example, a strap, a clamp, and/or a bandage. Further, the drug delivery device may include a controller 14 and an actuator 16 (e.g., a depressible button) that is arranged on an exterior of the housing 12.

The container 30 (which, in some examples, may be referred to as a primary container) has a wall 32 that includes an interior surface 32a defining an interior volume 33 that accommodates a plunger 34. The plunger 34 is moveably disposed within the container 30 and has a first end 34a that includes an interior surface 35. The interior surface 32a of the container 30 and the interior surface 35 of the plunger 34 define a reservoir 36 that contains a drug or medicament 38.

The volume of the drug 38 contained in the reservoir 36 prior to delivery may be: any volume in a range between approximately (e.g., ±10%) 0.5 - 20 mL, or any volume in a range between approximately (e.g., ±10%) 0.5 - 10 mL, or any volume in a range between approximately (e.g., ±10%) 1 - 10 mL, or any volume in a range between approximately (e.g., ±10%) 1 - 8 mL, or any volume in a range between approximately (e.g., ±10%) 1 - 5 mL, or any volume in a range between approximately (e.g., ±10%) 1 - 3.5 mL, or any volume in a range between approximately (e.g., ±10%) 1 - 3 mL, or any volume in a range between approximately (e.g., ±10%) 1 - 2.5 mL, or any volume in a range between approximately (e.g., ±10%) 1 - 2 mL, or any volume equal to or less than approximately (e.g., ±10%) 4 mL, or any volume equal to or less than approximately (e.g., ±10%) 3.5 mL, or any volume equal to or less than approximately (e.g., ±10%) 3 mL, or any volume equal to or less than approximately (e.g., ±10%) 2.5 mL, or any volume equal to or less than approximately (e.g., ±10%) 2 mL, or any volume equal to or less than approximately (e.g., ±10%) 1.5 mL, or any volume equal to or less than approximately (e.g., ±10%) 1 mL, or any volume equal to or greater than approximately (e.g., ±10%) 2 mL, or any volume equal to or greater than approximately (e.g., ±10%) 2.5 mL, or any volume equal to or greater than approximately (e.g., ±10%) 3 mL. The reservoir may be completely or partially filled with the drug or medicament 38. The drug or medicament 38 may be one or more of the drugs listed below under the heading "Drug Information", such as, for example, a granulocyte colony-stimulating factor (G-CSF), a PCSK9 (Proprotein Convertase Subtilisin/Kexin Type 9) specific antibody, a sclerostin antibody, or a calcitonin gene-related peptide (CGRP) antibody.

The housing 12 may include a bottom wall 12b to contact or to be releasably coupled (e.g., adhered with an adhesive) with the patient's skin 11, and a top wall 12c including one or more visual feedback mechanisms 13 such as, for example a window, an opening, and/ or an illumination system (not illustrated) for viewing the container 30 and the drug or medicament 38 contained therein. The one or more visual feedback mechanisms 13 may also be used to communicate information to the user about the operational state of the drug delivery device 10 and/or the condition of the drug or medicament 38. An opening 40 may be formed in the bottom wall 12b, and optionally a pierceable sterile barrier or septum 42 may extend across the opening 40 to seal the interior 12a of the housing 12 prior to use. In some embodiments, the pierceable sterile barrier 42 may be omitted, and instead a removable sealing member (not illustrated) may cover and seal the opening 40 prior to use. The exterior of the needle insertion mechanism 100 may be defined by a housing (not illustrated) that is separate from the drug delivery device housing 12.

A fluid flow connection 18 connects the container 30, and more specifically the reservoir 36, to the needle insertion mechanism 100. The actuator 16 is configured to initiate operation of the drug delivery device 10 by activating, via mechanical and/or electrical means (shown in dotted lines in Fig. 1), the activation mechanism 20, the needle insertion mechanism 100, the controller 14, and/or other mechanisms and/or electronics. In some examples, wireless communication may be employed to cause the drug delivery device 10 to be activated. In embodiments where the actuator 16 is a button that is depressed or otherwise physically moved by a user or patient, the actuator 16 may be configured to exert a motive force and/or transmit a signal needed to activate the needle insertion mechanism 100, the fluid flow connection 18, the activation mechanism 20, the controller 14, and/or other mechanisms. In such embodiments, the actuator 16 may be physically connected to, either directly or indirectly via a mechanical linkage, the needle insertion mechanism 100, the activation mechanism 20, the fluid flow connection 18, and/or other mechanisms such that manually depressing or otherwise interacting with the actuator 16 supplies the motive force necessary to activate the needle insertion mechanism 100, the activation mechanism 20, the fluid flow connection 18, and/or other mechanisms.

The fluid flow connection 18 defines a sterile fluid flow path 19 between the container 30 and the needle insertion mechanism 100. The fluid flow connection 18 may be in the form of a flexible tube member. In some examples, a container access mechanism 50 is coupled to the fluid flow connection 18 and is configured to insert a container needle 52 through a septum 54 associated with and/or covering the container 30 to establish fluid communication between the container 30 and the sterile fluid flow path 19 in response to activation of the drug delivery device 10, for example, via the actuator 16. In the illustrated examples, relative movement between the container 30 and the container access mechanism 50 causes the container needle 52 to pierce the septum 54. In some examples, the container needle 52 may be staked to the container 30 such that the container needle 52 cannot move relative to the wall 32 of the container 30; whereas, in other examples, the container needle 52 may be moveable relative to the container 30 and may access the reservoir 36 of the container 30 by piercing through a septum or other sterile barrier covering an opening in the container 30 during operation or set up the drug delivery device 10. In some examples, the needle insertion mechanism 100 and the container 30 and/or other components such as the container access mechanism 50 may be integrated into a single unit, and thus the fluid flow connection 18 may not be included in the drug delivery device 10.

For example, in some embodiments, manually depressing or otherwise moving the actuator 16 may cause the fluid flow connection 18 and the container access mechanism 50 to move towards the container 30, or cause the container 30 to move towards the fluid flow connection 18 and the container access mechanism 50, and thereby cause the container needle 52 to penetrate through the seal member or septum 54, thereby creating a fluid flow path between the reservoir 36 and the fluid flow path 19.

Additionally, or alternatively, the actuator 16 may operate as an input device that transmits an electrical, optical, and/or mechanical signal to the controller 14, which in turn may execute programmable instructions to control operation of the needle insertion mechanism 100, the activation mechanism 20, the fluid flow connection 18, and/or other mechanisms. In such embodiments, the controller 14 may include a processor (e.g., a microprocessor) and a non-volatile memory for storing the programmable instructions to be executed by the processor. Furthermore, in such embodiments, the drug delivery device 10 may include an internal actuator (e.g., an electric motor, a pneumatic or hydraulic pump, and/or a source of pressurized gas or liquid) which is separate from the actuator 16 and which, in response to a control signal received from the controller 14, exerts the motive force needed to activate the needle insertion mechanism 100, the activation mechanism 20, the container access mechanism 50, and/or other mechanisms.

The activation mechanism 20 may include any number of components and/or sub-components to drive, urge, and/or exert a force on the plunger 34 to cause the drug or medicament 38 stored within the container 30 to be dispensed therefrom and to operate the needle insertion mechanism 100. In some examples, the activation mechanism 20 may use a drive fluid 22 in the form of a compressed CO₂ gas or other compressed gas and/or a compressed liquid to drive, urge, and/or exert the force on the plunger 34. The drive fluid 22 may initially be stored within a pressure vessel or other container 21, and the activation mechanism 20 may be configured to release the compressed gas and/or liquid from the pressure vessel or other container 21 by opening a valve (not illustrated), which allows the compressed gas and/or liquid to flow into the container 30. In other examples, the activation mechanism 20 may be in the form of a hydro-pneumatic actuation system whereby a hydraulic and/or pneumatic force is exerted on the drive fluid 22 to move the plunger 34 through the container 30 to expel the drug 38 therefrom. In other examples, the activation mechanism 20 may include any number of resilient members (e.g., springs) that exert an urging force on the plunger 34. Examples of suitable activation mechanisms 20 are described in U.S. App. No. 62/543,058, filed on Aug. 9, 2017. Other examples of suitable activation mechanisms 20 are possible.

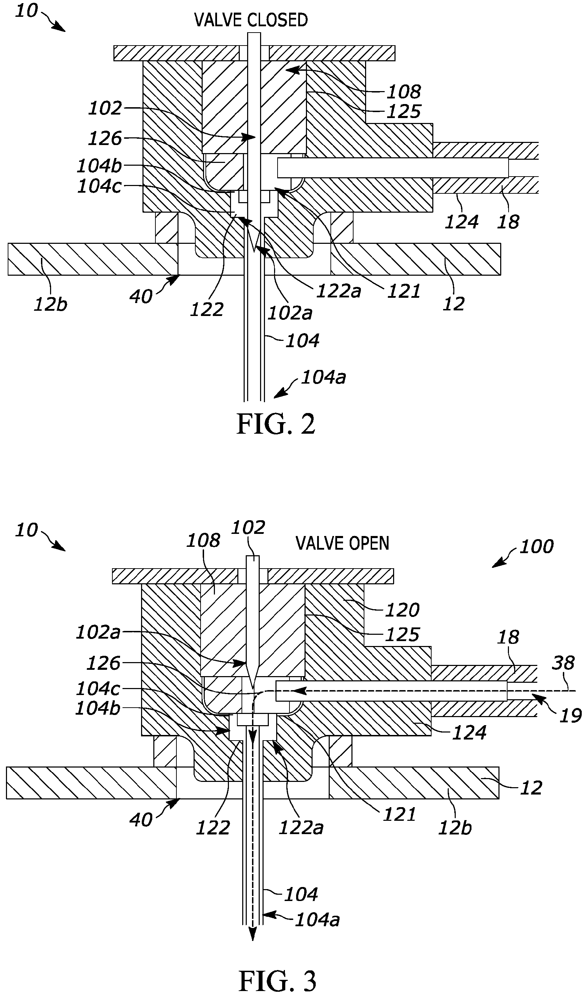

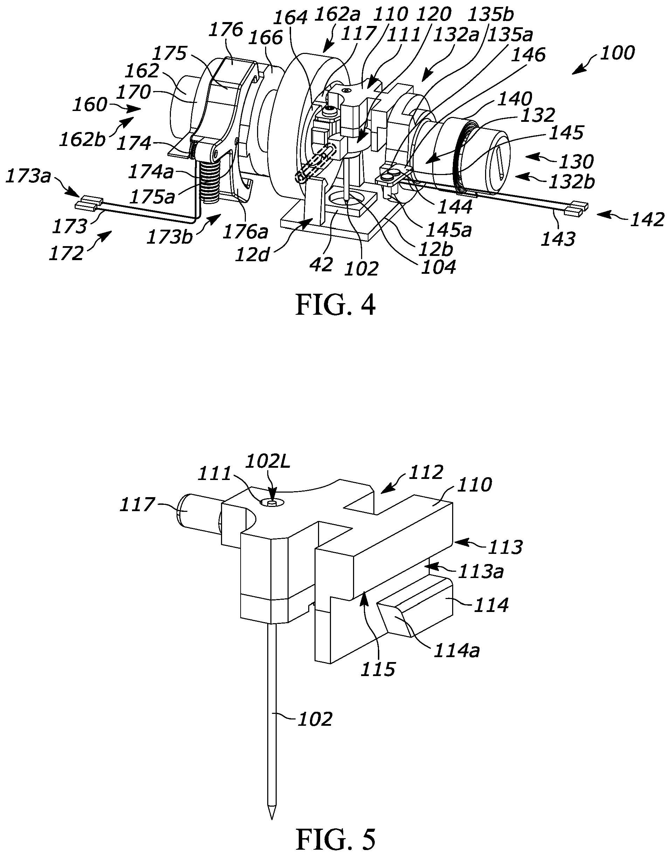

With reference generally to Figs. 2-14, the needle insertion mechanism 100 operates in multiple stages to insert a generally solid introducer needle 102 (which, in some examples, may be hollow) having a first end 102a (which, in some examples, may be closed) and a second end 102b (Fig.5) and further inserts a cannula 104 having a first end 104a and a second end 104b into the user. In some examples, by using a solid needle 102, the overall height of the needle insertion mechanism may be reduced when compared to designs using hollow needles. Such an arrangement may be advantageous in certain devices having space constraints. Generally, the needle insertion mechanism 100 includes a movable actuation assembly 130 (as will be described with reference to Figs. 4-14) that moves the needle insertion mechanism 100 between a storage state and a number of operational states such as an extended position where the needle 102 and/or the cannula 104 are inserted into a patient's tissue 11, to a first/partial needle retracted position that restricts flow of the drug 38 (as illustrated in Fig. 2), and a second/fully needle retracted position that allows flow of the drug 38 (as illustrated in Fig. 3). The needle insertion mechanism 100 further includes the valve assembly 160 that selectively permits and restricts flow of the drug 38. As will be discussed in further detail hereinafter, in some examples, the valve assembly 160 may cooperate with and/or be a subcomponent of the actuation assembly 130.

The needle 102 may be constructed of material that is rigid or flexible. In examples where the needle 102 is rigid, the needle 102 may be made of a material that is more rigid and/or harder than the cannula 104. For example, the needle 102 may be made of metal and the cannula 104 may be made of plastic or another polymer. The relative flexibility of the cannula 104 may allow it to be disposed subcutaneously within the patient's tissue 11 for a period of a time without causing pain or discomfort to the patient. In examples where the needle 102 is flexible, the needle 102 may be constructed from a super-elastic material such as nitinol, a polymer, or another material that allows the needle 102 to follow a curved path without sustaining damage. In some examples, the needle 102 may function as a trocar for creating a pathway through the patient's tissue 11 to facilitate insertion of the cannula 104.

The cannula 104 is in the form of a generally hollow member that permits fluid flow from the second end 104b to the first end 104a. The second end 104b of the cannula 104 defines an annular ledge 104c having a greater cross-sectional dimension (e.g., diameter) than the remainder of the cannula 104.

The needle insertion mechanism 100 further includes a needle yoke 110, a cannula yoke 120, and the aforementioned actuation assembly 130 and valve assembly 160. The needle yoke 110 defines a body that is operably coupled with the actuation assembly 130 and includes a needle coupling portion 111 to couple the needle 102 thereto (thereby causing the needle 102 to move with the needle yoke 110). More specifically, in the illustrated example, the needle coupling portion 111 is in the form of a hole or an opening that accepts the second end 102b of the needle 102, which may have a stopper or a plug 102c attached thereto. The needle 102 may be coupled to the needle yoke 110 via any number of approaches such as, for example, a friction fit coupling, an adhesive, a threaded coupling, and/or a fastener. For example, in examples where the needle 102 is solid, the stopper or plug 102c may be in the form of an adhesive or glue used to retain the needle 102. In examples that use a hollow needle, the fluid flow connection 18 may be coupled with the proximal end of the needle, and as such, a cap or sealing member may not be incorporated into such a design. Other examples are possible.

The cannula yoke 120 also defines a body that is also operably coupled with the actuation mechanism 130 and has a first end and a second end. The cannula yoke 120 includes a cannula yoke flow path 121 extending between the first and second ends 120a, 120b, a cannula coupling portion 122 to couple the cannula 104 thereto, a fluid flow connection coupling portion 124, and a valve coupling portion 125 in the form of a cavity or valve chamber. More specifically, in the illustrated example, the cannula coupling portion 122 is in the form of a hole or an opening that defines a ledge 122a that accepts the second end 104b of the cannula 104. In the illustrated examples, the annular ledge 104c couples with the ledge 122a of the cannula coupling portion 122. The cannula 104 may be coupled with the cannula yoke 120 via any number of approaches such as, for example, a friction fit coupling, an adhesive, a threaded coupling, via fasteners, etc.

The fluid flow connection coupling portion 124 is in the form of a hole or an opening that accepts a portion of the fluid flow connection 18. The fluid flow connection 18 may be coupled with the cannula yoke 120 via any number of approaches such as, for example, a friction fit coupling, an adhesive, a threaded coupling, and/or a fastener. Other examples are possible. The fluid flow connection 18 is positioned to define the opening of the cannula yoke flow path 121. As such, upon coupling the fluid flow connection 18 with the fluid flow connection coupling portion 124, the sterile fluid flow path 19 continues through the cannula yoke 120 and allows the drug or medicament 38 to exit at the second end 120b thereof and through the cannula 104.

The valve chamber 125 is dimensioned to receive a valve body 126 having a flow path that extends to the cannula 104. Generally speaking, the needle 102 is adapted to engage a portion of the valve body 126 to selectively block or permit flow of the drug 38 through the valve body 126 and through the cannula 104. In some examples, the valve body 126 may be integrated into the cannula yoke 120 instead of being a separate component. Further, the needle insertion mechanism 100 may include any number of additional components such as, for example, a seal ring or septum 108 to guide movement of the needle 102 and/or to provide a sealing element to restrict the drug or medicament 38 (or any other fluid) from flowing to unintended areas of the drug delivery device 10. In some examples, the second end 104b of the cannula operates as the valve body. In these examples, when the needle 102 is inserted therein, the needle 102 blocks fluid from flowing through the cannula 104.

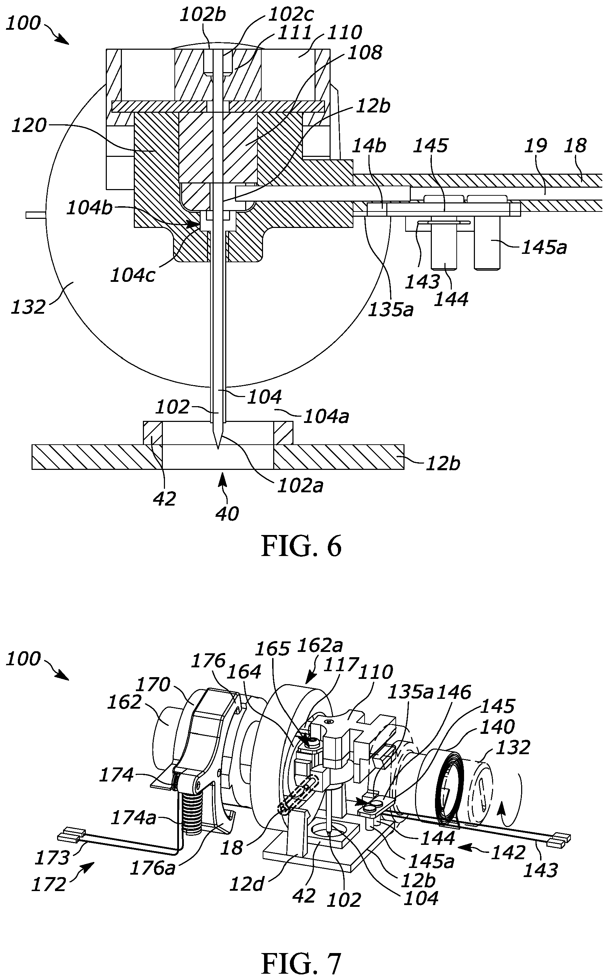

With brief reference to Fig. 6, the needle yoke 110 and the cannula yoke 120 each have a storage state where the needle 102 and the cannula 104 are retracted within the housing 12. With reference to Fig. 2, after the bottom wall 12b of the housing 12 is attached to the patient's skin 11, upon engaging the activation mechanism 20 (e.g., via the actuator 16), the drug delivery device 10 may enable, connect, or open necessary connections to establish fluid communication between the container 30 and the fluid flow connection 18. Simultaneously or subsequently, the needle insertion mechanism 100 may be activated via the activation mechanism 20 to insert the needle 102 and the cannula 104 into the patient's tissue 11. More specifically, the needle yoke 110 and the cannula yoke 120 each move to a first operational state (e.g., a lowered, extended position with respect to the illustrated orientation) which in turn causes the needle 102 and the cannula 104 to move outside of the housing 12. Put differently, the first ends 102a, 104a of the needle 102 and the cannula 104, respectively, are deployed from a retracted position to a deployed position through the opening 40 of the housing 12. In the present embodiment, this may include the needle insertion mechanism 100 inserting the needle 102 and the cannula 104 through the septum 42 and into the patient's skin 11 and subcutaneous tissue.

In the illustrated example, the second end 120b of the cannula yoke 120 is dimensioned to be inserted into the opening 40, and as illustrated in Fig. 7, a latching member 12d located on the bottom wall 12b of the housing 12 engages a portion of the cannula yoke 120 to retain and prevent the cannula yoke 120 from moving in an axial direction. Accordingly, the cannula 104 will remain in an extended position in the patient's tissue 11.

In the illustrated embodiment of Figs. 4-14, the actuation assembly 130 is in the form of a scotch yoke assembly that engages the needle yoke 110 and/or the cannula yoke 120. The actuation assembly 130 includes a first spindle 132 operably coupled with the needle yoke 110, a resilient member 140, and an actuator escapement or release mechanism 142. Similarly, in the illustrated embodiment, the valve assembly 160 includes a second spindle 162 operably coupled with the needle yoke, a second resilient member 170, and the valve escapement or release mechanism 172, the needle yoke 110, the needle 102, and the valve body 126. In some examples, the valve body 126 may be in the form of a spacer installed within the valve coupling portion 125 to reduce the "dead volume" or unprimed air space within the valve assembly. Other examples are possible such as, for example, omitting the valve body and instead shaping the cavity in the cannula yoke differently to create a smaller void.

Briefly turning to Fig. 5, the needle yoke 110 further includes a guide slot 112 to constrain the needle yoke 110 to vertical motion during operation by engaging a guide member (not illustrated), an actuation assembly engagement portion (i.e., a first spindle engagement portion 113), and a valve assembly engagement portion (i.e., second spindle engagement portion 117). The first spindle engagement portion 113 is in the form of a lower tab 114 and an upper tab 115 that cooperate to define a slot 113a. In the illustrated example, the width of the lower tab 114 is less than the width of the upper tab 115. The lower tab 114 includes an angled surface 114a to assist during assembly. As illustrated in Fig. 5, in this example, the second spindle engagement portion 117 is in the form of a pin. As will be discussed in detail below, the pin 117 generally engages the second spindle 132 to move the needle yoke 110 up and down to operate the valve. Further, in some examples, the angled surface 114a may allow the pin 134 to be "back driven" while loading the needle yoke 110 into the assembly.

The first spindle 132 is rotatably mounted within the housing 12 using any number of suitable approaches and includes a first end 132a and second end 132b. Positioned along a length of the first spindle 132 is the resilient member 140, which, in the illustrated examples, is in the form of a watch spring. The resilient member 140 serves to exert an urging force on the first spindle 132 that causes its rotation. In other examples, the resilient member 140 may take alternate forms such as, for example a torsion spring. Other examples are possible.

The first end 132a of the first spindle 132 includes a needle insertion mechanism engagement portion in the form of a drive pin 134 that slidably engages the first spindle engagement portion 113 of the needle yoke 110. More specifically, the drive pin 134 is configured to be inserted into the slot 113a and engage the lower tab 114 and the upper tab 115. The first end 132a of the spindle 132 also includes an actuation escapement assembly engagement portion in the form of a first catch 135a and a second catch 135b. Generally speaking, each of the drive pin 134 and the actuation escapement assembly engagement portion are configured to rotate with the spindle 132, and as such, the components coupled thereto (or features formed thereon) are also configured to rotate.

As previously noted, the actuation assembly 130 includes the actuator escapement assembly 142. The actuator escapement assembly 142 may be mounted to the needle insertion mechanism 100 and may include a driving member 143, an urging member 144 operably coupled with the driving member 143, and a pivotable member 145 having one or more pivot pins 145a and that may be operably coupled with the urging member 144.

The driving member 143 is in the form of an electromechanical actuator that includes a first end and a second end coupled with the urging member 144 using any suitable fastening method. In some examples, the driving member 143 is operably coupled to the actuator 16 and/or the controller 14 via an electrical connection. More specifically, the driving member 143 may be in the form of a muscle wire constructed from nickel-titanium (or a similar) shape-memory alloy that is selectively energized or de-energized during operation of the needle insertion mechanism 100. In other examples, the driving member 143 may be in the form of an electromechanical polymer that changes in length in response to an applied electrical current and/or change in temperature. When energized (for example, via a battery), the driving member 143 is adapted to contract, thereby reducing its overall length. The driving member 143 may use any type of coupling such as crimp connectors, plugs, or other contacts to receive the electrical signal from the actuator 16 and/or the controller 14.

In the illustrated examples, the urging member 144 is in the form of a pin coupled with the pivotable member 145. In other examples, the urging member may take other forms such as a spring. Other examples are possible.

The pivotable member 145 further includes an engaging portion in the form of a platform 146. Generally, the pivotable member 145 pivots about the pivot pin 145a to selectively position the platform 146 in a first position (Fig. 4) and a second position (Fig. 7). When the driving member 143 is energized, the driving member 143 contracts, thereby pulling the urging member 144 and moving the pivotable member 145 to the second position.

As previously noted, the valve assembly 160 is in the form of a second spindle 162 operably coupled with the needle yoke 110 and includes the resilient member 170 and valve escapement assembly 172. The second spindle 162 is adapted to, in response to a valve input, move the needle yoke 110 between the valve open and valve closed positions. Like the first spindle 132, the second spindle 162 is rotatably mounted within the housing 12 using any number of suitable approaches and includes a first end 162a and a second end 162b. Positioned along a length of the second spindle 162 is the resilient member 170, which, in the illustrated examples, is in the form of a watch spring. The resilient member 170 serves to exert an urging force on the second spindle 162 that causes its rotation. In other examples, the resilient member 170 may take alternate forms such as, for example a torsion spring. Other examples are possible.

The first end 162a of the second spindle 162 includes a needle insertion mechanism engagement portion in the form of a track 164 that slidably engages the second spindle engagement portion (i.e., the pin 117) of the needle yoke 110. More specifically, the pin 117 is configured to be inserted into the track 164 to control movement of the needle yoke 110. The track 164 has a generally ovoid shape such that, upon rotation of the second spindle 162, the vertical position of the needle yoke 110 (and thus the needle 102) varies. The needle insertion mechanism engagement portion further includes an elongated slot 165.

The second end 162b of the spindle 162 also includes a valve escapement assembly engagement portion in the form of a number of engagement surfaces or catches 166. Generally speaking, each of the needle insertion mechanism engagement portion and the valve escapement assembly engagement portion are configured to rotate with the spindle 162, and as such, the components coupled thereto (or features formed thereon) are also configured to rotate.

The valve escapement assembly 172 may be mounted to the needle insertion mechanism 100 and may include a driving member 173, an urging member 174 operably coupled with the driving member 173, and a pivotable member 175 having one or more pivot pins 175a and that may be operably coupled with the urging member 174.

Like the actuator escapement assembly 142, the driving member 173 is in the form of a linear actuator that includes a first end 173a and a second end 173b coupled with the urging member 174 using any suitable fastening method. In some examples, the driving member 173 is operably coupled to the actuator 16 and/or the controller 14 via an electrical connection. More specifically, the driving member 173 may be in the form of a muscle wire constructed from nickel-titanium (or a similar) shape-memory alloy that is selectively energized or de-energized during operation of the needle insertion mechanism 100. In other examples, the driving member 173 may be in the form of an electromechanical polymer that changes in length in response to an applied electrical current and/or change in temperature. When energized (for example, via a battery), the driving member 173 is adapted to contract, thereby reducing its overall length. The driving member 173 may use any type of coupling such as crimp connectors, plugs, or other contacts to receive the electrical signal from the actuator 16 and/or the controller 14.

In the illustrated examples, the urging member 174 is in the form of a pin coupled with the pivotable member 175. The valve escapement assembly 172 further includes a resilient member 174a (in the form of a compression spring in this example).

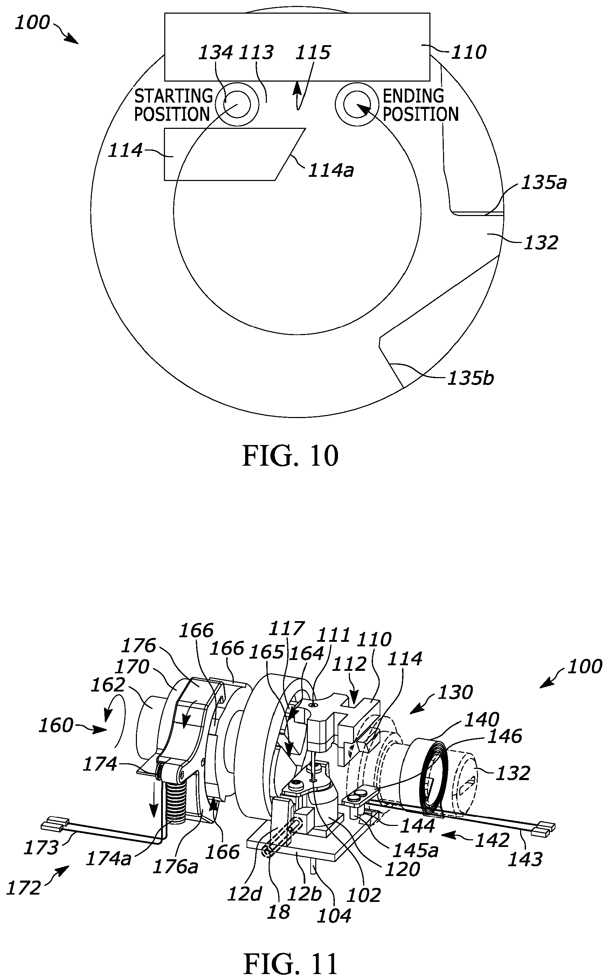

The pivotable member 175 further includes an engaging portion in the form of a first finger 176 and a second finger 176a. Generally, the pivotable member 175 pivots about the pivot pin 175a to selectively move the fingers 176, 176a between a first position (Fig. 4) and a second position (Fig. 11). When the driving member 173 is energized, the driving member 173 contracts, thereby pulling the urging member 174 and moving the pivotable member 175 to the second position. When the driving member 173 is not energized, the resilient member 174a urges the urging member 174 to pivot about the pivot pin 175a to the first position.

As previously noted, in Figs. 4 and 6, the needle insertion mechanism 100 is positioned in a storage state whereby the needle yoke 110 and the cannula yoke 120 are both in raised, retracted positions. In this configuration, the platform 146 of the pivotable member 145 engages the first catch 135a of the actuation escapement mechanism engagement portion of the first spindle 132. Accordingly, the platform 146 prevents the first spindle 132 from rotating via the urging force exerted thereupon by the resilient member 140. With reference to Fig. 7, upon engaging the activation mechanism 20, the drive member 143 becomes energized and urges the urging member 144 to cause the pivotable member 145 to pivot and thus the platform 146 to move to the second position, thereby disengaging from the first catch 135a. The resilient member 140 then causes first spindle 132 to rotate, which in turn causes the drive pin 134 to rotate, thus engaging the lower tab 114 of the needle yoke 100. As illustrated in Fig. 10, this movement urges the needle yoke 110 and thus the cannula yoke 120 to a lowered, extended position.

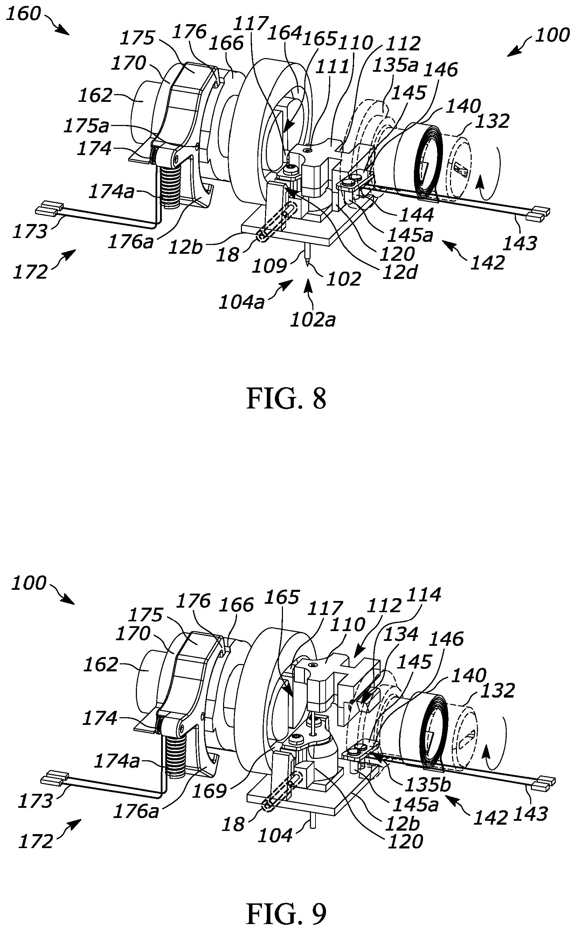

With reference to Fig. 8, the needle 102 and the cannula 104 are fully extended below the bottom wall 12b of the device 10 (illustrated in Fig. 1). Notably, the second spindle 162 provides clearance to allow the needle yoke 110 to move to the lowered position. More specifically, the pin 117 of the needle yoke 110 travels through the elongated slot 165 of the needle insertion mechanism engagement portion of the second spindle 162 when the needle yoke 110 moves to the lowered position. As previously noted, the latching member 12d located on the bottom wall 12b of the housing 12 engages a portion of the cannula yoke 120 to retain and prevent the cannula yoke 120 (and thus the cannula 104) from moving in an axial direction.

With reference to Figs. 9 and 10, the first spindle 132 continues to rotate until the second catch 135b of the actuation escapement engagement portion engages the platform 146 of the pivotable member 145, which is now positioned in the second position. The continued rotation of the spindle 132 causes the needle yoke 110 to move beyond the fully lowered, extended position back to a second, retracted operational state. In other words, when the second catch 135b engages the platform 146, the drive pin 134 is in a raised ending position (see Fig. 10), thereby automatically retracting the needle yoke 110 (and thus the needle 102) while leaving the first end 104a of the distal open end of the cannula 104 inside the patient for subcutaneous delivery of the drug or medicament 38. As also illustrated in Fig. 9, the pin 117 again travels through the elongated slot 165 of the needle insertion mechanism engagement portion of the second spindle 162 to an upper position where the pin 117 is disposed in the track 164. In this position, as illustrated in Fig. 2, the valve assembly 160 is closed where the needle 102 remains in the top of the cannula 104 (and/or engages the valve body 126) to retain a seal. In some examples, however, it may be desired to immediately deliver the first aliquot, and as such, the needle insertion mechanism may be arranged to allow the needle 102 to move directly to the valve open position after cannula 104 insertion.

As illustrated in Fig. 10, the total rotation of the first spindle 132 is less than 360° to enable the needle yoke 110 to be fully retained by the drive pin 134 in the starting position and to allow the needle yoke 110 to freely translate upwardly therefrom in the ending position. In the illustrated example, the starting and ending positions may be symmetric such that the needle yoke 110 starts and ends at the same vertical position. Such a configuration advantageously allows a physical stop to be placed closely above the needle yoke 110 to constrain it from translating higher than when in the valve closed position.

A delay prior to delivery of the drug 38 may be implemented if desired and/or necessary for the specific drug or medicament 38 being administered. During this delay period, the system remains in the flow restricting (valve closed) state. Because the valve assembly 160 is closed, blood and/or other bodily fluids are restricted from entering the sterile fluid flow path 19, thereby reducing a likelihood of clogs and/or clots in the sterile fluid flow path 19.

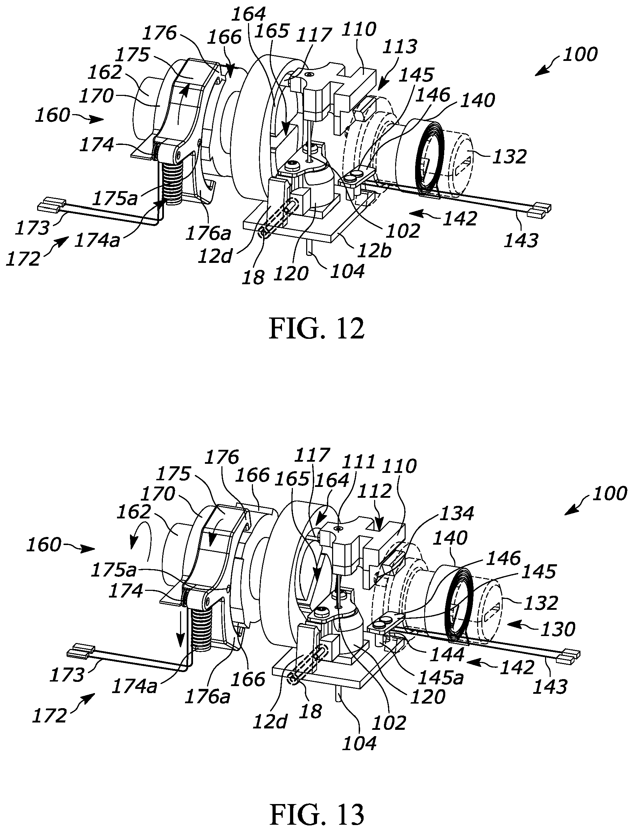

With reference to Figs. 11-14, when administration of the drug or medicament 38 is desired, the actuator 16 and/or the controller 14 transmits an electrical signal to the valve assembly 160, and specifically the driving member 173. The valve assembly moves the needle yoke 110 vertically (relative to the cannula yoke 120) between the valve open and valve closed positions. As previously noted and as illustrated in Fig. 13, the signal causes the driving member 173 to energize and contract, thereby urging the urging member downward to cause the pivotable member 175 to pivot about the pivot pin 175a. Such pivoting causes the first finger 176 to be disengaged from one of the catches 166, whereupon the resilient member 170 causes the second spindle 162 to rotate. This rotation causes the pin 117, which is constrained within the track 164, to move vertically upon traversing the track 164.

As illustrated in Fig. 12, the second spindle 162 is permitted to rotate in approximately 90° intervals due to placement of the catches 166. More specifically, the release of the first finger 176 causes the second finger 176a to engage a catch 166 which temporarily halts the rotation of the second spindle 162. When de-energized, the driving member 173 lengthens and the compression spring 174a urges and returns the pivotable member 175 to the first position. As such, the second finger 176a disengages the catch 166 and the first finger 176 in turn engages the next catch 166. Accordingly, the second spindle 162 undergoes approximately 90° of rotation upon disengaging and re-engaging the first finger 176. In some examples, the 90° rotational increment used to move the needle yoke 110 during valve actuation may instead be 60° for use with a tri-lobed cam design. Other examples are possible.

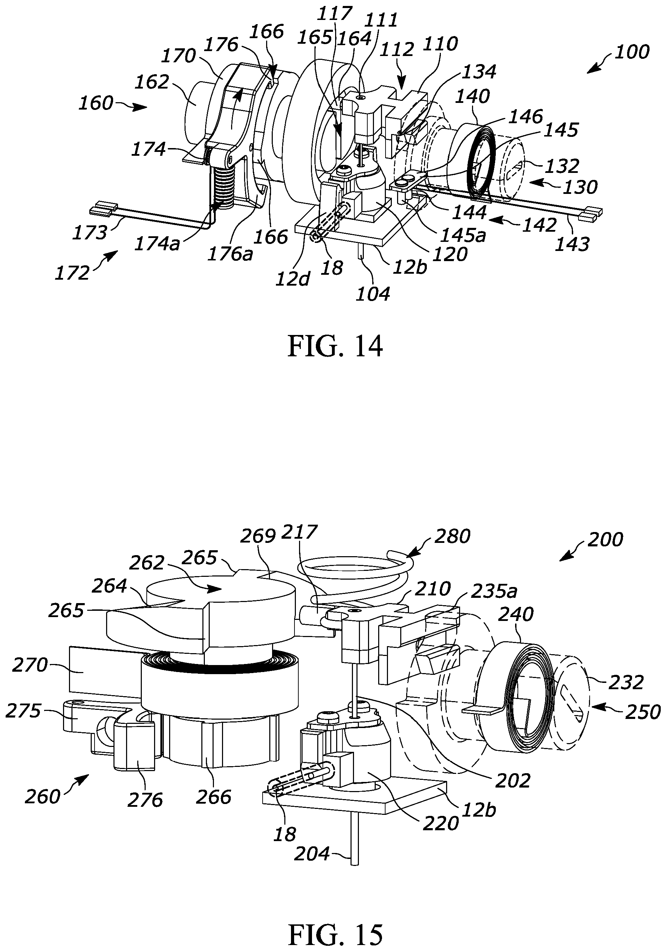

As illustrated in Figs. 3 and 12, because of the ovoid shape of the track 164, in this position, the needle yoke 110 and thus the needle 110 are disposed in a retracted, valve open position that permits all or a portion of the drug 38 to be administered. As illustrated in Figs. 13 and 14, the valve assembly 160 may close the valve by again engaging the valve escapement assembly 172 to cause the pivotable member 175 to pivot to disengage and re-engage the first finger 176 from and with the catch 166. This disengagement causes the second spindle 162 to again rotate, thereby causing the pin 117 to move through the track 164. As illustrated in Fig. 14, upon reengaging the first finger 176, the second spindle 162 causes the needle yoke 110 to be positioned in the valve closed position (Fig. 2) that restricts fluid flow. The valve assembly 160 may again be engaged to allow for additional drug dosings.

In some examples, the slide valve arrangement may be provided with different configurations. For example, the needle 102 may be generally hollow and may include a side port that can be sealed inside the valve body 126.

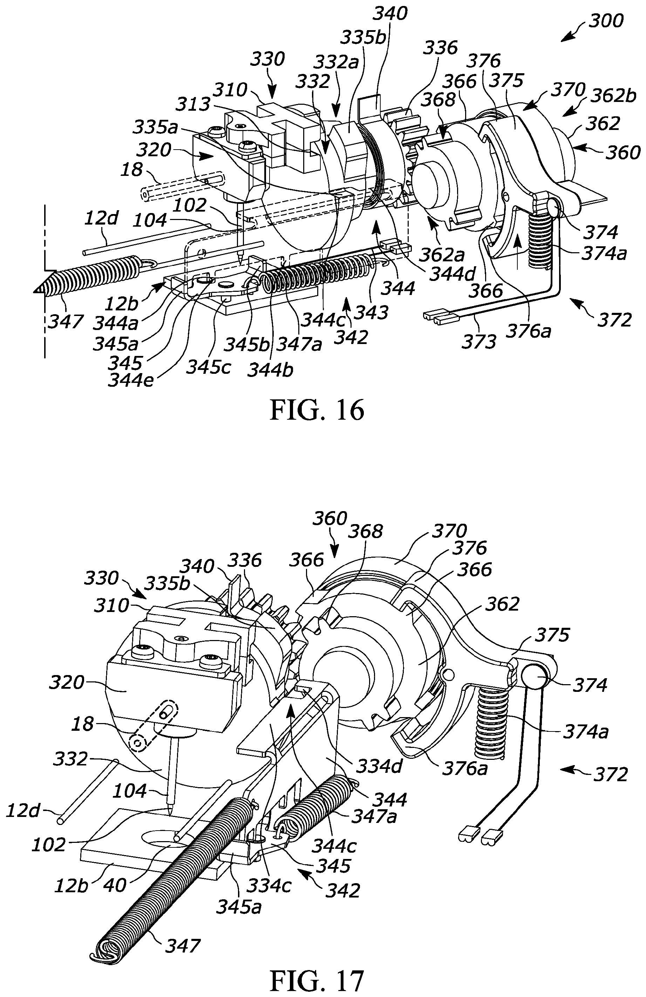

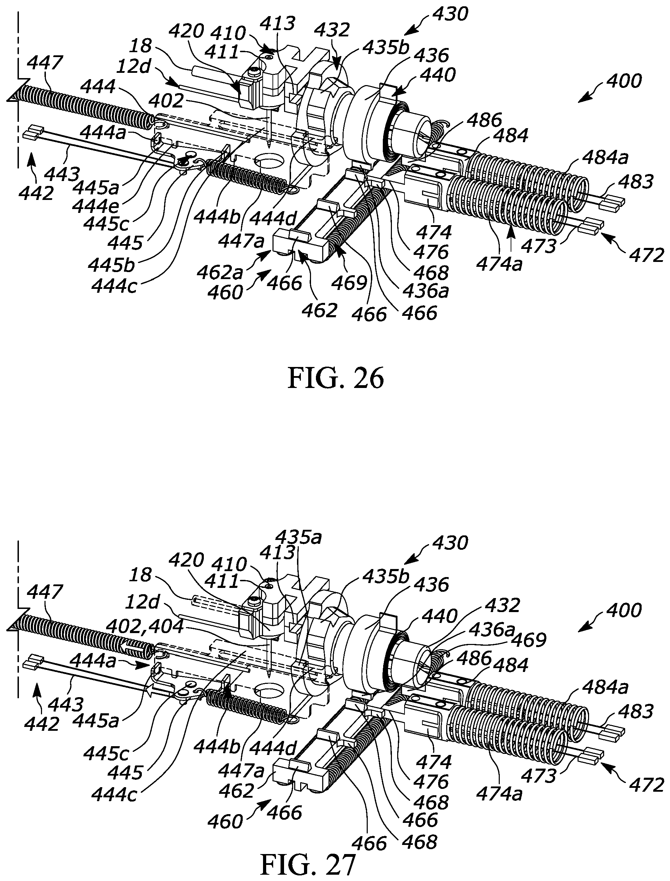

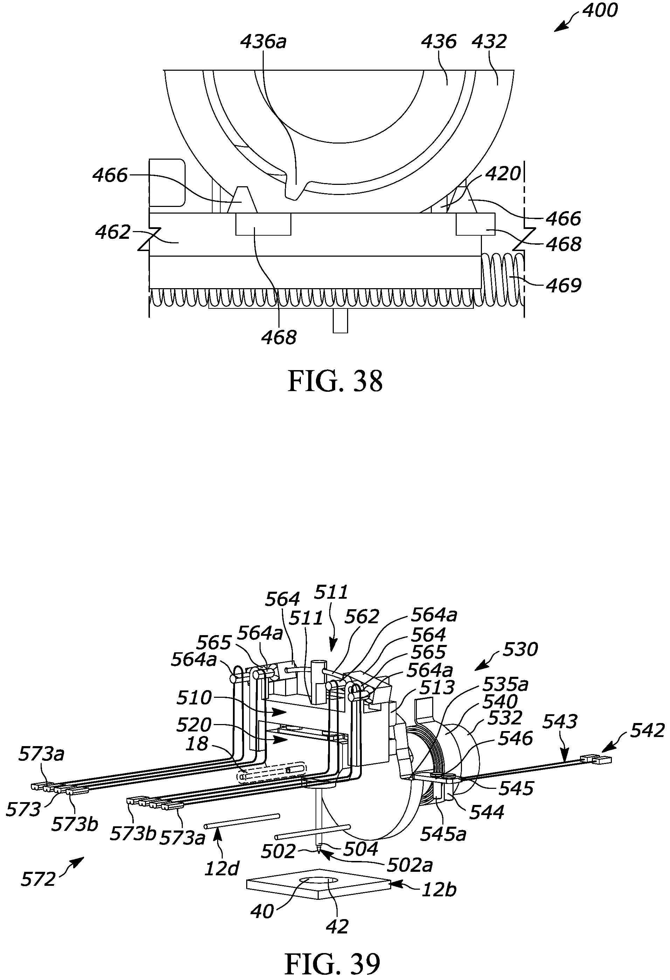

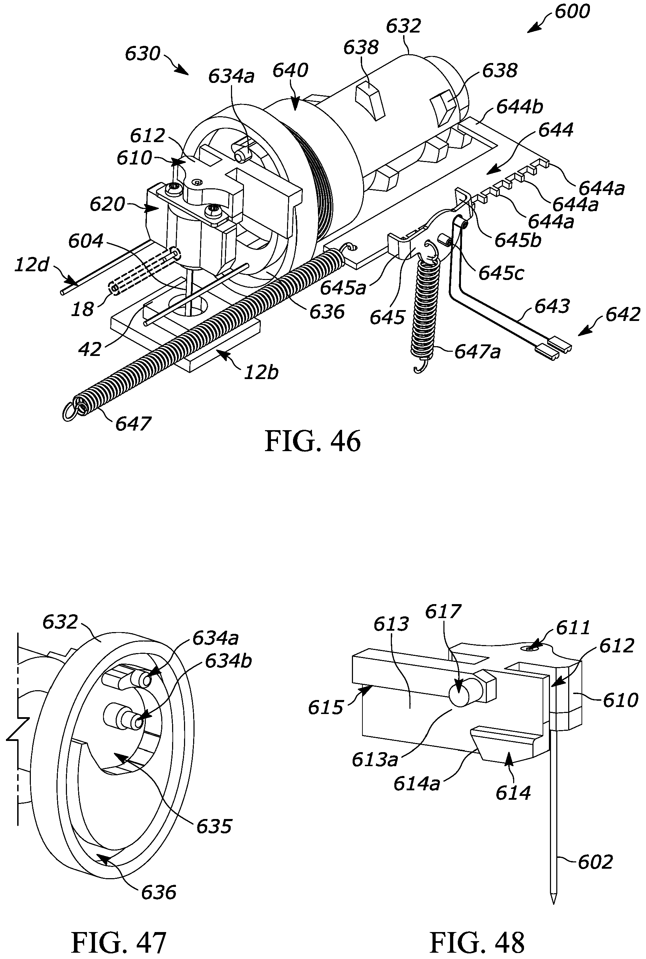

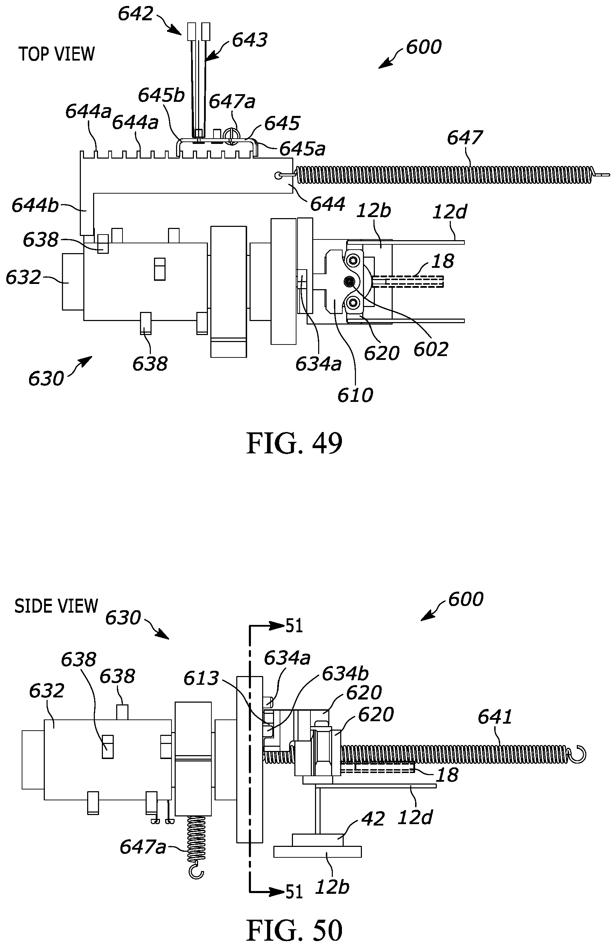

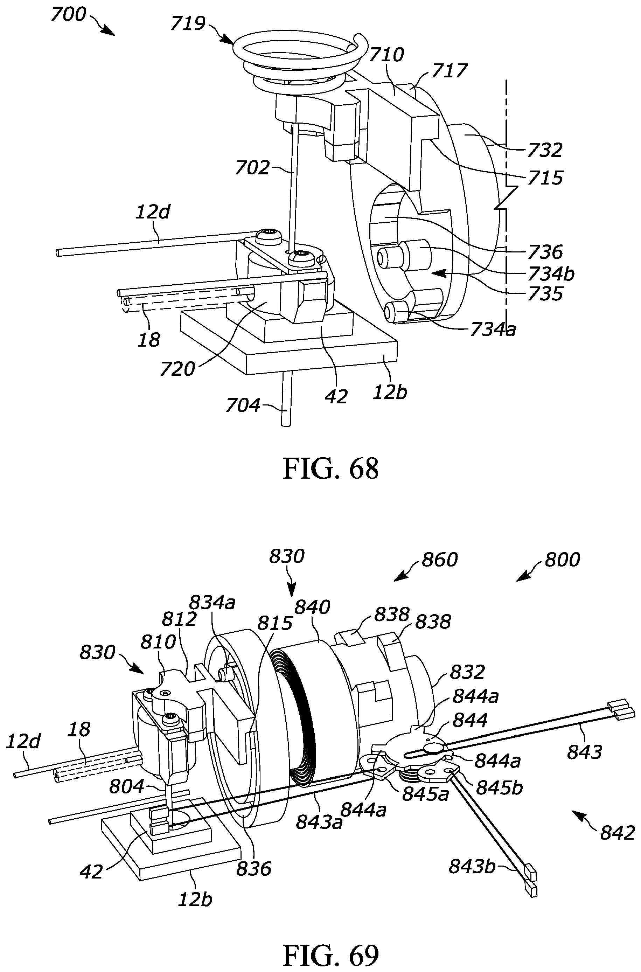

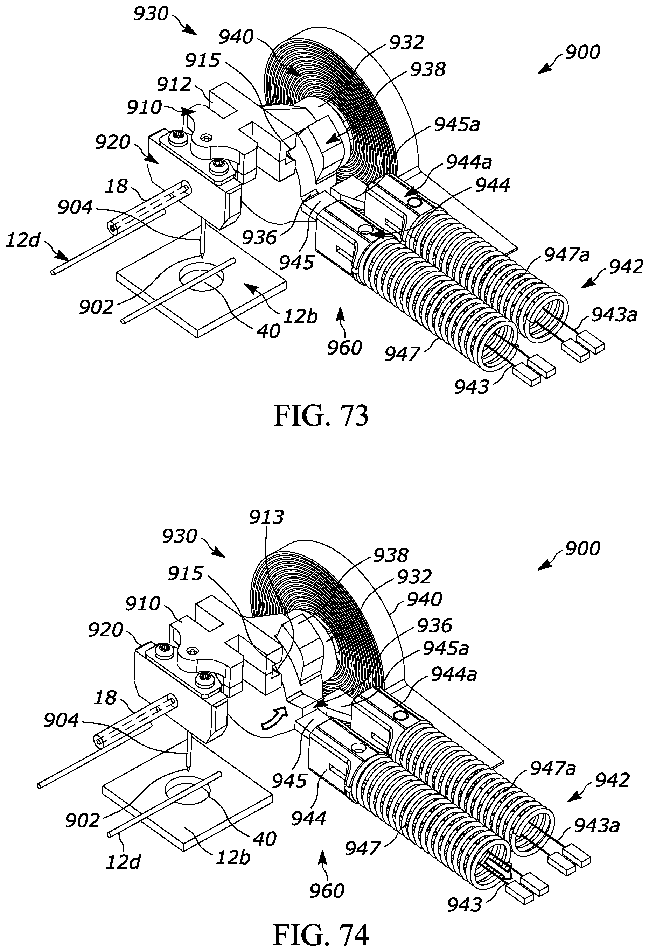

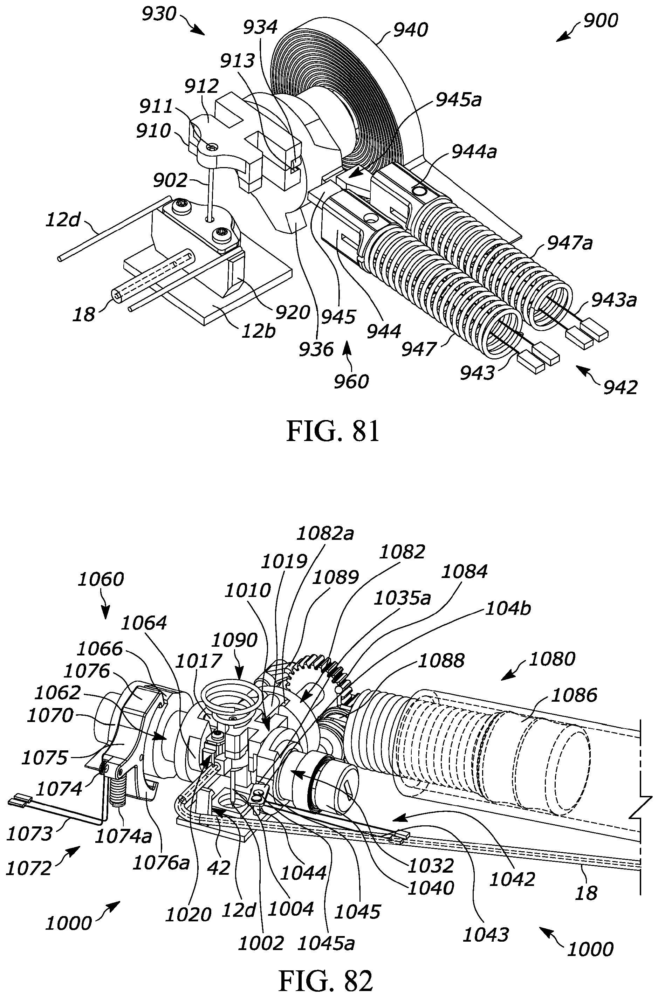

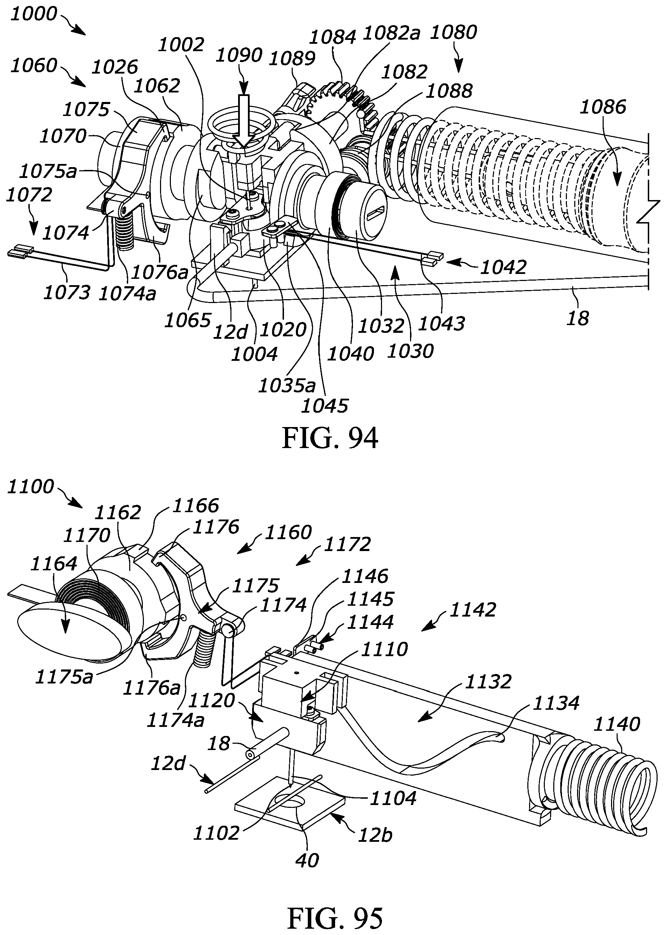

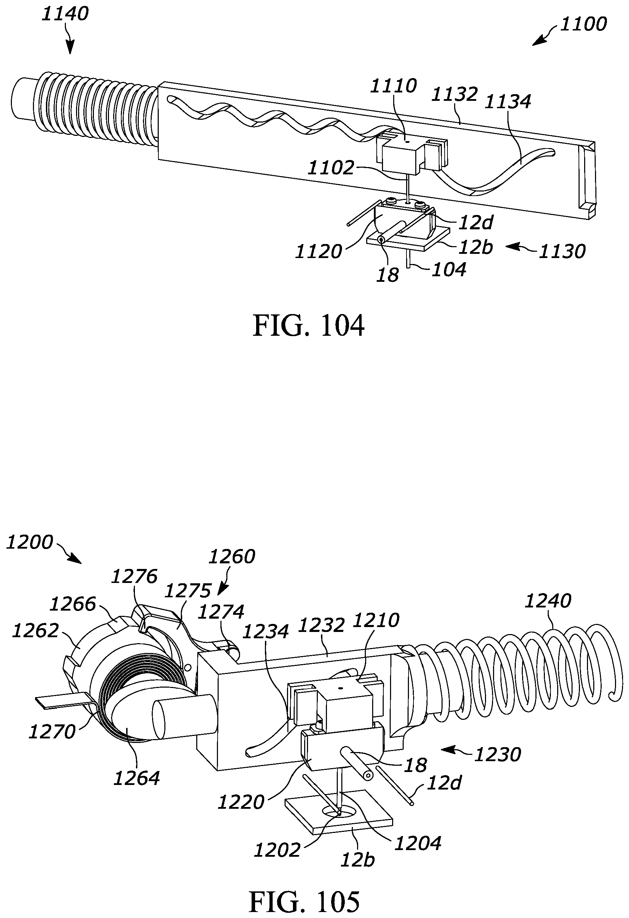

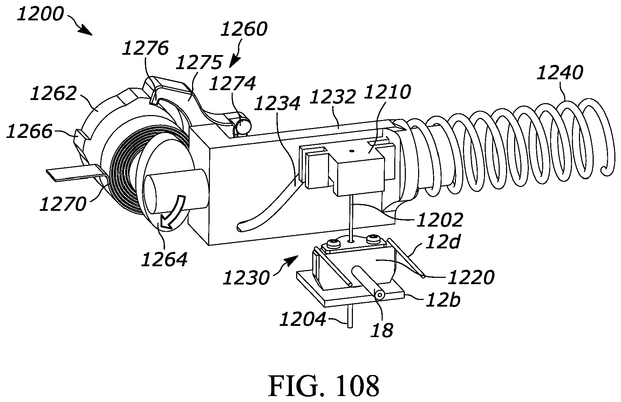

The needle insertion mechanism described herein may be provided in any number of alternative designs. For example, Fig. 15 illustrates a second example needle insertion mechanism 200 for use with a drug delivery device 10 having an integrated, ramped active valve, Figs. 16-25 illustrate a third example needle insertion mechanism 300 for use with a drug delivery device 10 having a gear driven valve assembly, Figs. 26-38 illustrate a fourth example needle insertion mechanism 400 for use with a drug delivery device 10 having a rack and pinion valve assembly, Figs. 39-45 illustrate a fifth example needle insertion mechanism 500 for use with a drug delivery device 10 having a bi-stable spring mechanism valve assembly, Figs. 46-67 illustrate a sixth example needle insertion mechanism 600 for use with a drug delivery device 10 having a single spindle valve assembly, Fig. 68 illustrates a seventh example needle insertion mechanism 700 for use with a drug delivery device 10 having a spring loaded spindle valve assembly, Figs. 69-72 illustrate an eighth example needle insertion mechanism 800 for use with a drug delivery device 10 having a rotational escapement valve assembly, Figs. 73-81 illustrate a ninth example needle insertion mechanism 900 for use with a drug delivery device 10 having a multi-rotational scotch yoke valve assembly, Figs. 82-94 illustrate a tenth example needle insertion mechanism 1000 for use with a drug delivery device 10 having a plunger displacement timing valve assembly, Figs. 95-104 illustrate an eleventh example needle insertion mechanism 1100 for use with a drug delivery device 10 having a linear slide valve assembly, and Figs. 105-108 illustrate a twelfth example needle insertion mechanism 1200 for use with a drug delivery device having a gas-powered needle insertion mechanism and linear slide valve assembly. It is appreciated that any of the needle insertion mechanisms 200, 300, 400, 500, 600, 700, 800, 900, 1000, 1100, 1200 illustrated in Figs. 15-108 may include similar features as the needle insertion mechanism 100, and accordingly, elements illustrated in Figs. 15-108 are designated by similar reference numbers indicated in the embodiment illustrated in Figs. 1-14 increased by multiples of 100. Accordingly, these features will not be described in substantial detail. Further, it is appreciated that any of the elements described with regards to the needle insertion mechanism 100 may be incorporated into any of the needle insertion mechanisms 200, 300, 400, 500, 600, 700, 800, 900, 1000, 1100, 1200, and vice-versa.

In the embodiment of Fig. 15, the valve assembly 260 may be in the form of a vertically-oriented second spindle 262 having a ramped portion 264 that engages the pin 217 of the needle yoke 210. More specifically, rotation of the second spindle 260 causes the pin 217 to move vertically upon traversing the ramp 264 until falling off the ledge 265. At this time, the pivotable member 275 may re-engage with the catches 266 of the second spindle 262.

In this example, the force exerted by the second spindle 262 may be in a single direction. A resilient member 280 (in the form of a compression spring in this example) may be incorporated to return the needle yoke 210 to the opposite valve position. Such a configuration may provide for drug delivery to be directly metered by alternately opening and closing the slide valve. In some examples, the drug 38 and fluid flow path 18 may be pressurized during the delay period.

In the embodiment of Figs. 16-25, the needle insertion mechanism 300 includes a second spindle 362 that is operably coupled with the first spindle 332 instead of with the needle yoke 310. In this example, the second end 332b of the first spindle 332 includes a gear 336 which may be coupled therewith and/or formed thereon. Similarly, the first end 362a of the second spindle 362 includes a second gear 368 that engages the gear 336 which may be coupled therewith and/or formed thereon. The second gear 368 coupled with the second spindle 362 may be a partial gear member to allow for additional rotation of the second spindle 362 during valve activation. The needle insertion mechanism 300 further includes an actuator escapement assembly 342 having a drive member 343, a linear escapement slide 344, and a pivotable urging member 345.

The linear escapement slide 344 includes a front tooth 344a, a back rocker tab 344b, an upper ledge 344c that defines an opening 344d, and a second tooth 344e. The linear escapement slide 344 is coupled with a first resilient member 347. The pivotable urging member 345 includes a first tooth 345a and a second tooth 345b. The pivotable urging member 345 is operably coupled with the drive member 343 and is pivotable about a pivot pin 345c. The pivotable urging member 345 is further coupled with a second resilient member 347a.

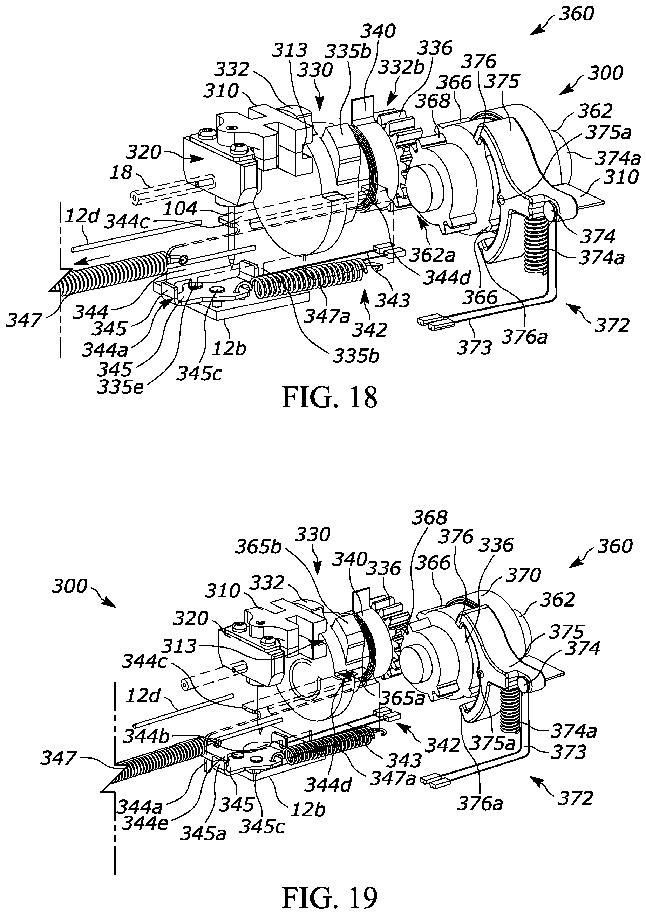

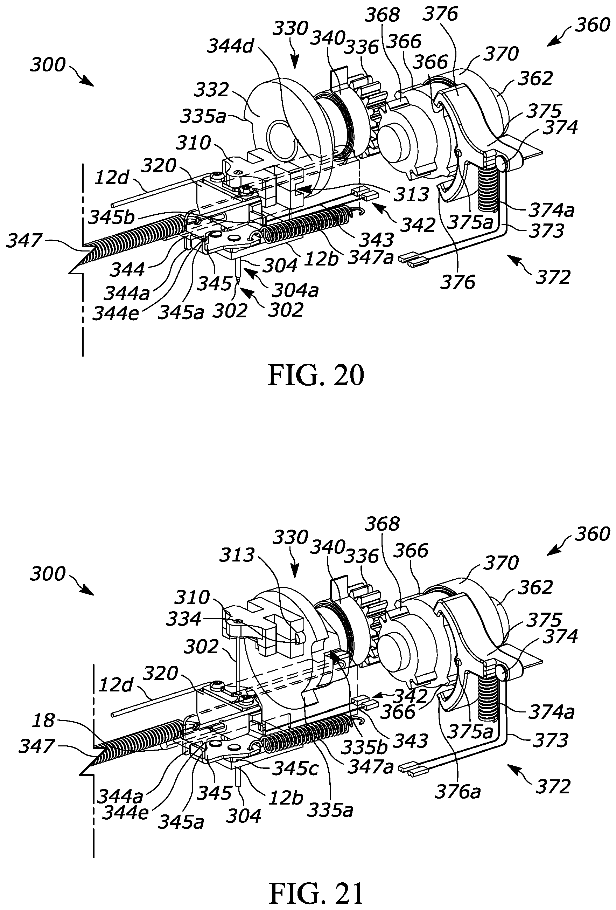

As illustrated in Fig. 16 and 17, in the storage state, the gears 336, 368 are disengaged, meaning the gear teeth of the second gear 368 are not disposed in a position that can engage the gear 336. Further, the first spindle 332 is retained in a stationary position by engagement of the upper ledge 344c with the first catch 335a of the first spindle 332. Upon activation of the device 10, the drive member 343 of the actuator escapement assembly 342 energizes to release the first escapement of the first spindle 332. More specifically, the drive member 343 causes the pivotable urging member 345 to pivot about the pivot pin 345c, thereby disengaging the front tooth 344a of the linear escapement slide 344 from the first tooth 345a of the pivotable urging member 345. As illustrated in Figs. 18 and 19, this release causes the first resilient member 347, coupled with the linear escapement slide 344, to urge the linear escapement slide 344 away from the first spindle 332. As illustrated in Fig. 19, this movement eventually aligns the opening 344d of the upper ledge 344c with the first catch 335a, thereby allowing the first spindle 332 to rotate to cause the needle yoke 310 and the cannula yoke 320 to move to a lowered position (Fig. 20). The back rocker tab 344b of the linear escapement slide 344 engages the second tooth 345b of the pivotable urging member 345 to limit travel of the linear escapement slide 344 while the drive member 343 is energized.

As illustrated in Fig. 19, upon de-energizing, the drive member 343 lengthens and the second resilient member 347a urges the pivotable urging member 345 to pivot about the pivot pin 345c to return to the first position. This rotation causes the second tooth 345b to release from the back rocker tab 344b of the linear escapement slide 344, thereby causing the linear escapement slide 344 to again be urged in a direction away from the first spindle 332. The first tooth 345a of the pivotable urging member 345 then engages with the second tooth 344e of the linear escapement slide 344 to limit movement thereof.

As previously noted, with reference to Fig. 20, the first spindle 332 continues to rotate to drive the needle yoke 310 and the cannula yoke 320 downward. As before, the cannula yoke 320 latches near the bottom of the stroke of the first spindle 332. With reference to Fig. 21, as the first spindle 332 continues to rotate, the drive pin 334 rotates to contact the upper portion of the slot 313 formed on the needle yoke 310 to move the needle yoke 310 upwards. The upper ledge 344c of the linear escapement slide 344 engages the second catch 335b formed on the first spindle 332 to stop the spindle 332 from rotating. As with the needle insertion mechanism 100, 200, in this position, the valve assembly 360 is in a retracted, valve closed position whereby the needle 302 engages the top of the cannula 304 to retain a seal (not illustrated).

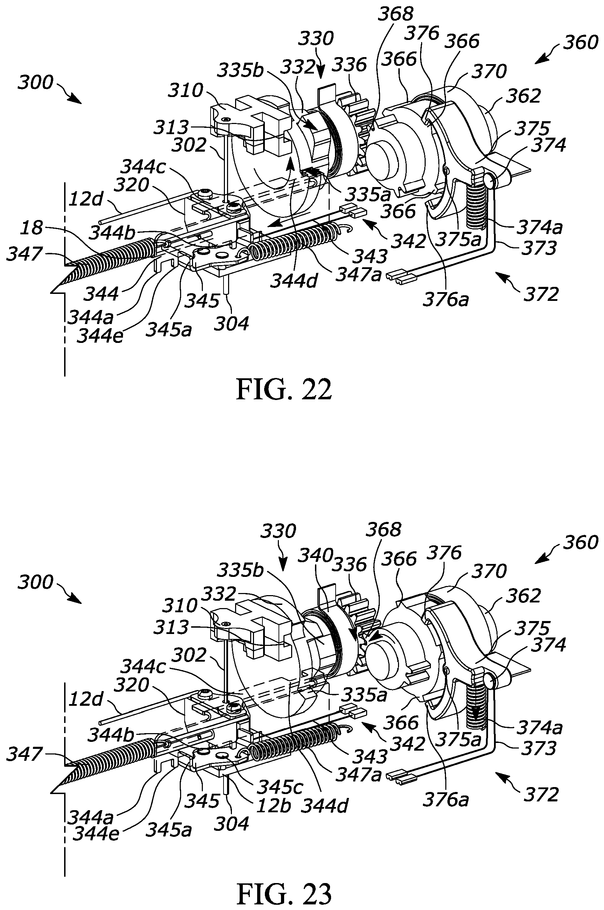

As illustrated in Fig. 22, a second activation of the actuator escapement assembly 342 whereby the drive member 343 energizes to rotate the pivotable urging member 345 causes the upper ledge 344c of the linear escapement slide 344 to disengage from the second catch 335b of the first spindle 332. Accordingly, the first spindle 332 again rotates until the upper ledge 344c of the linear escapement slide 344 again engages the first catch 335a of the first spindle 332. This rotation of the first spindle 332 causes the drive pin 334 to urge the needle yoke 310 upwards to a valve open position whereby the drug 38 may be administered.

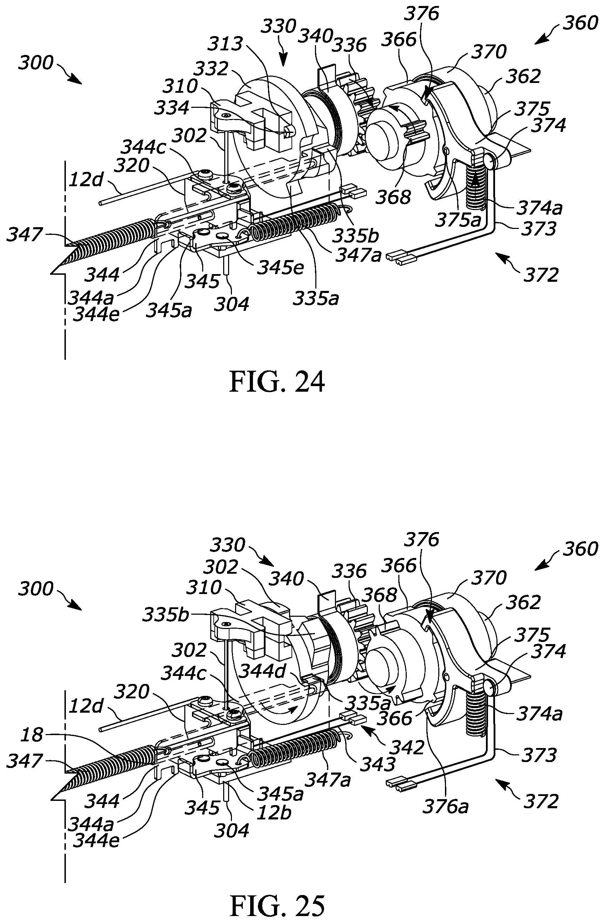

With reference to Fig. 23, the valve escapement assembly 372 may then be activated to move the valve assembly 360 to a valve closed position. As with the valve escapement assembly 172, the driving member 373 is first engaged to cause the pivotable member 375 to pivot about the pivot pin 375a, thereby disengaging the first finger 376 from the catch 366, thereby permitting the second spindle 362 to rotate. This rotation causes the second gear 368 to engage the gear 336 coupled with the first spindle 332, thereby driving the first spindle 332 backwards against the remaining torque of the resilient member 340 used for needle 302 insertion and retraction. As illustrated in Fig. 24, when the first finger 376 again engages a catch 366, the second spindle 362 ceases rotation. In this configuration the gear teeth of the first and second gears 336, 338 remain engaged, and as such, the valve 360 remains in the valve closed configuration. In this configuration, the upper ledge 344c of the linear escapement slide 344 is not in engagement with the first spindle 332.

With reference to Fig. 25, the valve escapement assembly 372 may again be activated to administer additional aliquots. More specifically, upon engaging the driving member 373, the first finger 376 disengages from the catch 366 on the second spindle 362, thereby causing the gear teeth of the second gear 368 to disengage from the gear teeth of the first gear 336. Accordingly, the first spindle 332 is released to rotate back to the valve open position, and the upper ledge 344c of the linear escapement slide 344 again engages the first catch 335a formed on the first spindle 332. As before, in this position, the needle yoke 310, and thus the needle 302, are positioned in the valve open position. Similar steps may be repeated whereby the second spindle 362 selectively engages the first spindle 332 to raise and lower the needle yoke 310.

In some of these examples, additional gear and/or rack members may be incorporated between the second spindle 362 and the first spindle 332. Further, the number of gear teeth formed on both the first and the second gears 336, 368 may be varied. In some examples, a single gear tooth on each spindle may be sufficient depending on the travel required to actuate the valve assembly. Further, in some examples, it may be desirable to immediately deliver the first aliquot, and as such, the mechanism may be designed to urge the needle directly to the valve open position after insertion of the cannula. In some examples, the rotational increments used may be varied, and the rotation required from the second spindle may be altered, depending on the gear ratio used. In some forms, drug delivery may be triggered by a common escapement mechanism, and may be metered directly by closing the slide valve with this mechanism. In the case of a spring or gas driven drug delivery system, the drug contents and fluid path may be pressurized during the delay period.

In the embodiment of Figs. 26-38, the needle insertion mechanism 400 includes a rack and pinion mechanism design to insert and retract the needle 402. The rack and pinion mechanism translates linear motion into circular motion or vice versa. In the illustrated example, driving the rack linearly causes the pinion to rotate, and rotating the pinion causes the rack to move linearly. In examples where immediate (i.e., less than approximately 20 seconds) single dose delivery is desired, the needle insertion mechanism 400 can drive a scotch yoke mechanism to only insert the needle 402 into the patient's tissue 11 and retract it once drug delivery is complete. In other examples where multidose delivery is desired, the needle insertion mechanism 400 can be combined with a sliding sleeve valve 460 incorporating a rigid needle and soft cannula.

The needle insertion mechanism 400 is similar to the needle insertion mechanism 300, but differs in that instead of urging the first spindle 432 with a gear mechanism, a rack and pinion assembly is instead used. More specifically, the needle insertion mechanism 400 includes a valve actuation rack 462 that is operably coupled with and urges a pinion 436 coupled with the first spindle 432 and/or integrally formed thereon, which in turn moves the needle yoke 410 between the valve open and valve closed positions. In the illustrated example, the pinion 436 includes a single tooth 436a, but in other examples, the pinion 436 may include any number of teeth. The needle insertion mechanism 400 further includes an actuator escapement assembly 442 having a drive member 443, a linear escapement slide 444, and a pivotable urging member 445.

As illustrated in Fig. 26, the linear escapement slide 444 includes a front tooth 444a, a back rocker tab 444b, an upper ledge 444c that defines an opening 444d, and a second tooth 444e. The linear escapement slide 444 is coupled with a first resilient member 447. The pivotable urging member 445 includes a first tooth 445a and a second tooth 445b. The pivotable urging member 445 is operably coupled with the drive member 443 and is pivotable about a pivot pin 445c. The pivotable urging member 445 is further coupled with a second resilient member 447a. The valve actuation rack 462 includes a number of catches or protrusions 466 and a number of engaging tabs 468. A resilient member 469 in the form of an extension or a compression spring is coupled with the valve actuation rack 462.

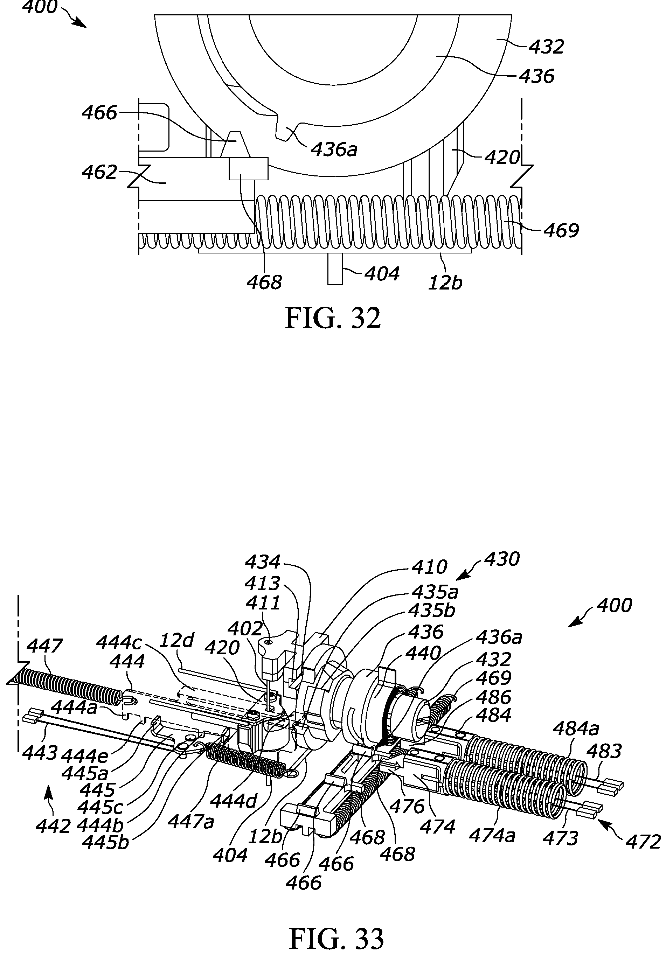

As illustrated in Figs. 26 and 32, in the storage state, the valve actuation rack 462 does not engage the pinion 436, meaning the protrusions 466 of the valve actuation rack 462 are not disposed in a position that can engage the tooth 436a of the pinion 436. Further, as with the previously-described needle insertion mechanism 300, the first spindle 432 is retained in a stationary position by engagement of the upper ledge 444c with the first catch 435a of the first spindle 432. As illustrated in Fig, 27, upon activation of the device 10, the drive member 443 of the actuator escapement assembly 442 energizes to release the first escapement of the first spindle 432.

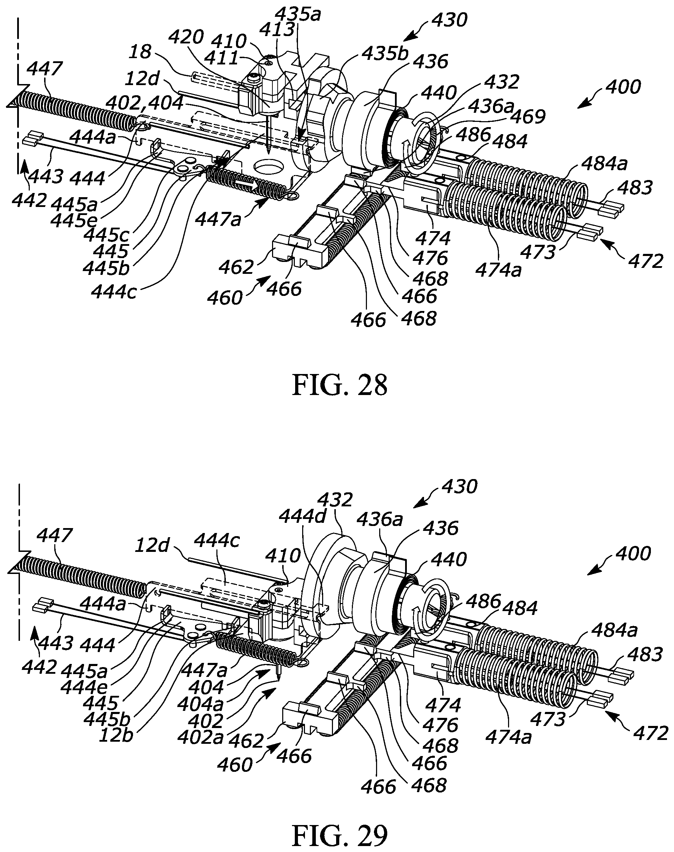

More specifically, the drive member 443 causes the pivotable urging member 445 to pivot about the pivot pin 445c, thereby disengaging the front tooth 444a of the linear escapement slide 444 from the first tooth 445a of the pivotable urging member 445. This release causes the first resilient member 447, coupled with the linear escapement slide 444, to urge the linear escapement slide 444 away from the first spindle 432. As illustrated in Fig. 28, this movement eventually aligns the opening 444d of the upper ledge 444c with the first catch 435a, thereby allowing the first spindle 432 to rotate to cause the needle yoke 410 and the cannula yoke 420 to move to a lowered position (Fig. 29). The back rocker tab 444b of the linear escapement slide 444 engages the second tooth 445b of the pivotable urging member 445 to limit travel of the linear escapement slide 444 while the drive member 443 is energized.

As illustrated in Fig. 28, upon de-energizing, the drive member 443 lengthens and the second resilient member 447a urges the pivotable urging member 445 to pivot about the pivot pin 445c to return to the first position. This engagement causes the second tooth 445b to release from the back rocker tab 444b of the linear escapement slide 444, thereby causing the linear escapement slide 444 to again be urged in a direction away from the first spindle 432. The first tooth 445a of the pivotable urging member 445 then engages with the second tooth 444e of the linear escapement slide 444 to limit movement thereof.

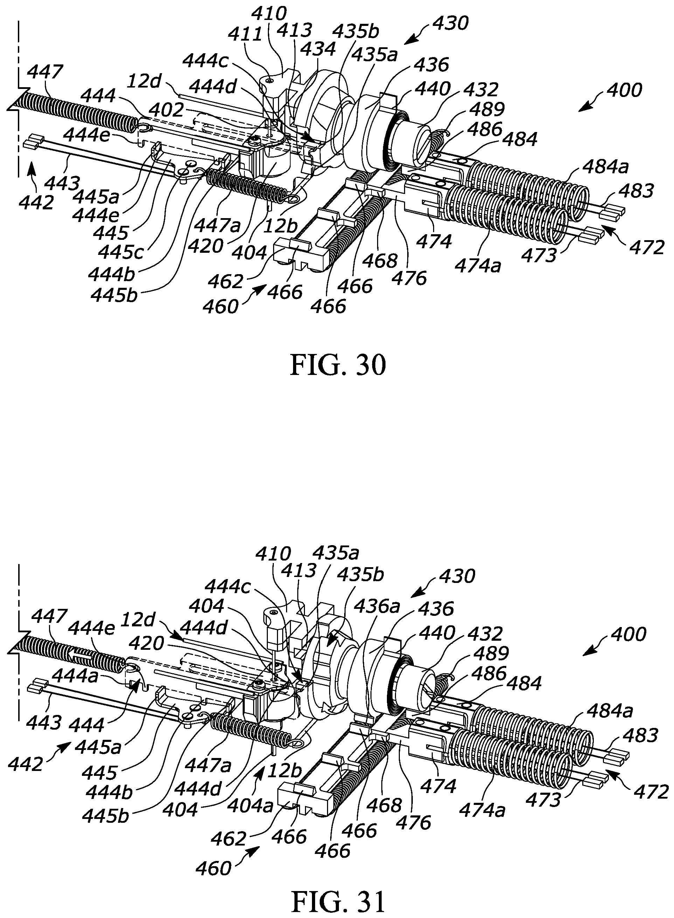

As previously noted, with reference to Fig. 29, the first spindle 432 continues to rotate, driving the needle yoke 410 and the cannula yoke 420 downward. As before, the cannula yoke 420 latches near the bottom of the stroke of the first spindle 432. With reference to Fig. 30, as the first spindle 432 continues to rotate, the drive pin 434 rotates to contact the upper portion of the slot 413 formed on the needle yoke 410 to move the needle yoke 410 upwards. The upper ledge 444c of the linear escapement slide 444 engages the second catch 435b formed on the first spindle 432 to stop the spindle 432 from rotating. As with the needle insertion mechanisms 100, 200, 300, in this position, the valve assembly 460 is in a retracted, valve closed position whereby the needle 402 engages the top of the cannula 304 to retain a seal (not illustrated).

As illustrated in Fig. 31, a second activation of the actuator escapement assembly 442 whereby the drive member 443 energizes to rotate the pivotable urging member 445 causes the upper ledge 444c of the linear escapement slide 444 to disengage from the second catch 435b of the first spindle 432. Accordingly, the first spindle 432 again rotates until the upper ledge 444c of the linear escapement slide 444 again engages the first catch 435a of the first spindle 432. This rotation of the first spindle 432 causes the drive pin 434 to urge the needle yoke 410 upwards to a valve open position whereby the drug 38 may be administered.

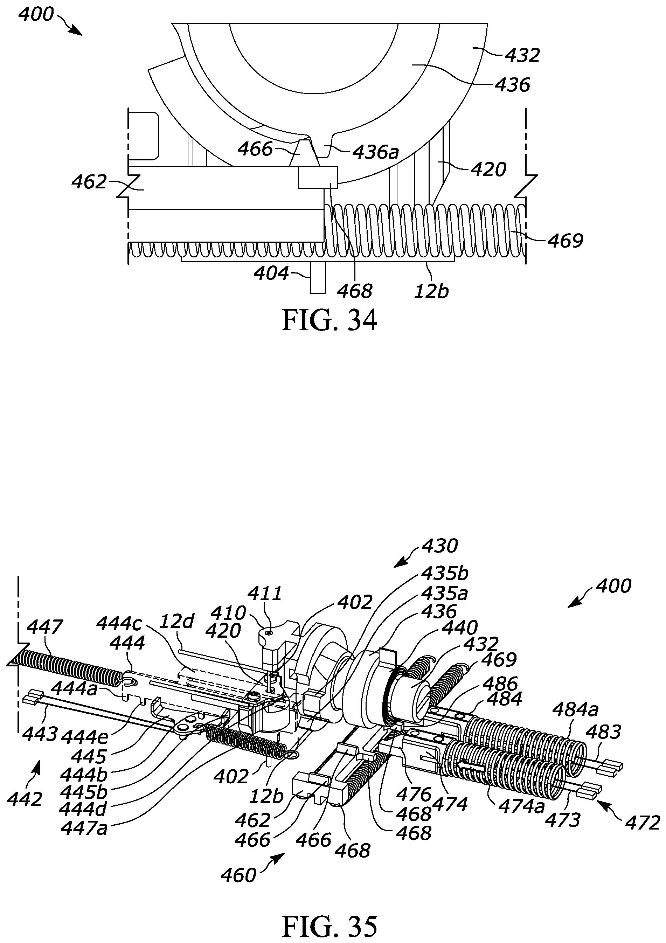

With reference to Figs. 33 and 34, the valve escapement assembly 472 may then be activated to move the valve assembly 460 to a valve closed position. In this example, the valve escapement assembly 472 includes a first driving member 473, a first urging member 474, a first resilient member 474a in the form of a compression spring, a first finger 476, and a second driving member 483, a second urging member 484, a second resilient member 484a also in the form of a compression spring, and a second finger 486. As with the valve escapement assembly 372, the first driving member 473 is first energized to pull the first finger 476, thereby disengaging the first finger 476 from the first engaging tab 468 to allow the resilient member 469 to urge the valve actuation rack 462 to slide forward. Accordingly, as illustrated in Fig. 34, the protrusion 466 of the valve actuation rack 462 engages and the tooth 436a of the pinion 436 forward, which in turn causes the first spindle 432 to be driven backwards against the remaining torque of the resilient member 440 used for needle 402 insertion and retraction.

As illustrated in Fig. 35, when the first driving member 473 is de-energized, the first resilient member 474a urges the first urging member 474 toward the first spindle 432, which urges the first finger 476 to its original position. Further, in this position, the second finger 486 engages the first engaging tab 468. As shown in Fig, 36, in this configuration, the tooth 436a remains engaged with the first protrusion 466, and as such, the valve 460 remains in the valve closed configuration. In this configuration, the upper ledge 444c of the linear escapement slide 444 is not in engagement with the first spindle 432.

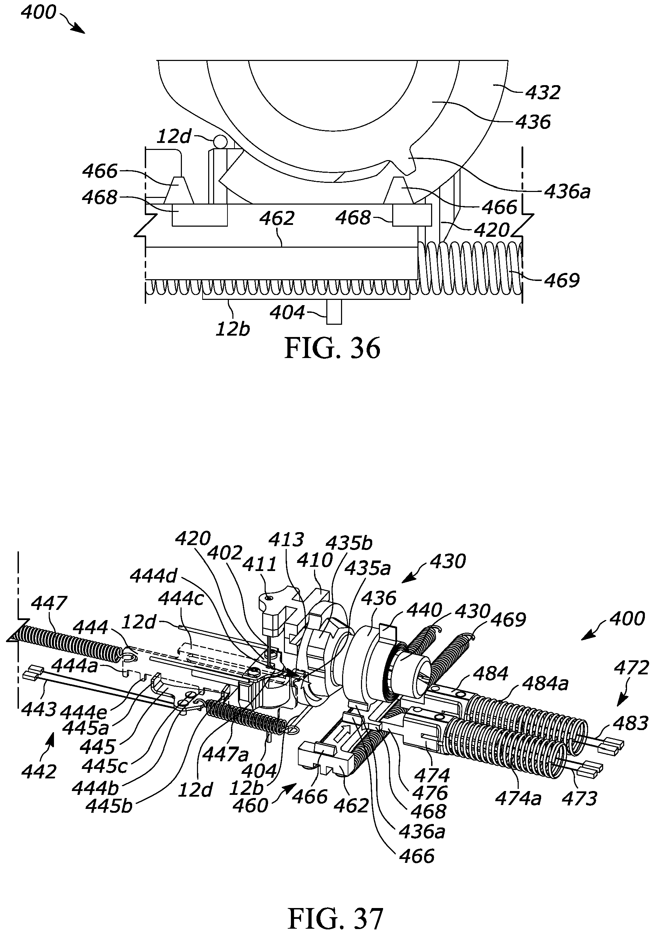

With reference to Figs. 37 and 38, the valve escapement assembly 472 may again be activated to administer a second drug dosage. More specifically, upon engaging the first driving member 473, the first finger 476 disengages from the engaging tab 468, thereby causing the tooth 436a to disengage from each protrusion 466. In some examples, the second driving member 483 may also be engaged to move the second finger 486 to disengage it from the engaging tab 468. Accordingly, the first spindle 432 is released to rotate back to the valve open position, and the upper ledge 444c of the linear escapement slide 444 again engages the first catch 435a formed on the first spindle 432. As before, in this position, the needle yoke 410, and thus the needle 402, are positioned in the valve open position. Similar steps may be repeated whereby the valve actuation rack 462 selectively engages the first spindle 432 to raise and lower the needle yoke 410.

In some of these examples, additional gear teeth may be added to the pinion and/or the valve actuation rack. Further, the number of gear teeth formed on both the first and the second gears 336, 368 may be varied. In some examples, a clock spring may be used with an additional pinion to drive the rack. Further, in some examples, it may be desirable to immediately deliver the first aliquot, and as such, the mechanism may be designed to urge the needle directly to the valve open position after insertion of the cannula. In some examples, the rotational increments used may be varied, and the rotation required from the second spindle may be altered, depending on the gear ratio used. In some forms, drug delivery may be triggered by a common escapement mechanism, and may be metered directly by closing the slide valve with this mechanism. In the case of a spring or gas driven drug delivery system, the drug contents and fluid path may be pressurized during the delay period. Other escapement mechanisms may be used to control the rotation of the spindle or release of the valve actuation rack including the rocker linear escapement used for insertion.

In the embodiment of Figs. 39-45, the needle insertion mechanism 500 includes a bi-stable spring mechanism design to insert and retract the needle 502. The design is based on controlled buckling of a linear spring that uses a similar escapement mechanism as a triggering system to switch between two stable configurations corresponding to needle insertion and retraction positions. Once the bi-stable spring is in one of the distinct equilibrium positions, it does not need external energy to be maintained. In examples where immediate (i.e., less than approximately 20 seconds) single dose delivery is desired, the needle insertion mechanism 500 can drive a scotch yoke mechanism to only insert the needle 502 into the patient's tissue 11 and retract it once drug delivery is complete. In other examples where multidose delivery is desired, the needle insertion mechanism 500 can be combined with a sliding sleeve valve 560 incorporating a rigid needle and soft cannula.

In this example, the first spindle 532 engages the actuator escapement assembly 542 in a similar manner as in the previously-described needle insertion mechanism 100. More specifically, the spindle 532 includes a needle insertion mechanism engagement portion in the form of a drive pin (not visible) that slidably engages the first spindle engagement portion 513 of the needle yoke 510. The spindle 532 also includes an actuation escapement assembly engagement portion in the form of a first catch 535a and a second catch (not illustrated). The actuator escapement assembly 542 may be mounted to the needle insertion mechanism 500 and may include a driving member 543, an urging member 544 operably coupled with the driving member 543, and a pivotable member 545 having one or more pivot pins 545a and that may be operably coupled with the urging member 544.

The driving member 543 is in the form of a linear actuator that is coupled with the urging member 544 using any suitable fastening method. As with the previously-described driving members, when energized (for example, via a battery), the driving member 543 is adapted to contract, thereby reducing its overall length. In the illustrated examples, the urging member 544 is in the form of a pin coupled with the pivotable member 545. In other examples, the urging member 544 may take other forms such as a spring. Other examples are possible. The pivotable member 545 further includes an engaging portion in the form of a platform 546. Generally, the pivotable member 545 pivots about the pivot pin 545a to selectively position the platform 546. When the driving member 543 is energized, the driving member 543 contracts, thereby pulling the urging member 544 and moving the pivotable member 545 to the second position.

The valve assembly 560 includes a bi-stable spring 562 and first and second pivotable members or bodies 564 operably coupled therewith. In the illustrated example, a portion of the bi-stable spring 562 is inserted through the needle 502, but in other examples, the bi-stable spring 562 may be coupled with the needle 502 via any number of suitable approaches. The pivotable members 564 are also pivotably coupled with the needle yoke 510 via a mounting portion 511. Each of the pivotable members 564 includes tabs 564a extending therefrom to be engaged by the valve escapement assembly 572. More specifically, in the illustrated example, the tabs 564a are positioned on opposing sides of a pivot point 565 of the pivotable member.

Like the actuator escapement assembly 542, the driving member 573 of the valve escapement assembly 572 is in the form of a number of linear actuators. In the illustrated example, the driving member 573 includes four linear actuators (i.e., two outer linear actuators 573a and two inner linear actuators 573b), each of which being operably coupled with one of the tabs 564a of the pivotable members 564. In some examples, the driving member 573 is operably coupled to the actuator 16 and/or the controller 14 via an electrical connection. When energized (for example, via a battery), the driving member 573 is adapted to contract, thereby reducing its overall length. Generally speaking, the outer actuators 573a and the inner actuators 573b are engaged to selectively urge the bi-stable spring 562 between first and second positions.

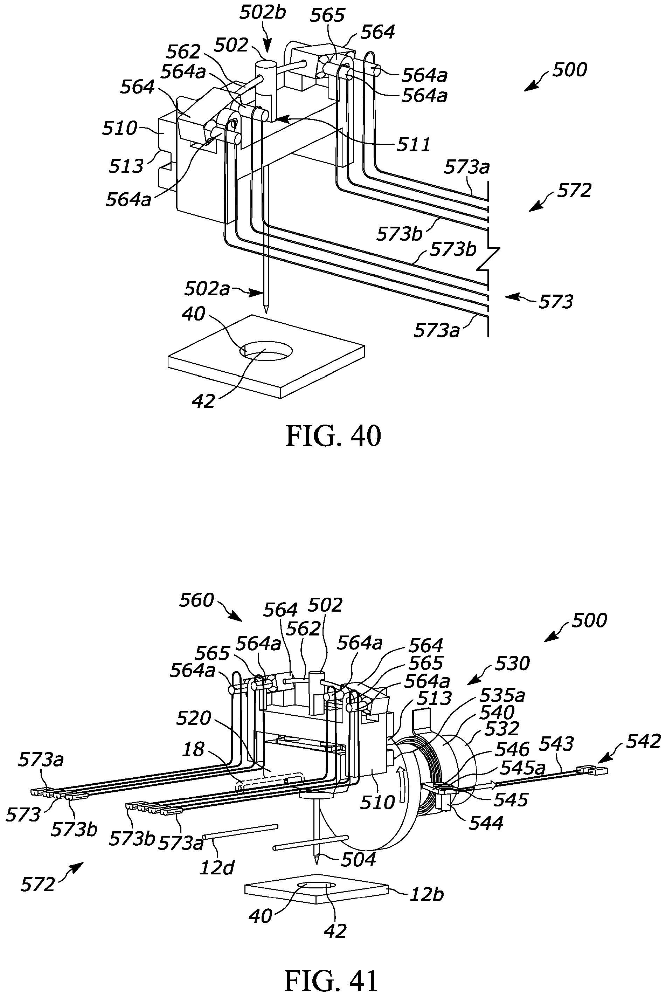

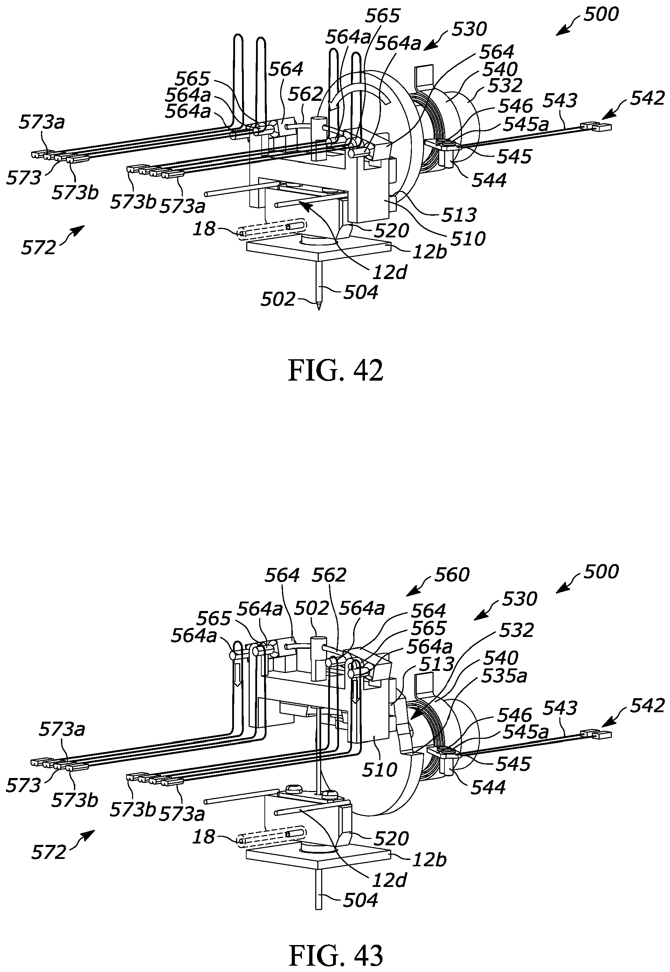

As illustrated in Fig. 39, the needle insertion mechanism 500 is positioned in a storage state whereby the needle yoke 510 and the cannula yoke 520 are both in raised, retracted positions. In this configuration, the platform 546 of the pivotable member 545 engages the first catch 535a of the first spindle 532 to prevent the first spindle 532 from rotating via the urging force exerted thereupon by the resilient member 540. With reference to Fig. 41, upon engaging the activation mechanism 20, the drive member 543 is energized and urges the urging member 544 to cause the platform 546 to move to the second position, thereby disengaging from the first catch 535a. The resilient member 540 then causes first spindle 532 to rotate, which in turn causes the drive pin (not visible) to rotate, thus engaging the lower tab 514 of the needle yoke 510. As illustrated in Fig. 42, this movement urges the needle yoke 510 and the cannula yoke 520 to a lowered, extended position.

In Fig. 42, the needle 502 and the cannula 504 are fully extended below the bottom wall 12b of the device 10. As previously noted, the latching member 12d located near the bottom wall 12b of the housing 12 engages a portion of the cannula yoke 520 to retain and prevent the cannula yoke 520 (and thus the cannula 504) from moving in an axial direction. In this example, the latching member 12d is in the form of a leaf spring. Other examples of suitable latching members are possible.

With reference to Fig. 43, the first spindle 532 continues to rotate until a ball bearing (not illustrated) stops the spindle 532. In this position, the needle 502 is stopped in a valve open position where the needle 502 is positioned above the cannula 504 to allow fluid to flow through the cannula 504 for drug administration.

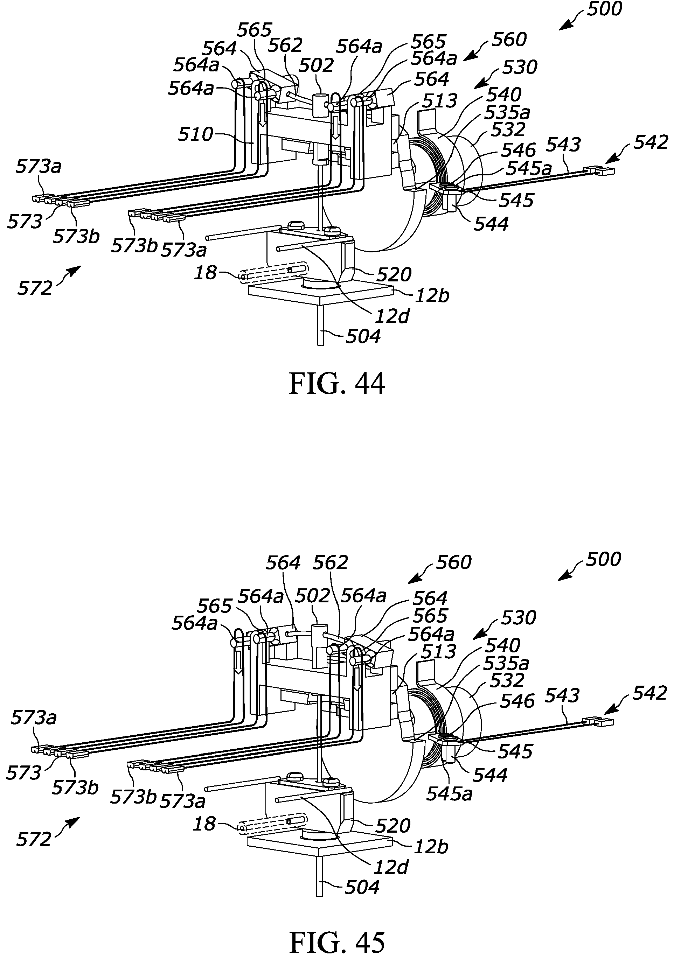

With reference to Fig. 44, when administration of the drug or medicament 38 is complete, the actuator 16 and/or the controller 14 transmits an electrical signal to the valve assembly 560, and specifically to the inner linear actuators 573b. Upon contracting, the inner linear actuators 573b urge the tabs 564a coupled thereto downwards, causing the pivotable member 564 to rotate. This rotation causes the bi-stable spring 562 to move to the lowered position, which in turn causes the needle 502 to move to a lowered, valve closed position. Because the bi-stable spring 562 holds the needle 502 in position, the inner linear actuators 573b only need to be momentarily activated. In this position, there may be a delay prior to subsequent drug deliveries. Here, there is no change in needle 502 or cannula 504 position. Because the valve assembly 560 is closed, blood and/or other bodily fluids are unable to enter the device fluid path, reducing the likelihood of clogs and/or clots in the fluid path 18.

With reference to Fig. 45, the needle 502 may be retracted to the valve open position by toggling the bi-stable spring 562 to the valve open position by activating the outer linear actuators 573a. This in turn pulls the tabs 564a coupled thereto downwards, causing the pivotable member 564 to rotate which urges the bi-stable spring 562 to move to the raised position, which in turn causes the needle 502 to move to the raised, valve open position.

In some examples, the bi-stable spring 562 may take the form of a flat sheet instead of a wire. Further, in some examples, the bi-stable spring 562 may be oriented in the closed position after insertion of the cannula 504 such that a delay can be implemented before the initial dose. In some alternatives, a slide valve using an introducer needle with a side port that can be sealed inside the cannula 504 or septum 508 (not illustrated) may be used. Further other mechanisms may be used in place of the muscle wires to toggle the bi-stable spring. In some forms, drug delivery may be metered directly by modulating the valve assembly 560 with this mechanism. In the case of a spring or gas driven drug delivery system, the drug contents and fluid path may be pressurized during the delay period(s).

In the embodiment of Figs. 46-67, the needle insertion mechanism 600 includes a single spindle with an escapement mechanism design. In examples where immediate (i.e., less than approximately 20 seconds) single dose delivery is desired, the needle insertion mechanism 600 can drive a scotch yoke mechanism to only insert the needle 602 into the patient's tissue 11 and retract it once drug delivery is complete. In other examples where multidose delivery is desired, the needle insertion mechanism 600 can be combined with a sliding sleeve valve 660 incorporating a rigid needle and soft cannula.

In this example, the first spindle 632 includes an outer drive pin 634a, an inner drive pin 634b, a clearance area 635 for the pin 617 of the needle yoke 610, a cam track 636 for valve actuation, and a number of catches 638. Further, as illustrated in Fig. 48, the needle yoke 610 includes a guide slot 612 to constrain the needle yoke 610 to vertical motion during operation, a slot 613 at least partially defined by a driving tab 614 and an upper tab 615, and the above-mentioned pin 617. Generally, the upper tab 615 is used to retract the needle 602. The slot 613 extends asymmetrically so the outer drive pin 634a may lift the needle yoke 610 and escape from the inner drive pin 634b. Further, the pin 617 may be used to move the needle yoke 610 up and down to operate the valve assembly 660.

The escapement assembly 642 includes a drive member 643, a linear escapement slide 644, and a pivotable urging member 645. The linear escapement slide 644 includes a number of teeth 644a and a back tab 644b. The linear escapement slide 644 is coupled with a first resilient member 647. The pivotable urging member 645 includes a first tooth 645a and a second tooth 645b. The pivotable urging member 645 is operably coupled with the drive member 643 and is pivotable about a pivot pin 645c. The pivotable urging member 645 is further coupled with a second resilient member 647a.

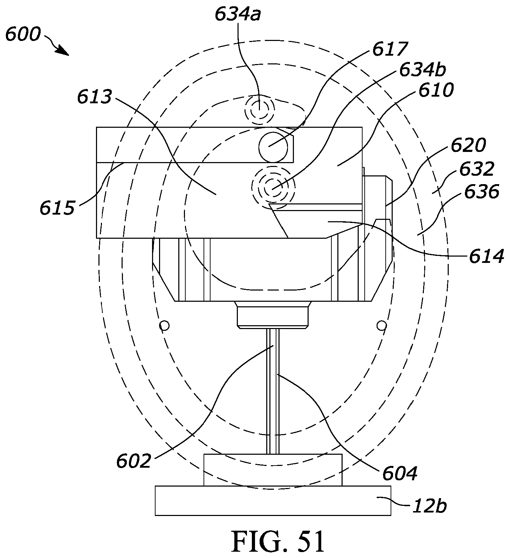

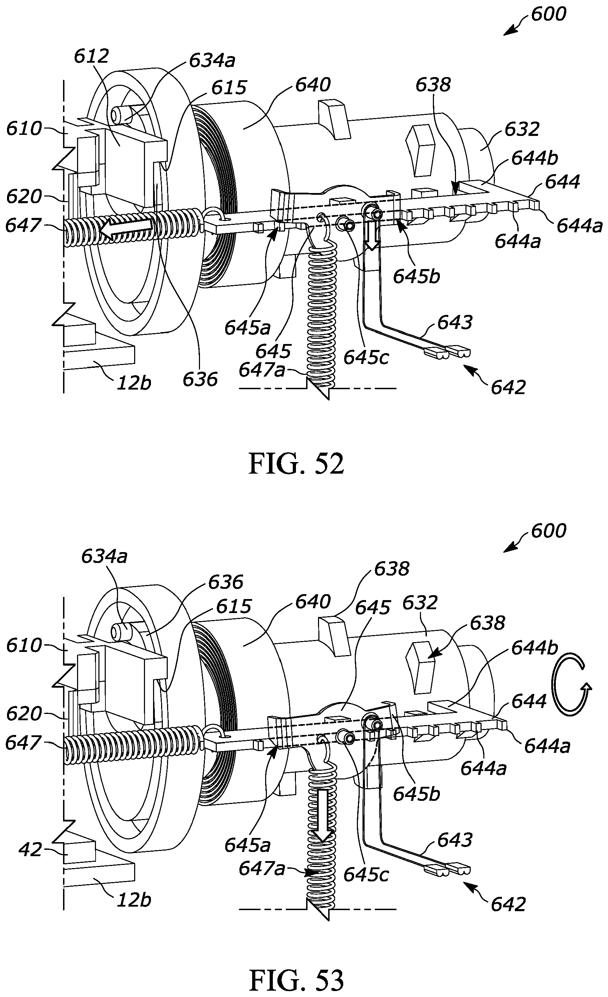

With reference to Figs. 46-51, in the storage state, the needle 602 and the cannula 604 are both fully retracted. Further, as illustrated in Fig. 51, the needle yoke pin 617 is disposed between the outer and inner drive pins 634a, 634b. With reference to Figs. 52 and 53, upon activation of the device 10, the drive member 643 energizes to release the first escapement of the first spindle 632. More specifically, the drive member 643 pulls the pivotable urging member 645 downwards, thus disengaging the first tooth 645a of the pivotable urging member 645 from the teeth 644a of the linear escapement slide 644. The first resilient member 647 then urges the linear escapement slide 644 in a direction towards the needle yoke 610. The pivotable urging member 645 limits advancement of the linear escapement slide 644 by engaging the teeth 644a thereof with the second tooth 645b. In this position, the back tab 644b engages a catch 638 on the first spindle 632, thereby preventing rotation thereof.

Referring now to Fig. 53, upon de-energizing, the drive member 643 lengthens, allowing the second resilient member 647a to cause the pivotable urging member 645 to again pivot about the pivot pin 645c. Accordingly, the second tooth 645b disengages from the teeth 644a of the linear escapement slide 644, whereupon the first resilient member 647 urges the linear escapement slide 644 further towards the needle yoke 610 until the first tooth 645a of the pivotable urging member 645 again engages a tooth 644a of the linear escapement slide. At this time, the back tab 644b becomes disengaged from a catch 638, and the spindle 632 is released to rotate until the back tab 644b engages a subsequent catch 638.

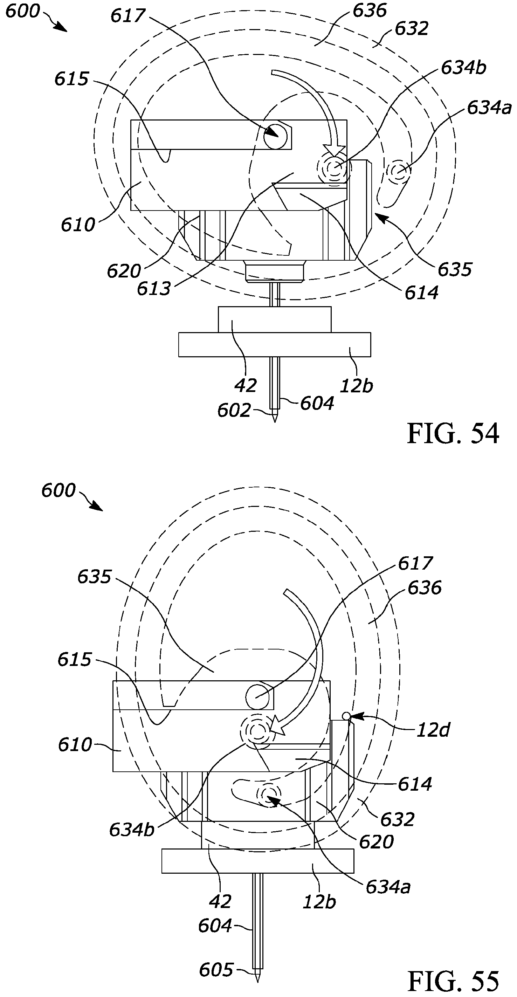

As illustrated in Figs. 54 and 55, with the spindle 632 released, the inner drive pin 634b operates like the previously-described scotch yoke mechanisms to drive the needle yoke 610 downward. Both the outer drive pin 634a and the needle yoke pin 617 have clearance during insertion. As illustrated in Fig. 55, the cannula yoke 620 latches near the bottom of the stroke.

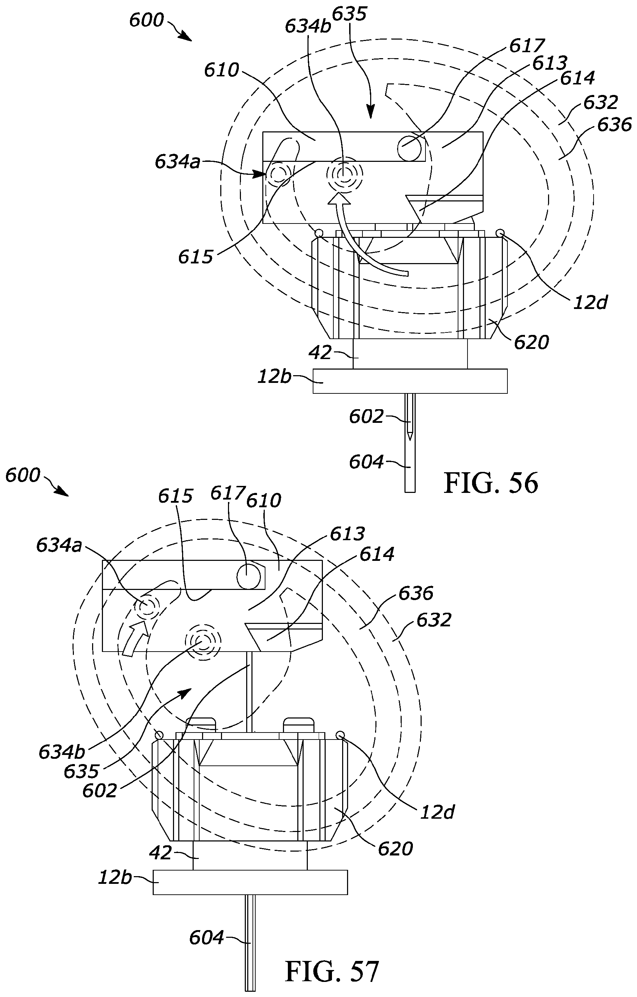

As illustrated in Figs. 56-58, the spindle 632 continues to rotate, moving the needle 602 toward the valve closed position. As illustrated in Fig. 56, initially, the inner drive pin 634b contacts the upper tab 615 of the needle yoke 610 to lift the needle yoke 610 upward. As illustrated in Fig. 57, approximately midway through retraction, the outer drive pin 634a begins to lift the needle yoke 610. The rotation stops when a catch 638 on the spindle 632 again engages the back tab 644b of the linear escapement slide 644. In this embodiment, the spindle 632 stops after rotating approximately 315°. The spindle 632 is stopped in the valve closed position where the needle 602 remains in the top of the cannula 604 to retain a seal. If necessary, a delay may be implemented prior to the initial aliquot of drug delivery.

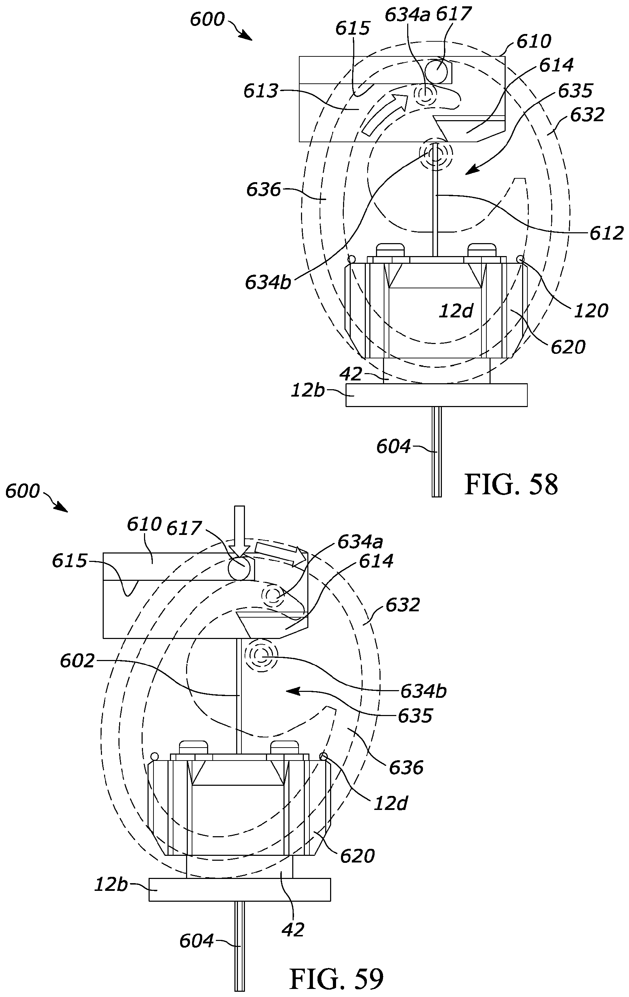

As illustrated in Fig. 58, to administer the drug 38, the drive member 643 is again energized to trigger the next rotational escapement increment of the spindle 632. The back tab 644b of the linear escapement slide 644 again disengages from the catch 638 of the first spindle 632, and the first spindle rotates approximately 45°. The outer drive pin 634a lifts the needle yoke 610 to the top of the stroke, thereby moving the needle 602 to the valve open position. In this example, the outer drive pin 634a lifts the needle yoke 610 high enough for the inner drive pin 634b to escape from the slot 613. At this time, the drug 38 may be delivered to the user.

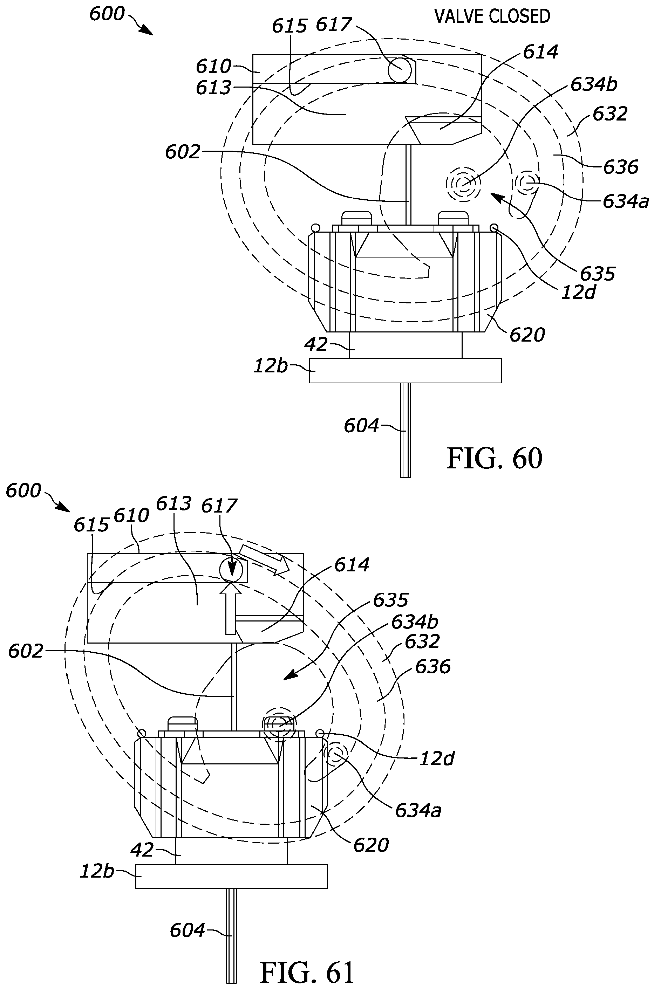

With reference to Figs. 59 and 60, the needle insertion mechanism 600 is moved to the valve closed position. The drive member 643 is again energized to trigger the next rotational escapement increment of the spindle 632. The back tab 644b of the linear escapement slide 644 again disengages from the catch 638 of the first spindle 632, and the first spindle rotates approximately 90°. The needle yoke pin 617, now disposed within the cam track 636, is driven downward to the valve closed position (Fig. 60). In this example, the needle yoke 610 has clearance with the inner and outer drive pins 634a, 634b to avoid binding. As before, if necessary, a delay may be implemented prior to the next aliquot of drug delivery.

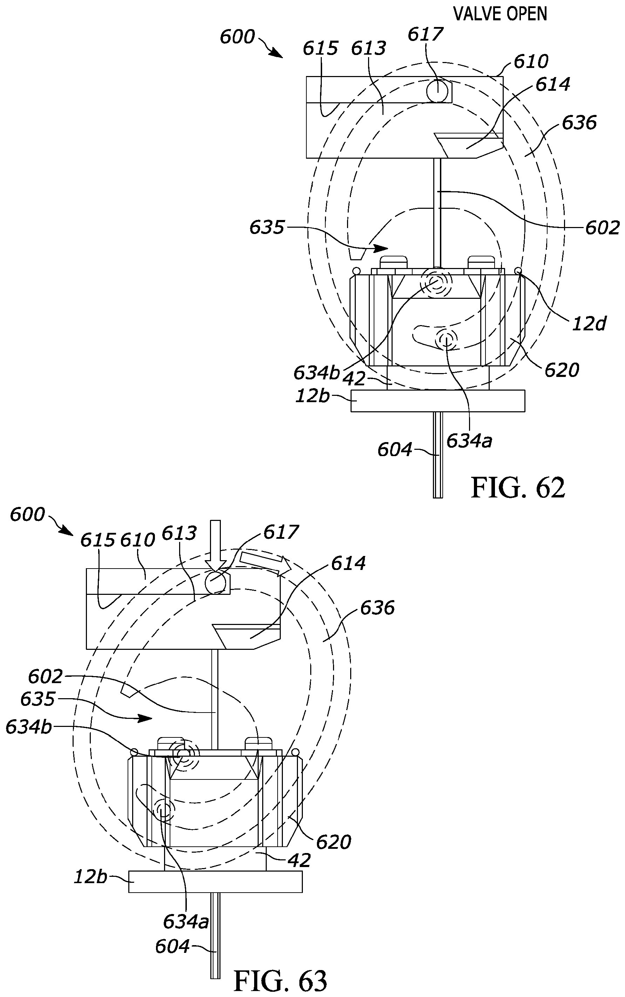

With reference to Figs. 61 and 62, to again administer the drug 38, the drive member 643 is again energized to trigger the next rotational escapement increment of the spindle 632. The back tab 644b of the linear escapement slide 644 again disengages from the catch 638 of the first spindle 632, and the first spindle rotates approximately 90°. Again, the inner surface of the cam track 636 urges the needle yoke pin 617 upwards to the valve open position (Fig. 62). At this time, the drug 38 may be delivered to the user.

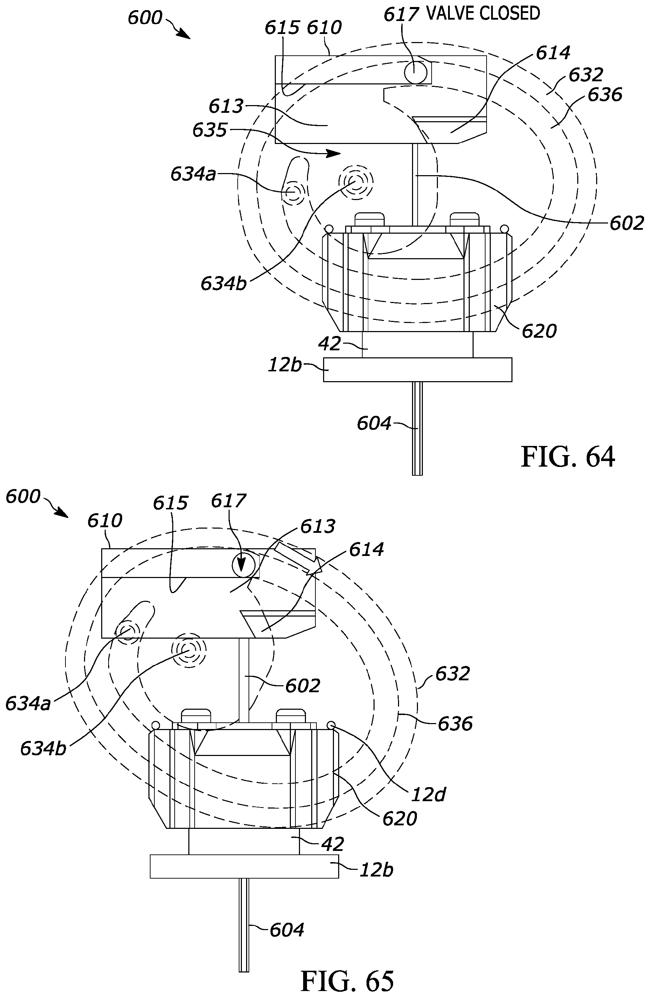

With reference to Figs. 63 and 64, the needle insertion mechanism 600 is again moved to the valve closed position. The drive member 643 is again energized to trigger the next rotational escapement increment of the spindle 632. The back tab 644b of the linear escapement slide 644 again disengages from the catch 638 of the first spindle 632, and the first spindle rotates approximately 90°. The needle yoke pin 617, is driven downward to the valve closed position (Fig. 64) by the outer surface of the cam track 636. As before, if necessary, a delay may be implemented prior to the next aliquot of drug delivery.

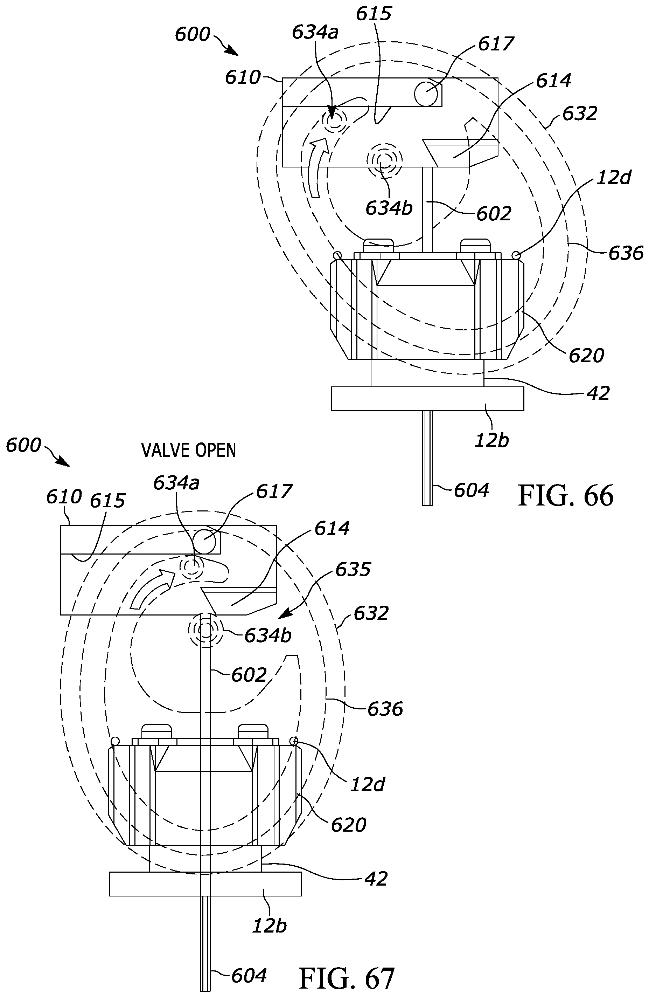

With reference to Figs. 65-67, to again administer the drug 38, the drive member 643 is again energized to trigger the next rotational escapement increment of the spindle 632. The back tab 644b of the linear escapement slide 644 again disengages from the catch 638 of the first spindle 632, and the first spindle rotates approximately 90°. As illustrated in Fig. 65, initially, the inner surface of the cam track 636 urges the needle yoke pin 617 upwards, due to the discontinuity of the cam track 636 (needed to provide clearance during initial retraction), the needle yoke pin 617 exits the cam track 636 as the first spindle 632 rotates, and the needle yoke 610 is briefly disengaged from contacting the spindle 632. As illustrated in Fig. 66, as the first spindle 632 continues to rotate, the outer drive pin 634a contacts the upper tab 615 of the needle yoke 610 and lifts the needle yoke 610 to the valve open position (Fig. 67). At this time, the drug 38 may be delivered to the user.

Any number of additional aliquots may be administered to the user by continuing to rotate the first spindle 632 to urge the needle yoke 610.

In some examples, to immediately deliver the first aliquot, the needle insertion mechanism 600 may be arranged to move the needle 602 directly to the valve open position after insertion of the cannula 604. Further, the rotational increments used may be different. For example, a 60 rotation between valve positions may be used with a tri-lobed cam design. In some alternatives, a slide valve using an introducer needle with a side port that can be sealed inside the cannula or septum may be used. Further, drug delivery may be triggered by a common escapement mechanism. In some forms, drug delivery may be metered directly by closing the slide valve with this mechanism. In the case of a spring or gas driven drug delivery system, the drug contents and fluid path may be pressurized during the delay period.

In the embodiment of Fig. 68, the needle insertion mechanism 700 is similar to the needle insertion mechanism 600, but here, the force exerted by the spindle 732 to move the needle yoke 710 to the next valve position is in a single direction. Here, a bias spring 719 is incorporated to return the needle yoke 710 to the opposite valve position.

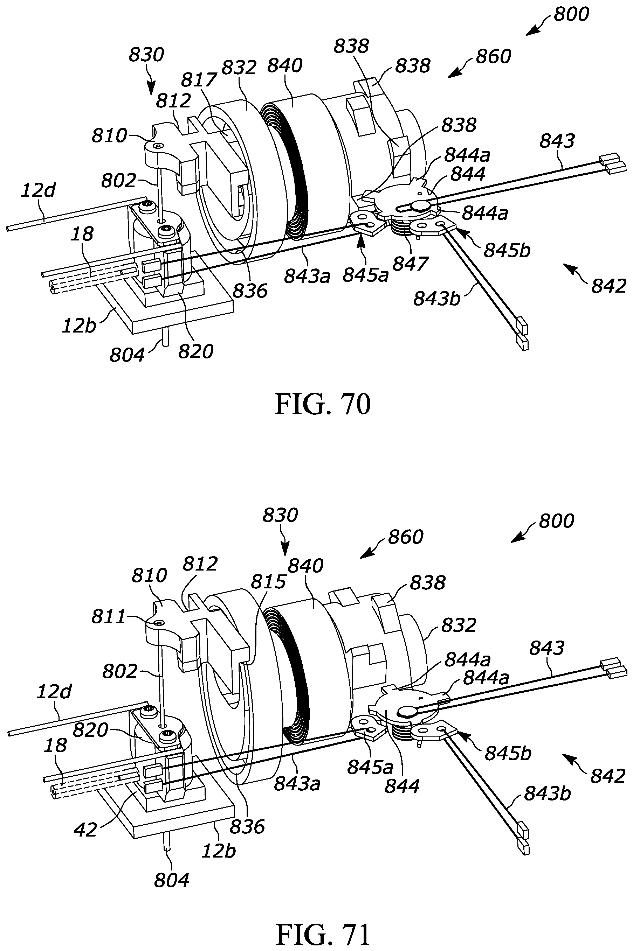

In the embodiment of Figs. 69-72, the needle insertion mechanism 800 is similar to the needle insertion mechanism 600, but here, the escapement assembly 842 is in the form of a rotational escapement member 844 having a number of teeth 844a. The escapement assembly 842 includes a drive member 843 coupled directly with the rotational escapement member 844 as well as number of additional drive members 843a coupled with pivotable pawls 845 that selectively engage the teeth 844a of the rotational escapement member 844. As illustrated in Fig. 70, after the cannula 804 is inserted and the cannula yoke 820 is retained, the needle yoke 810 is moved to the first valve closed position. Here, a catch 838 of the first spindle 832 engages one of the teeth 844a of the rotational escapement member 844 to prevent rotation of the first spindle 832. The drive member 843 is coupled with a pin to rotate the rotational escapement member 844 backwards to reset the escapement by deflecting or "backdriving" a resilient member 847 (in the form of a torsion spring in this example). In addition, each pivotable pawl 845 pivots on a pin member and is urged by a resilient member that deflects when the muscle wire is energized, then subsequently returns the pawl 845 to the starting position when the muscle wire is de-energized.

As illustrated in Fig. 71, the valve is positioned in the first valve open position. The first pawl 845a is pivoted by energizing the drive member 843a coupled therewith, which causes the rotational escapement member 844 to rotate, due to the urging force applied by the resilient member 847. The rotational escapement member 844 continues to rotate until the second pivotable pawl 845b engages a tooth 844a of the rotational escapement member 844. In this position, the spindle 832 is stopped due to a catch 838 engaging a tooth 844a of the rotational escapement member 844.

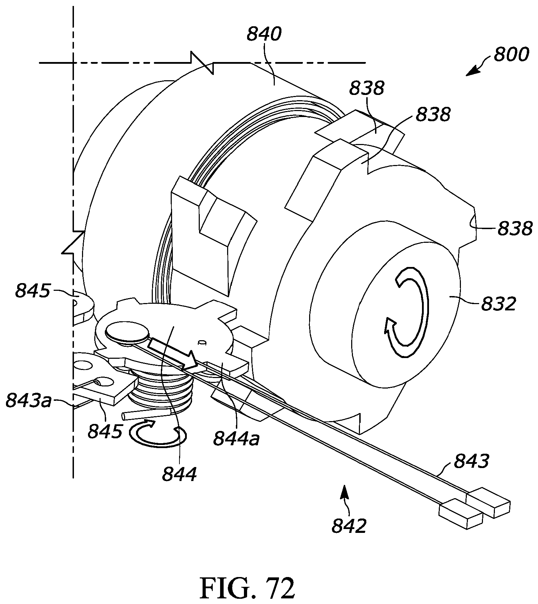

As illustrated in Fig. 72, in this example, the drive member 843 coupled directly with the rotational escapement member 844 may be energized to urge the rotational escapement member 844 in the opposite direction by overcoming the force/torque applied by the resilient member 847. Additional activations of the escapement may be provided in approximately 90° increments. As the drive member 843 energizes, the first spindle 832 is released and again rotates until it catches on the next catch 838 after approximately 45° of rotation. The resilient member 847 returns the rotational escapement member 844, and the spindle 832 may release and catch after rotating another 45°.

In the embodiment of Figs. 73-81, the needle insertion mechanism 900 includes a multi-rotational scotch yoke mechanism design to insert and retract the needle 902. In examples where immediate (i.e., less than approximately 20 seconds) single dose delivery is desired, the needle insertion mechanism 900 can drive the scotch yoke mechanism to only insert the needle 902 into the patient's tissue 11 and retract it once drug delivery is complete. In other examples where multidose delivery is desired, the needle insertion mechanism 900 can be combined with a sliding sleeve valve 960 incorporating a rigid needle 902 and soft cannula 904.

In this example, the first spindle 932 includes first and second catches 936, 938 on an outer surface thereof that engage the escapement assembly 942. Here, the escapement assembly 942 includes a first drive member 943, a second drive member 943a, first and second urging members 944, 944a, first and second fingers 945, 945a, and first and second resilient members 947, 947a. The first and second fingers 945, 945a are configured to selectively engage the first and second catches 936, 938. More specifically, with reference to Fig. 73, in the storage state, the needle 902 and the cannula 904 are both fully retracted. Here, the first finger 945 engages the first catch 936 to restrict movement of the first spindle 932.

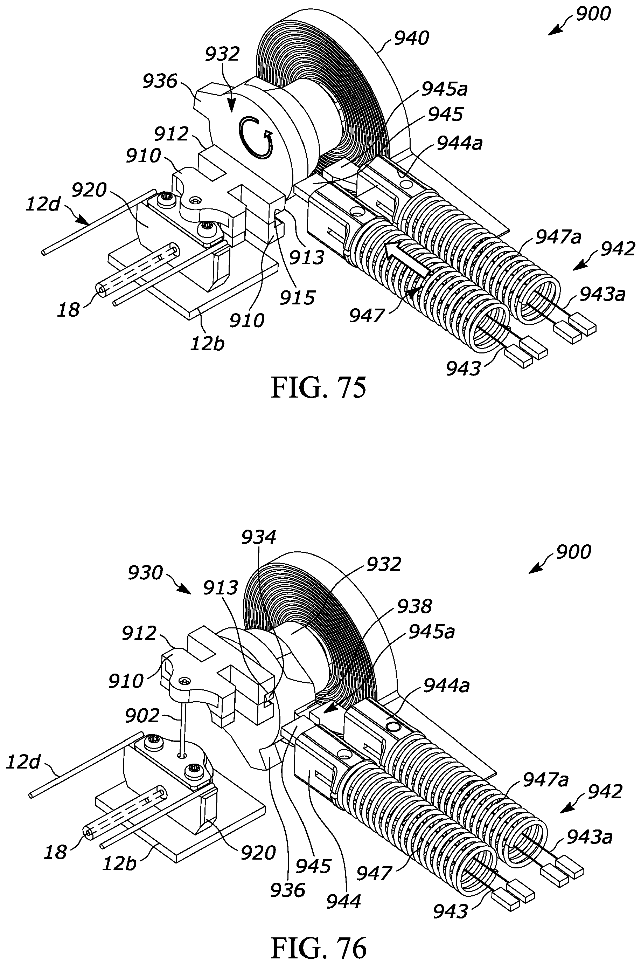

With reference to Fig. 74, upon activation of the device 10, the first finger 945 is disengaged from the first catch 936 by energizing the first drive member 943. The needle yoke 910 and the cannula yoke 920 are driven downward by the drive pin 934. As illustrated in Fig. 75, the needle yoke 910 and the cannula yoke 920 are both fully lowered to a position where the needle and the cannula (not illustrated) are fully extended below the bottom wall 12b of the device 10 as the first spindle 932 rotates. The cannula yoke 920 latches near the bottom of the stroke. Next, the first drive member 943 is de-energized and lengthens, which allows the first resilient member 947 to return the first urging member 944 to the first position.

As illustrated in Fig. 76, the first spindle 932 continues to rotate until the second catch 938 of the first spindle 932 engages the second finger 945a. The first spindle 932 rotates approximately 300° before being stopped. Here, the needle 902 is stopped in the valve closed position where the needle 902 remains within the top of the cannula 904 to retain a seal. If necessary, a delay prior to drug delivery may be implemented. Because the valve 960 is closed, blood and/or other bodily fluids are unable to enter the device fluid path, thereby reducing the likelihood of clogs and/or clots in the fluid path.

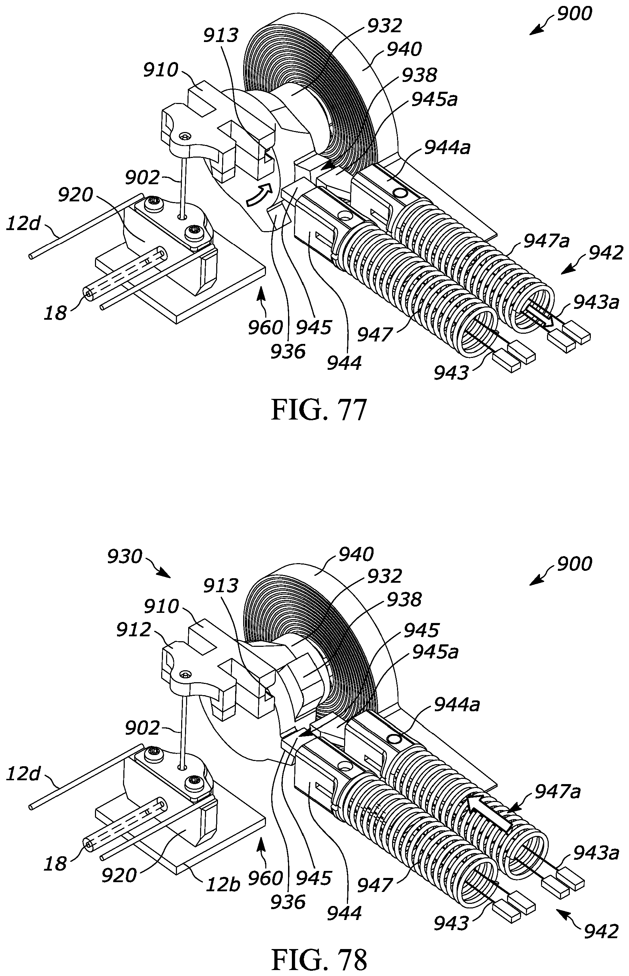

With reference to Fig. 77, to administer the drug 38, the second urging member 943a is energized to contract, thereby moving the second finger 945a of the second urging member 944a out of engagement with the second catch 938 on the first spindle 932. At this time, the spindle 932 rotates, causing the needle yoke 910 to be retracted. The first spindle 932 rotates until the first catch 936 engages the first finger 945 of the first urging member 944, which moves the needle yoke 910 to the valve open position (Fig. 78). Drug delivery may now occur. Further, upon de-energizing, the second drive member 943a lengthens, which allows the second resilient member 947a to urge the second urging member 944a to its original position.

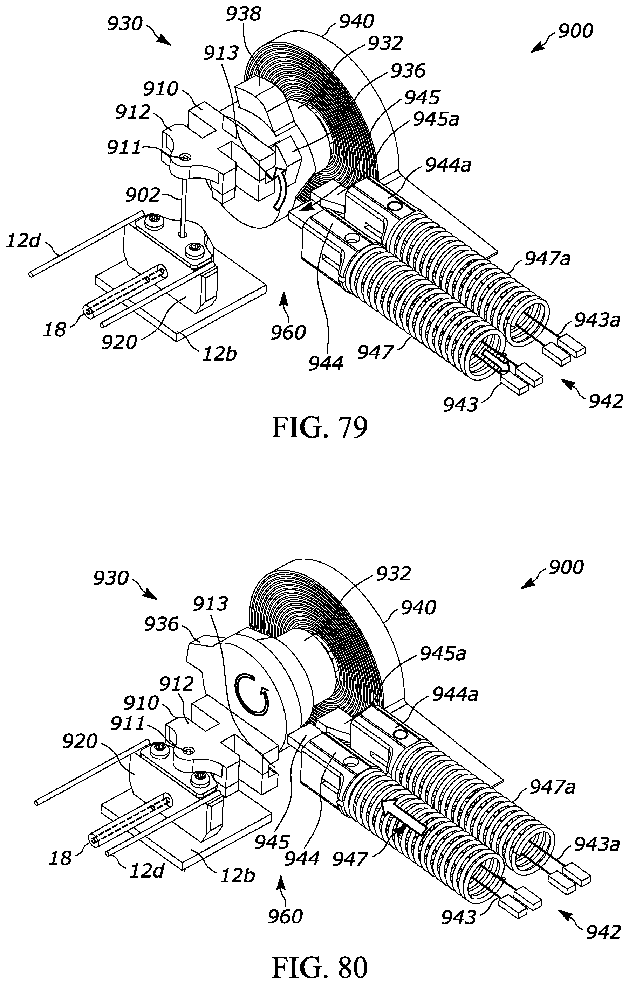

In this design, the resilient member 940 is large enough for multiple rotations of the first spindle 932. To move the needle 902 between valve positions, the first spindle 932 continues rotating in the same direction and the needle yoke 910 repeats its entire stroke. With reference to Figs. 79-81, closing the valve 960 occurs in a similar manner as the initial insertion steps, except that the cannula yoke 920 is already in the lowered position whereby the cannula 904 is already inserted in the patient's tissue 11. The first drive member 943 is energized, thus causing the first finger 945 of the first urging member 944 to be disengaged from the first catch 936. The needle yoke 910 is driven downward by the drive pin 934 of the first spindle 932 and fully reinserts into the cannula (Fig. 80). In this embodiment, the rotation stops when the second catch 938 of the first spindle 932 engages the second finger 945a. Here, the needle 902 is stopped in the valve closed position where the needle 902 remains in the top of the cannula 904 to retain a seal (Fig. 81). Additional doses may be administered by selectively engaging the first and second drive members 943, 943a.

In some examples, other escapement mechanisms for the spindle may be used. Further, an alternative version of the slide valve may be incorporated with this mechanism 900. For example, a slide valve using an introducer needle having a side port that can be sealed inside the cannula or septum may be used. Further, drug delivery may be triggered by an escapement linked to motion of the needle insertion mechanism. In some forms, drug delivery may be metered directly by closing the slide valve with this mechanism. In the case of a spring or gas driven drug delivery system, the drug contents and fluid path may be pressurized during the delay period.

In the embodiment of Figs. 82-94, the needle insertion mechanism 1000 includes a plunger displacement timing mechanism to control insertion and retraction. In examples where immediate (i.e., less than approximately 20 seconds) single dose delivery is desired, the needle insertion mechanism 1000 can drive the scotch yoke mechanism to only insert the needle 1002 into the patient's tissue 11 and retract it once drug delivery is complete. In other examples where multidose delivery is desired, the needle insertion mechanism 1000 can be combined with a sliding sleeve valve incorporating a rigid needle 1002 and soft cannula 1004.

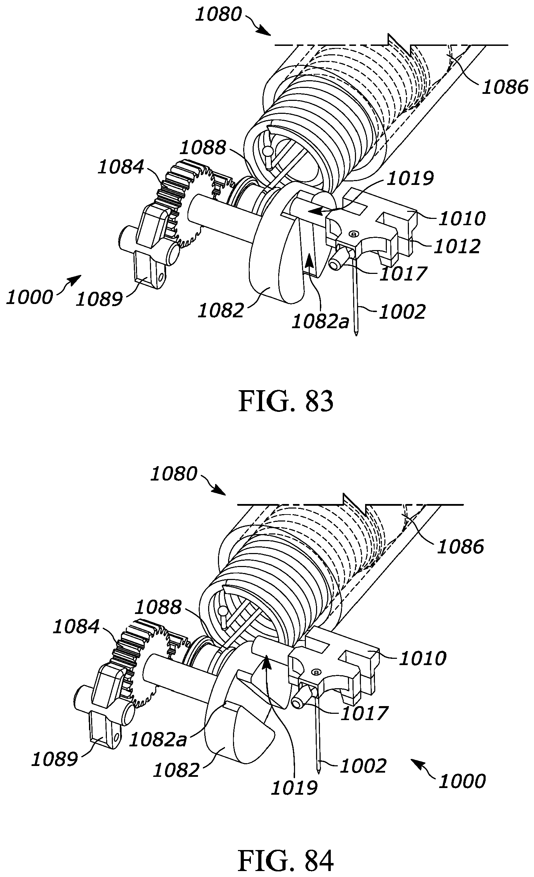

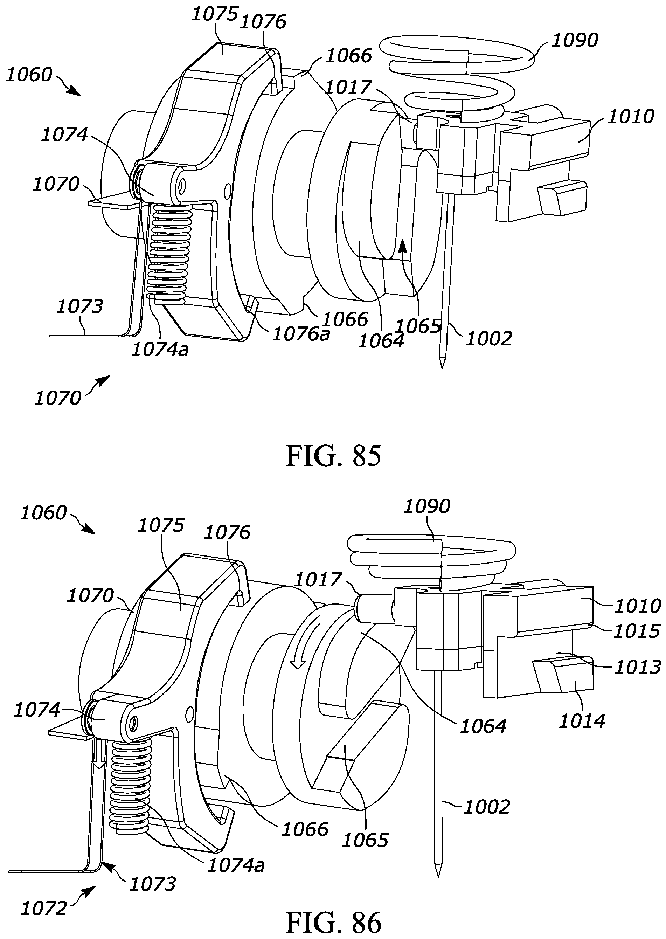

The needle insertion mechanism 1000 includes an insertion and retraction system similar to the needle insertion mechanisms 100 and 500, and further includes a valve lifting assembly similar to the needle insertion mechanism 100. The needle insertion mechanism 1000 further includes a dosing timing assembly 1080 that includes a dosing timing wheel 1082, a gear 1084, a plunger 1086, and a tether 1088 coupled with the plunger 1086. The needle yoke 1010 includes a timing pin 1019 that engages the dosing timing wheel 1082. Generally, the dosing timing wheel 1082 holds the needle 1002 in the valve open position while the drug 38 is dispensed by engaging the timing pin 1019 along an outer surface 1082a of the timing wheel 1082 (Fig. 84). As illustrated in Fig. 83, the needle yoke 1010 drops down into a slot 1082a formed on the dosing timing wheel 1082 after a prescribed time and displacement of the plunger 1086, thereby closing the valve 1060. As illustrated in Figs. 85 and 86, the second spindle 1062 also includes an elongated slot 1065 to accommodate the pin 1017 during the needle/cannula insertion and retraction steps. The second spindle 1062 also includes a track 1064 to lift the pin 1017, and thus the needle yoke 1010, briefly during valve actuation.

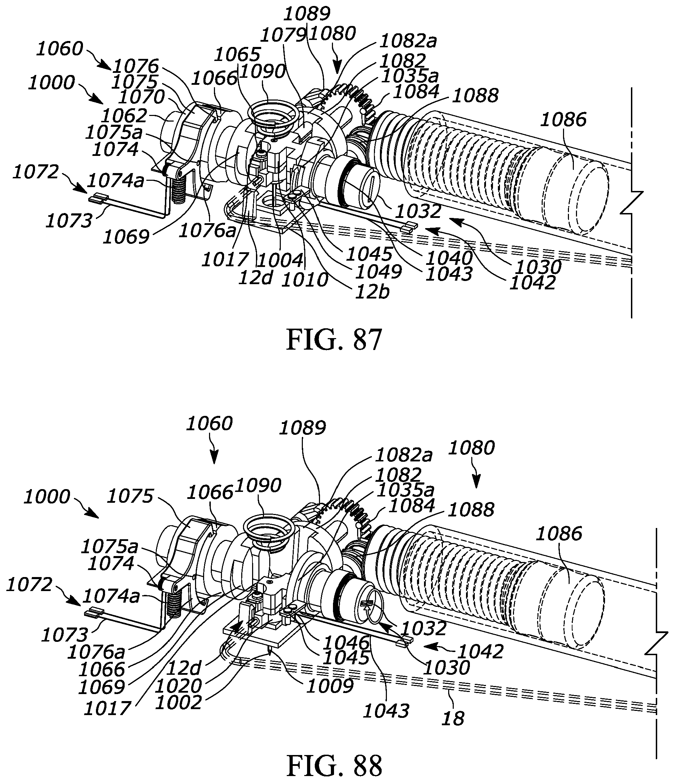

As illustrated in Fig. 82, in the initial storage state, both the needle 1002 and the cannula 1004 are fully retracted. The platform 1046 of the pivotable member 1045 engages the first catch 1035a to prevent the first spindle 1032 from rotating. With reference to Fig. 87, upon activation of the device 10, the pivotable member 1045 is pulled away from the first catch 1035a by the energized driving member 1043. The needle yoke 1010 and the cannula yoke 1020 are driven downward by the first spindle 1032. As illustrated in Fig. 88, continued rotation of the first spindle 1032 causes the needle 1002 and the cannula 1004 to be fully extended. The cannula yoke 1020 latches near the bottom of the stroke. During needle insertion and retraction, the valve assembly 1060 and the dosing timing assembly 1080 must have clearance to the needle yoke 1010.

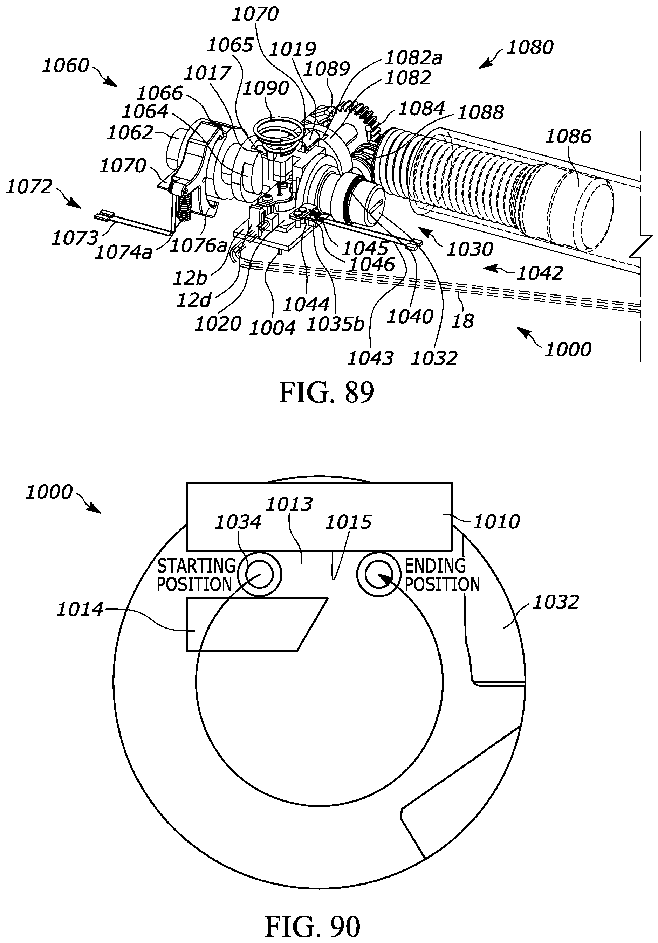

With reference to Figs. 89 and 90, the first spindle 1032 continues to rotate until the second catch 1035b engages the platform 1046 of the pivotable member 1045. The needle 1002 is stopped in the valve closed position where the needle 1002 remains in the top of the cannula 1004 to retain a seal. In this position, the needle yoke 1010 is free to move upwards from the drive pin 1034 on the first spindle 1032 (Fig. 90). If necessary, a delay prior to drug delivery may be implemented. Because the valve 1060 is closed, blood and/or other bodily fluids are unable to enter the device fluid path, reducing the likelihood of clogs and/or clots in the fluid path.

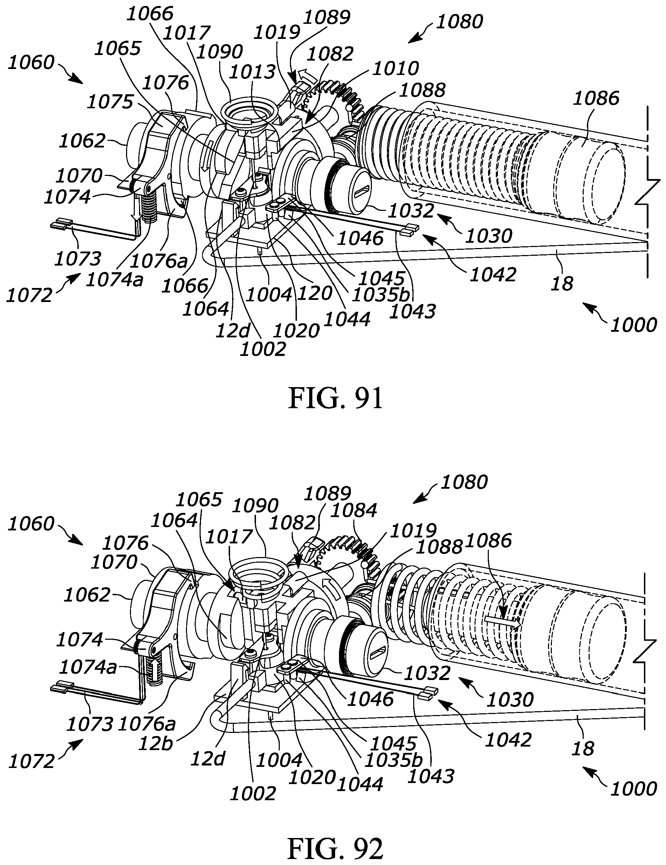

With reference to Fig. 91, the valve assembly 1060 moves the needle yoke 1010 to the valve open position. The valve escapement assembly 1072 selectively engages the second spindle 1062 to allow rotation of approximately 180° per activation. When the driving member 1073 activates, the pivotable member 1075 pivots and releases the second spindle 1062. The second spindle 1062 rotates until the catch 1066 engages the second finger 1076a on the pivotable member 1075. The needle yoke 1010 is lifted to the valve open position when the second finger 1076a of the pivotable member 1075 engages the catch 1066. The driving member 1073 remains energized for a predetermined period of time to enable sufficient time for the dosing timing wheel 1082 to begin rotating and the drug 38 to begin dispensing. As illustrated in Fig. 91, the timing pin 1019 is positioned above the slot 1082a of the dosing timing wheel 1082. Further, a pawl 1089 of the dosing timing assembly 1080 is lifted, thereby pressurizing the drug 38. This pawl may be used to hold the plunger 1086 from pressurizing the drug 38 during long term storage of the device 10. The pawl 1089 is released at the same time as the first valve opening.

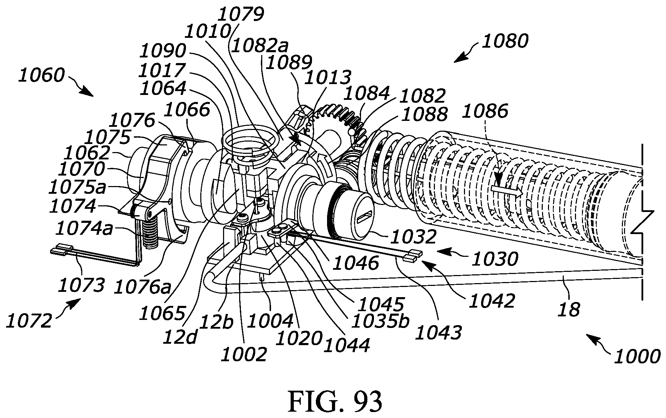

With reference to Figs. 92 and 93, once the valve 1060 opens, the plunger 1086 begins advancing and delivering the drug 38. The tether 1088, coupled with the plunger 1086, rotates the dosing timing wheel 1082 as the plunger 1086 advances. The dosing timing wheel 1082 holds the needle yoke 1010 in the valve open position as it rotates by engaging the timing pin 1019, and the driving member 1073 is de-energized, allowing the resilient member 1074a to urge the pivotable member 1075 back to its first position, thereby releasing the second spindle 1062 to complete its 180° rotation. As illustrated in Fig. 93, the second spindle 1062 leaves clearance for the needle yoke 1010 to return to the valve closed position after the aliquot of drug 38 has been dispensed.

With reference to Fig. 94, the needle 1002 is moved to the valve closed position under power of the resilient member 1090 (in the form of a compression spring in this example) when the timing wheel slot 1082a rotates into vertical position directly below the timing pin 1019. The drug delivery is stopped, and the drug remains under pressure. Additional doses may be administered by repeatedly energizing/de-energizing the driving member 1073.

In some forms, the pawl 1089 may be actuated by the valve assembly 1060. In these examples, the pawl may engage a second timing wheel such that the plunger motion is stopped by the pawl at the same time the valve is closed. If desired, a second timing wheel mechanism may be added that returns the pawl to its starting position after each aliquot has been delivered. This would provide the benefit of removing the static load (generated by the force of the plunger spring) that would otherwise be carried by the pin 1019 to the dosing timing wheel 1082 interface. In some examples, the rotation increments of the various modules may be different. In some alternatives, a slide valve using an introducer needle with a side port that can be sealed inside the cannula or septum may be used. Other orientations or axes of rotation of the valve assembly and dosing timing assembly may be used to move the needle yoke when changing valve states. In some examples, a long plunger rod having notches to control the dosing timing may be used instead of a tether and rotating wheel.

In the embodiment of Figs. 95-104, the needle insertion mechanism 1100 includes a linear slide and cam mechanism to insert and retract the needle 1102. In examples where immediate (i.e., less than approximately 20 seconds) single dose delivery is desired, the needle insertion mechanism 1100 can drive the scotch yoke mechanism to only insert the needle 1102 into the patient's tissue 11 and retract it once drug delivery is complete. In other examples where multidose delivery is desired, the needle insertion mechanism 1100 can be combined with a sliding sleeve valve 1160 incorporating a rigid needle 1102 and soft cannula 1104. After cannula 1104 insertion, the slide valve design allows the device fluid path to be sealed against the ingress of bodily fluids, thus reducing the likelihood of occlusion by blood clots in the fluid path. The mechanism can actuate the sliding valve 1160 multiple times to enable dosing of the drug 38 with the fluid path being protected from ingress of bodily fluids during delays between aliquots.

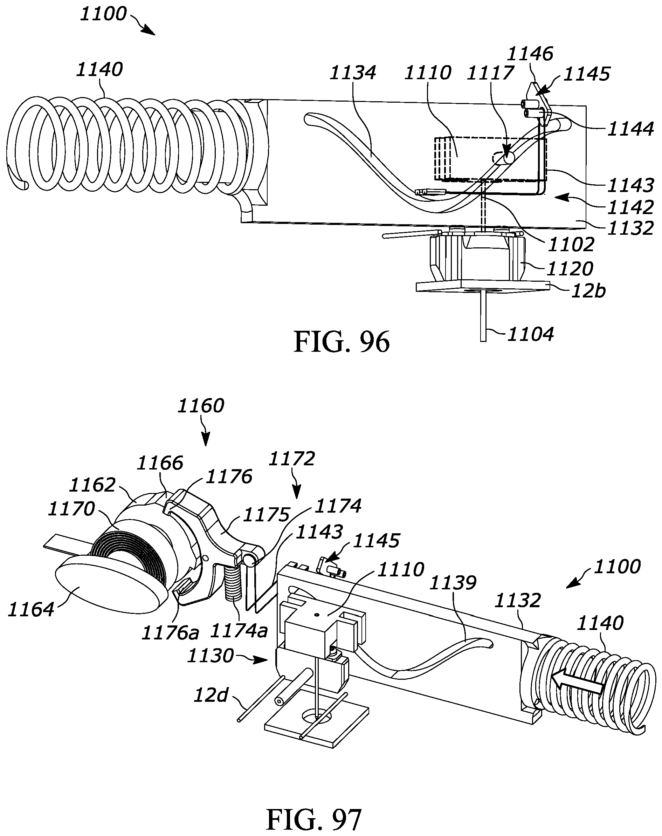

In this example, the needle insertion mechanism 1100 includes a linear slide 1132 having a sliding track 1134 that receives the pin 1117 of the needle yoke 1110. The linear slide 1132 is urged by a resilient member 1140. An actuator escapement assembly 1142 includes a drive member 1143 (Fig. 96), an urging member 1144 in the form of a pin coupled with a pivotable member 1145 having a platform 1146 to engage the linear slide 1132 in a first position. Further a valve assembly 1160 includes a first spindle 1162 having a valve actuation cam 1164 operably coupled and/or integrally formed therewith, a number of catches 1166, and a resilient member 1170. A valve escapement assembly 1172 includes a driving member 1173, an urging member 1174, a resilient member 1174a, a pivotable member 1175 pivotable about a pivot pin 1175a, a first finger 1176, and a second finger 1176a. As illustrated in Fig. 95, in the initial storage state, the needle 1102 and the cannula 1104 are both fully retracted. The platform 1146 engages the linear slide 1132 to prevent movement of the needle yoke 1110.

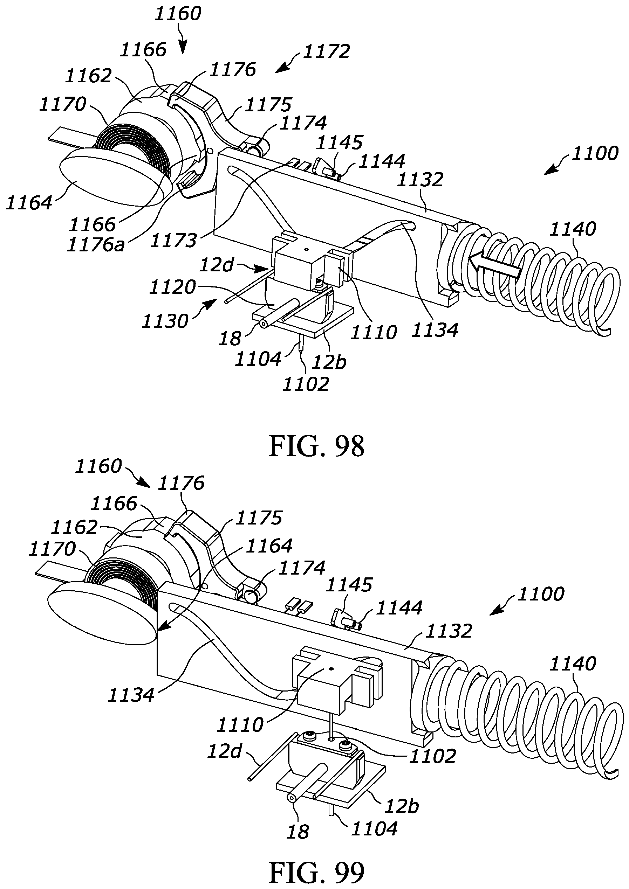

With reference to Figs. 96 and 97, upon activation of the device 10, the pivotable member 1145 is rotated out of engagement with the linear slide 1132 by the energized driving member 1143. The needle yoke 1110 and the cannula yoke 1120 are driven downward upon advancement of the linear slide 1132. More specifically, the pin 1117 traverses the track 1134 and moves to a lowered position. As illustrated in Fig. 98, the needle 1102 and the cannula 1104 are fully extended as the linear slide 1132 travels forward. The cannula yoke 1120 latches to the device floor 12b near the bottom of the stroke.

With reference to Fig. 99, the linear slide 1132 continues to move forward until engaging the valve actuation cam 1164. The needle 1102 is stopped in the valve closed position where the needle remains in the top of the cannula 1104 to retain a seal. If necessary, a delay may be implemented. Because the valve is closed, blood and/or other bodily fluids are unable to enter the device fluid path, thus reducing the likelihood of clogs and/or clots in the fluid path.

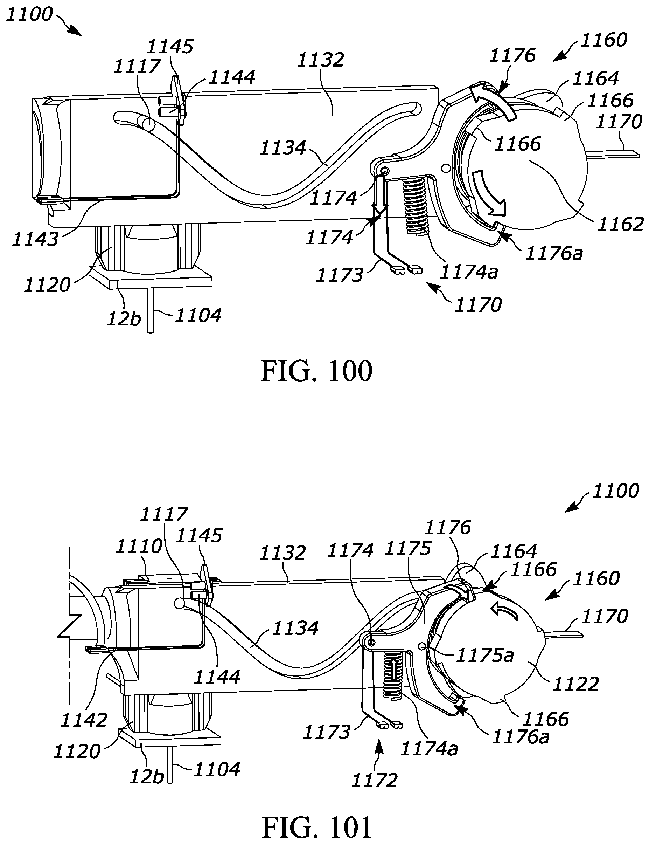

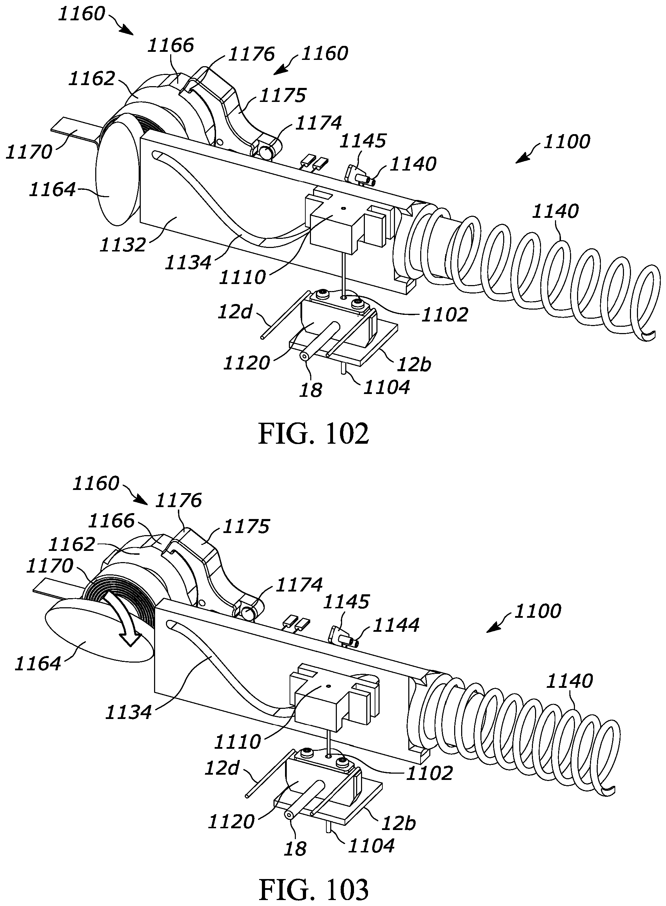

With reference to Figs. 100-102, the valve assembly 1160 is controlled by a rocker or pivotable escapement 1172 to allow approximately 90° of rotation per muscle wire activation. Generally, upon energizing the driving member 1173 the first spindle 1162 and the valve actuation cam 1164 attached therewith rotate approximately 90° to urge the linear slide 1132 forward and lift the needle 1102 to the valve open position (Fig. 102). More specifically, as illustrated in Fig. 100, when energized, the driving member 1173 urges the urging member 1174 to pivot the pivotable member 1175. This pivoting releases the first finger 1176 from engagement with the catch 1166, this allows the resilient member 1170 to rotate the first spindle 1162 approximately 90° until the second finger 1176a engages a second catch 1166 on the first spindle 1062. With reference to Fig. 101, when de-energized the driving member 1173 lengthens, allowing the resilient member 1174a to urge the pivotable member 1175 to return to the first position, and the second finger 1176a disengages from the catch 1166. The first finger 1176 then engages a catch 1166. At this time, the second spindle 1162 has rotated an additional 90° (approximately). As illustrated in Fig. 102, the resulting movement of the linear slide 1132 moves the needle yoke 1110 into the raised, valve open position, whereby drug delivery may occur.

With reference to Fig. 103, the driving member 1173 is again energized, which moves the needle yoke 1110 to the closed position by releasing the first spindle 1162 to rotate, thereby moving the linear slide 1132 . The linear slide 1132 is driven backwards, and the needle 1102 is moved to the valve closed position. Any number of additional aliquots may be delivered by repeatedly energizing/de-energizing the driving member 1173.

In some examples, to immediately deliver the first aliquot, the needle insertion mechanism 1100 may be designed to have the needle 1102 move directly to the valve open position after cannula insertion. Further, in other examples and as illustrated in Fig. 104, the cam may be omitted if the linear slide 1132 is lengthened and the cam track 1134 is shaped to move the needle between the valve open and valve closed positions using motion in a single direction. In this case, the advancement of the linear slide 1132 is controlled by a linear escapement mechanism (not shown).

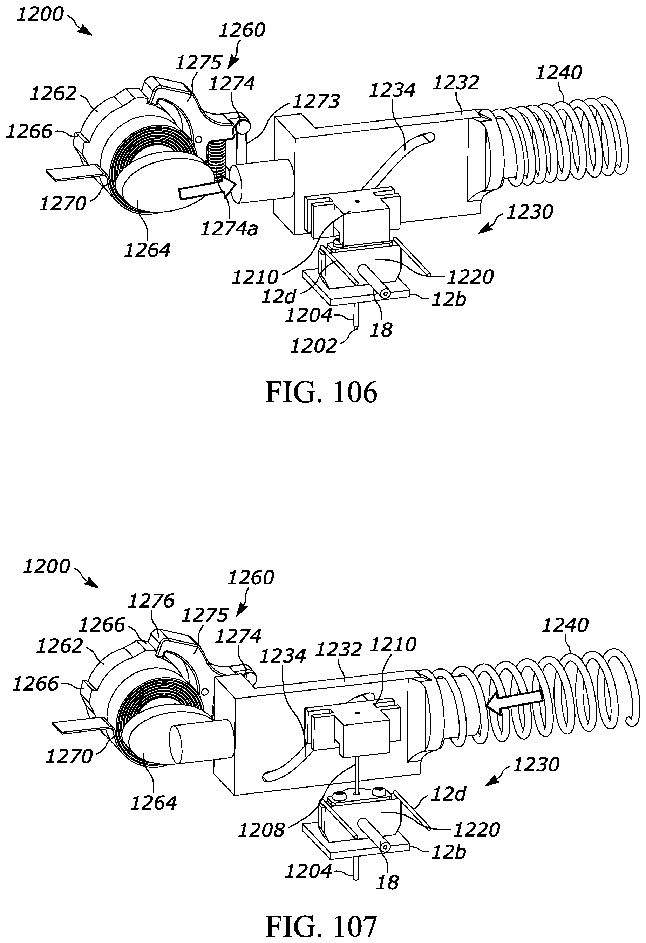

In other examples, and as illustrated in Figs. 105-108, an alternative needle insertion mechanism 1200 includes a linear slide 1232 that may reciprocate for needle insertion and retraction. More specifically, a reciprocating linear inserter may be implemented gas power for insertion and a return spring for retraction. As illustrated in Fig. 106, a gas source (not shown) exerts an axial force to urge the linear slide 1232 to the insertion position. The remaining steps for delivery of additional aliquots may be similar to the needle insertion mechanism 1100.

Other rotational increments of the valve cam may be used. For example, a 60° rotational increment may be used for a tri-lobed cam design. Further, in some forms, other escapement mechanisms may be used. In some examples, an alternative slide valve may be used that includes an introducer needle with a side port that can be sealed inside the cannula or septum. Further, drug delivery may be triggered via an escapement linked with motion of the needle insertion mechanism. In some forms, drug delivery may be metered directly by closing the slide valve with this mechanism. In the case of a spring or gas driven drug delivery system, the drug contents and fluid path may remain pressurized during the delay period.

So configured, the valve designs described herein allow the fluid path of the drug delivery device to be selectively sealed against the ingress of bodily fluids, thus reducing the likelihood of clogs or clots in the fluid path. The valve designs may be used in primed and/or non-primed (air filled) fluid paths because both may be susceptible to clot formation. However, the valve designs may be particularly beneficial for on-body injectors having non-primed (air filled) fluid paths because air is easier to displace than liquids, meaning the bodily fluids have an increased likelihood of flowing in the reverse direction and into the drug delivery device. In both primed and non-primed systems, such backflow of bodily fluids can lead to clot formation, which in turn may reduce the size of the flow path which in turn may require increased forces to urge the drug or medicament through the fluid path to be administered. The needle insertion mechanisms described herein advantageously support multiple dosings, and provides an optimized fluid path for controlled and delayed drug delivery. The valves described herein are advantageously positioned as close as possible to the first end 104a of the inserted cannula 104 in order to minimize the ingress volume of fluids that may potentially enter the fluid path.

In some approaches, any number of alternative components and/or arrangements may be used. For example, in some arrangements, the cannula may be integrated with or may replace the valve body. The septum of the valve may be integrated as a part of a larger flexible boot member that performs additional sealing functions within the device. Further, an open-tip needle without a side port may be used if the septum engagement is sufficiently large. Additionally, other types of needle insertion mechanisms may be used to operate the valve if they support at least three operating actions (i.e., cannula insertion, valve closing, and valve opening. Similarly, other types of mechanisms may be used beyond and/or in place of the muscle wire driven escapement and the Scotch yoke rotatable link mechanism(s).

The above description describes various devices, assemblies, components, and subsystems related to a drug delivery device. The devices, assemblies, components, subsystems, or drug delivery devices can further comprise or be used with a drug including but not limited to those drugs identified below as well as their generic and biosimilar counterparts. The term drug, as used herein, can be used interchangeably with other similar terms and can be used to refer to any type of medicament or therapeutic material including traditional and non-traditional pharmaceuticals, nutraceuticals, supplements, biologics, biologically active agents and compositions, large molecules, biosimilars, bioequivalents, therapeutic antibodies, polypeptides, proteins, small molecules and generics. Non-therapeutic injectable materials are also encompassed. The drug may be in liquid form, a lyophilized form, or in a reconstituted from lyophilized form. The following example list of drugs should not be considered as all-inclusive or limiting.

The drug will be contained in a reservoir. In some instances, the reservoir is a primary container that is either filled or pre-filled for treatment with the drug. The primary container can be a vial, a cartridge or a pre-filled syringe.

In some embodiments, the reservoir of the drug delivery device may be filled with or the device can be used with colony stimulating factors, such as granulocyte colony-stimulating factor (G-CSF). Such G-CSF agents include but are not limited to Neulasta^{®} (pegfilgrastim, pegylated filgastrim, pegylated G-CSF, pegylated hu-Met-G-CSF) and Neupogen^{®} (filgrastim, G-CSF, hu-MetG-CSF), UDENYCA^{®} (pegfilgrastim-cbqv), Ziextenzo^{®} (LA-EP2006; pegfilgrastim-bmez), or FULPHILA (pegfilgrastim-bmez).

In other embodiments, the drug delivery device may contain or be used with an erythropoiesis stimulating agent (ESA), which may be in liquid or lyophilized form. An ESA is any molecule that stimulates erythropoiesis. In some embodiments, an ESA is an erythropoiesis stimulating protein. As used herein, "erythropoiesis stimulating protein" means any protein that directly or indirectly causes activation of the erythropoietin receptor, for example, by binding to and causing dimerization of the receptor. Erythropoiesis stimulating proteins include erythropoietin and variants, analogs, or derivatives thereof that bind to and activate erythropoietin receptor; antibodies that bind to erythropoietin receptor and activate the receptor; or peptides that bind to and activate erythropoietin receptor. Erythropoiesis stimulating proteins include, but are not limited to, Epogen^{®} (epoetin alfa), Aranesp^{®} (darbepoetin alfa), Dynepo^{®} (epoetin delta), Mircera^{®} (methyoxy polyethylene glycol-epoetin beta), Hematide^{®}, MRK-2578, INS-22, Retacrit^{®} (epoetin zeta), Neorecormon^{®} (epoetin beta), Silapo^{®} (epoetin zeta), Binocrit^{®} (epoetin alfa), epoetin alfa Hexal, Abseamed^{®} (epoetin alfa), Ratioepo^{®} (epoetin theta), Eporatio^{®} (epoetin theta), Biopoin^{®} (epoetin theta), epoetin alfa, epoetin beta, epoetin iota, epoetin omega, epoetin delta, epoetin zeta, epoetin theta, and epoetin delta, pegylated erythropoietin, carbamylated erythropoietin, as well as the molecules or variants or analogs thereof.

Among particular illustrative proteins are the specific proteins set forth below, including fusions, fragments, analogs, variants or derivatives thereof: OPGL specific antibodies, peptibodies, related proteins, and the like (also referred to as RANKL specific antibodies, peptibodies and the like), including fully humanized and human OPGL specific antibodies, particularly fully humanized monoclonal antibodies; Myostatin binding proteins, peptibodies, related proteins, and the like, including myostatin specific peptibodies; IL-4 receptor specific antibodies, peptibodies, related proteins, and the like, particularly those that inhibit activities mediated by binding of IL-4 and/or IL-13 to the receptor; Interleukin 1-receptor 1 ("IL1-R1") specific antibodies, peptibodies, related proteins, and the like; Ang2 specific antibodies, peptibodies, related proteins, and the like; NGF specific antibodies, peptibodies, related proteins, and the like; CD22 specific antibodies, peptibodies, related proteins, and the like, particularly human CD22 specific antibodies, such as but not limited to humanized and fully human antibodies, including but not limited to humanized and fully human monoclonal antibodies, particularly including but not limited to human CD22 specific IgG antibodies, such as, a dimer of a human-mouse monoclonal hLL2 gamma-chain disulfide linked to a human-mouse monoclonal hLL2 kappa-chain, for example, the human CD22 specific fully humanized antibody in Epratuzumab, CAS registry number 501423-23-0; IGF-1 receptor specific antibodies, peptibodies, and related proteins, and the like including but not limited to anti-IGF-1R antibodies; B-7 related protein 1 specific antibodies, peptibodies, related proteins and the like ("B7RP-1" and also referring to B7H2, ICOSL, B7h, and CD275), including but not limited to B7RP-specific fully human monoclonal IgG2 antibodies, including but not limited to fully human IgG2 monoclonal antibody that binds an epitope in the first immunoglobulin-like domain of B7RP-1, including but not limited to those that inhibit the interaction of B7RP-1 with its natural receptor, ICOS, on activated T cells; IL-15 specific antibodies, peptibodies, related proteins, and the like, such as, in particular, humanized monoclonal antibodies, including but not limited to HuMax IL-15 antibodies and related proteins, such as, for instance, 145c7; IFN gamma specific antibodies, peptibodies, related proteins and the like, including but not limited to human IFN gamma specific antibodies, and including but not limited to fully human anti-IFN gamma antibodies; TALL-1 specific antibodies, peptibodies, related proteins, and the like, and other TALL specific binding proteins; Parathyroid hormone ("PTH") specific antibodies, peptibodies, related proteins, and the like; Thrombopoietin receptor ("TPO-R") specific antibodies, peptibodies, related proteins, and the like;Hepatocyte growth factor ("HGF") specific antibodies, peptibodies, related proteins, and the like, including those that target the HGF/SF:cMet axis (HGF/SF:c-Met), such as fully human monoclonal antibodies that neutralize hepatocyte growth factor/scatter (HGF/SF); TRAIL-R2 specific antibodies, peptibodies, related proteins and the like; Activin A specific antibodies, peptibodies, proteins, and the like; TGF-beta specific antibodies, peptibodies, related proteins, and the like; Amyloid-beta protein specific antibodies, peptibodies, related proteins, and the like; c-Kit specific antibodies, peptibodies, related proteins, and the like, including but not limited to proteins that bind c-Kit and/or other stem cell factor receptors; OX40L specific antibodies, peptibodies, related proteins, and the like, including but not limited to proteins that bind OX40L and/or other ligands of the OX40 receptor; Activase^{®} (alteplase, tPA); Aranesp^{®} (darbepoetin alfa) Erythropoietin [30-asparagine, 32-threonine, 87-valine, 88-asparagine, 90-threonine], Darbepoetin alfa, novel erythropoiesis stimulating protein (NESP); Epogen^{®} (epoetin alfa, or erythropoietin); GLP-1, Avonex^{®} (interferon beta-1a); Bexxar^{®} (tositumomab, anti-CD22 monoclonal antibody); Betaseron^{®} (interferon-beta); Campath^{®} (alemtuzumab, anti-CD52 monoclonal antibody); Dynepo^{®} (epoetin delta); Velcade^{®} (bortezomib); MLN0002 (anti-α4β7 mAb); MLN1202 (anti-CCR2 chemokine receptor mAb); Enbrel^{®} (etanercept, TNF-receptor /Fc fusion protein, TNF blocker); Eprex^{®} (epoetin alfa); Erbitux^{®} (cetuximab, anti-EGFR / HER1 / c-ErbB-1); Genotropin^{®} (somatropin, Human Growth Hormone); Herceptin^{®} (trastuzumab, anti-HER2/neu (erbB2) receptor mAb); Kanjinti^{™} (trastuzumab-anns) anti-HER2 monoclonal antibody, biosimilar to Herceptin^{®}, or another product containing trastuzumab for the treatment of breast or gastric cancers; Humatrope^{®} (somatropin, Human Growth Hormone); Humira^{®} (adalimumab); Vectibix^{®} (panitumumab), Xgeva^{®} (denosumab), Prolia^{®} (denosumab), Immunoglobulin G2 Human Monoclonal Antibody to RANK Ligand, Enbrel^{®} (etanercept, TNF-receptor /Fc fusion protein, TNF blocker), Nplate^{®} (romiplostim), rilotumumab, ganitumab, conatumumab, brodalumab, insulin in solution; Infergen^{®} (interferon alfacon-1); Natrecor^{®} (nesiritide; recombinant human B-type natriuretic peptide (hBNP); Kineret^{®} (anakinra); Leukine^{®} (sargamostim, rhuGM-CSF); LymphoCide^{®} (epratuzumab, anti-CD22 mAb); Benlysta^{™} (lymphostat B, belimumab, anti-BlyS mAb); Metalyse^{®} (tenecteplase, t-PA analog); Mircera^{®} (methoxy polyethylene glycol-epoetin beta); Mylotarg^{®} (gemtuzumab ozogamicin); Raptiva^{®} (efalizumab); Cimzia^{®} (certolizumab pegol, CDP 870); Soliris^{™} (eculizumab); pexelizumab (anti-C5 complement); Numax^{®} (MEDI-524); Lucentis^{®} (ranibizumab); Panorex^{®} (17-1A, edrecolomab); Trabio^{®} (lerdelimumab); TheraCim hR3 (nimotuzumab); Omnitarg (pertuzumab, 2C4); Osidem^{®} (IDM-1); OvaRex^{®} (B43.13); Nuvion^{®} (visilizumab); cantuzumab mertansine (huC242-DM1); NeoRecormon^{®} (epoetin beta); Neumega^{®} (oprelvekin, human interleukin-11); Orthoclone OKT3^{®} (muromonab-CD3, anti-CD3 monoclonal antibody); Procrit^{®} (epoetin alfa); Remicade^{®} (infliximab, anti-TNFa monoclonal antibody); Reopro^{®} (abciximab, anti-GP IIb/IIia receptor monoclonal antibody); Actemra^{®} (anti-IL6 Receptor mAb); Avastin^{®} (bevacizumab), HuMax-CD4 (zanolimumab); Mvasi^{™} (bevacizumab-awwb); Rituxan^{®} (rituximab, anti-CD20 mAb); Tarceva^{®} (erlotinib); Roferon-A^{®}-(interferon alfa-2a); Simulect^{®} (basiliximab); Prexige^{®} (lumiracoxib); Synagis^{®} (palivizumab); 145c7-CHO (anti-IL15 antibody, see U.S. Patent No. 7,153,507); Tysabri^{®} (natalizumab, anti-α4integrin mAb); Valortim^{®} (MDX-1303, anti-B. anthracis protective antigen mAb); ABthrax^{™}; Xolair^{®} (omalizumab); ETI211 (anti-MRSA mAb); IL-1 trap (the Fc portion of human IgG1 and the extracellular domains of both IL-1 receptor components (the Type I receptor and receptor accessory protein)); VEGF trap (Ig domains of VEGFR1 fused to IgG1 Fc); Zenapax^{®} (daclizumab); Zenapax^{®} (daclizumab, anti-IL-2Rα mAb); Zevalin^{®} (ibritumomab tiuxetan); Zetia^{®} (ezetimibe); Orencia^{®} (atacicept, TACI-Ig); anti-CD80 monoclonal antibody (galiximab); anti-CD23 mAb (lumiliximab); BR2-Fc (huBR3 / huFc fusion protein, soluble BAFF antagonist); CNTO 148 (golimumab, anti-TNFα mAb); HGS-ETR1 (mapatumumab; human anti-TRAIL Receptor-1 mAb); HuMax-CD20 (ocrelizumab, anti-CD20 human mAb); HuMax-EGFR (zalutumumab); M200 (volociximab, anti-α5β1 integrin mAb); MDX-010 (ipilimumab, anti-CTLA-4 mAb and VEGFR-1 (IMC-18F1); anti-BR3 mAb; anti-C. difficile Toxin A and Toxin B C mAbs MDX-066 (CDA-1) and MDX-1388); anti-CD22 dsFv-PE38 conjugates (CAT-3888 and CAT-8015); anti-CD25 mAb (HuMax-TAC); anti-CD3 mAb (NI-0401); adecatumumab; anti-CD30 mAb (MDX-060); MDX-1333 (anti-IFNAR); anti-CD38 mAb (HuMax CD38); anti-CD40L mAb; anti-Cripto mAb; anti-CTGF Idiopathic Pulmonary Fibrosis Phase I Fibrogen (FG-3019); anti-CTLA4 mAb; anti-eotaxin1 mAb (CAT-213); anti-FGF8 mAb; anti-ganglioside GD2 mAb; anti-ganglioside GM2 mAb; anti-GDF-8 human mAb (MYO-029); anti-GM-CSF Receptor mAb (CAM-3001); anti-HepC mAb (HuMax HepC); anti-IFNα mAb (MEDI-545, MDX-198); anti-IGF1R mAb; anti-IGF-1R mAb (HuMax-Inflam); anti-IL12 mAb (ABT-874); anti-IL12/IL23 mAb (CNTO 1275); anti-IL13 mAb (CAT-354); anti-IL2Ra mAb (HuMax-TAC); anti-IL5 Receptor mAb; anti-integrin receptors mAb (MDX-018, CNTO 95); anti-IP10 Ulcerative Colitis mAb (MDX-1100); BMS-66513; anti-Mannose Receptor/hCGβ mAb (MDX-1307); anti-mesothelin dsFv-PE38 conjugate (CAT-5001); anti-PD1mAb (MDX-1106 (ONO-4538)); anti-PDGFRα antibody (IMC-3G3); anti-TGFβ mAb (GC-1008); anti-TRAIL Receptor-2 human mAb (HGS-ETR2); anti-TWEAK mAb; anti-VEGFR/Flt-1 mAb; and anti-ZP3 mAb (HuMax-ZP3).

In some embodiments, the drug delivery device may contain or be used with a sclerostin antibody, such as but not limited to romosozumab, blosozumab, BPS 804 (Novartis), Evenity^{™} (romosozumab-aqqg), another product containing romosozumab for treatment of postmenopausal osteoporosis and/or fracture healing and in other embodiments, a monoclonal antibody (IgG) that binds human Proprotein Convertase Subtilisin/Kexin Type 9 (PCSK9). Such PCSK9 specific antibodies include, but are not limited to, Repatha^{®} (evolocumab) and Praluent^{®} (alirocumab). In other embodiments, the drug delivery device may contain or be used with rilotumumab, bixalomer, trebananib, ganitumab, conatumumab, motesanib diphosphate, brodalumab, vidupiprant or panitumumab. In some embodiments, the reservoir of the drug delivery device may be filled with or the device can be used with IMLYGIC^{®} (talimogene laherparepvec) or another oncolytic HSV for the treatment of melanoma or other cancers including but are not limited to OncoVEXGALV/CD; OrienX010; G207, 1716; NV1020; NV12023; NV1034; and NV1042. In some embodiments, the drug delivery device may contain or be used with endogenous tissue inhibitors of metalloproteinases (TIMPs) such as but not limited to TIMP-3. In some embodiments, the drug delivery device may contain or be used with Aimovig^{®} (erenumab-aooe), anti-human CGRP-R (calcitonin gene-related peptide type 1 receptor) or another product containing erenumab for the treatment of migraine headaches. Antagonistic antibodies for human calcitonin gene-related peptide (CGRP) receptor such as but not limited to erenumab and bispecific antibody molecules that target the CGRP receptor and other headache targets may also be delivered with a drug delivery device of the present disclosure. Additionally, bispecific T cell engager (BiTE^{®}) antibodies such as but not limited to BLINCYTO^{®} (blinatumomab) can be used in or with the drug delivery device of the present disclosure. In some embodiments, the drug delivery device may contain or be used with an APJ large molecule agonist such as but not limited to apelin or analogues thereof. In some embodiments, a therapeutically effective amount of an anti-thymic stromal lymphopoietin (TSLP) or TSLP receptor antibody is used in or with the drug delivery device of the present disclosure. In some embodiments, the drug delivery device may contain or be used with Avsola^{™} (infliximab-axxq), anti-TNF α monoclonal antibody, biosimilar to Remicade^{®} (infliximab) (Janssen Biotech, Inc.) or another product containing infliximab for the treatment of autoimmune diseases. In some embodiments, the drug delivery device may contain or be used with Kyprolis^{®} (carfilzomib), (2S)-N-((S)-1-((S)-4-methyl-1-((R)-2-methyloxiran-2-yl)-1-oxopentan-2-ylcarbamoyl)-2-phenylethyl)-2-((S)-2-(2-morpholinoacetamido)-4-phenylbutanamido)-4-methylpentanamide, or another product containing carfilzomib for the treatment of multiple myeloma. In some embodiments, the drug delivery device may contain or be used with Otezla^{®} (apremilast), N-[2-[(1S)-1-(3-ethoxy-4-methoxyphenyl)-2-(methylsulfonyl)ethyl]-2,3-dihydro-1,3-dioxo- 1H-isoindol-4-yl]acetamide, or another product containing apremilast for the treatment of various inflammatory diseases. In some embodiments, the drug delivery device may contain or be used with Parsabiv^{™} (etelcalcetide HCl, KAI-4169) or another product containing etelcalcetide HCl for the treatment of secondary hyperparathyroidism (sHPT) such as in patients with chronic kidney disease (KD) on hemodialysis. In some embodiments, the drug delivery device may contain or be used with ABP 798 (rituximab), a biosimilar candidate to Rituxan^{®}/MabThera^{™}, or another product containing an anti-CD20 monoclonal antibody. In some embodiments, the drug delivery device may contain or be used with a VEGF antagonist such as a non-antibody VEGF antagonist and/or a VEGF-Trap such as aflibercept (Ig domain 2 from VEGFR1 and Ig domain 3 from VEGFR2, fused to Fc domain of IgG1). In some embodiments, the drug delivery device may contain or be used with ABP 959 (eculizumab), a biosimilar candidate to Soliris^{®}, or another product containing a monoclonal antibody that specifically binds to the complement protein C5. In some embodiments, the drug delivery device may contain or be used with Rozibafusp alfa (formerly AMG 570) is a novel bispecific antibody-peptide conjugate that simultaneously blocks ICOSL and BAFF activity. In some embodiments, the drug delivery device may contain or be used with Omecamtiv mecarbil, a small molecule selective cardiac myosin activator, or myotrope, which directly targets the contractile mechanisms of the heart, or another product containing a small molecule selective cardiac myosin activator. In some embodiments, the drug delivery device may contain or be used with Sotorasib (formerly known as AMG 510), a KRAS^{G12C} small molecule inhibitor, or another product containing a KRAS^{G12C} small molecule inhibitor. In some embodiments, the drug delivery device may contain or be used with Tezepelumab, a human monoclonal antibody that inhibits the action of thymic stromal lymphopoietin (TSLP), or another product containing a human monoclonal antibody that inhibits the action of TSLP. In some embodiments, the drug delivery device may contain or be used with AMG 714, a human monoclonal antibody that binds to Interleukin-15 (IL-15) or another product containing a human monoclonal antibody that binds to Interleukin-15 (IL-15). In some embodiments, the drug delivery device may contain or be used with AMG 890, a small interfering RNA (siRNA) that lowers lipoprotein(a), also known as Lp(a), or another product containing a small interfering RNA (siRNA) that lowers lipoprotein(a). In some embodiments, the drug delivery device may contain or be used with ABP 654 (human IgG1 kappa antibody), a biosimilar candidate to Stelara^{®}, or another product that contains human IgG1 kappa antibody and/or binds to the p40 subunit of human cytokines interleukin (IL)-12 and IL-23. In some embodiments, the drug delivery device may contain or be used with Amjevita^{™} or Amgevita^{™} (formerly ABP 501) (mab anti-TNF human IgG1), a biosimilar candidate to Humira^{®}, or another product that contains human mab anti-TNF human IgG1. In some embodiments, the drug delivery device may contain or be used with AMG 160, or another product that contains a half-life extended (HLE) anti-prostate-specific membrane antigen (PSMA) x anti-CD3 BiTE^{®} (bispecific T cell engager) construct. In some embodiments, the drug delivery device may contain or be used with AMG 119, or another product containing a delta-like ligand 3 (DLL3) CAR T (chimeric antigen receptor T cell) cellular therapy. In some embodiments, the drug delivery device may contain or be used with AMG 119, or another product containing a delta-like ligand 3 (DLL3) CART (chimeric antigen receptor T cell) cellular therapy. In some embodiments, the drug delivery device may contain or be used with AMG 133, or another product containing a gastric inhibitory polypeptide receptor (GIPR) antagonist and GLP-1R agonist. In some embodiments, the drug delivery device may contain or be used with AMG 171 or another product containing a Growth Differential Factor 15 (GDF15) analog. In some embodiments, the drug delivery device may contain or be used with AMG 176 or another product containing a small molecule inhibitor of myeloid cell leukemia 1 (MCL-1). In some embodiments, the drug delivery device may contain or be used with AMG 199 or another product containing a half-life extended (HLE) bispecific T cell engager construct (BiTE^{®}). In some embodiments, the drug delivery device may contain or be used with AMG 256 or another product containing an anti-PD-1 x IL21 mutein and/or an IL-21 receptor agonist designed to selectively turn on the Interleukin 21 (IL-21) pathway in programmed cell death-1 (PD-1) positive cells. In some embodiments, the drug delivery device may contain or be used with AMG 330 or another product containing an anti-CD33 x anti-CD3 BiTE^{®} (bispecific T cell engager) construct. In some embodiments, the drug delivery device may contain or be used with AMG 404 or another product containing a human anti-programmed cell death-1(PD-1) monoclonal antibody being investigated as a treatment for patients with solid tumors. In some embodiments, the drug delivery device may contain or be used with AMG 427 or another product containing a half-life extended (HLE) anti-fms-like tyrosine kinase 3 (FLT3) x anti-CD3 BiTE^{®} (bispecific T cell engager) construct. In some embodiments, the drug delivery device may contain or be used with AMG 430 or another product containing an anti-Jagged-1 monoclonal antibody. In some embodiments, the drug delivery device may contain or be used with AMG 506 or another product containing a multi-specific FAP x 4-1BB-targeting DARPin^{®} biologic under investigation as a treatment for solid tumors. In some embodiments, the drug delivery device may contain or be used with AMG 509 or another product containing a bivalent T-cell engager and is designed using XmAb^{®} 2+1 technology. In some embodiments, the drug delivery device may contain or be used with AMG 562 or another product containing a half-life extended (HLE) CD19 x CD3 BiTE^{®} (bispecific T cell engager) construct. In some embodiments, the drug delivery device may contain or be used with Efavaleukin alfa (formerly AMG 592) or another product containing an IL-2 mutein Fc fusion protein. In some embodiments, the drug delivery device may contain or be used with AMG 596 or another product containing a CD3 x epidermal growth factor receptor vIII (EGFRvIII) BiTE^{®} (bispecific T cell engager) molecule. In some embodiments, the drug delivery device may contain or be used with AMG 673 or another product containing a half-life extended (HLE) anti-CD33 x anti-CD3 BiTE^{®} (bispecific T cell engager) construct. In some embodiments, the drug delivery device may contain or be used with AMG 701 or another product containing a half-life extended (HLE) anti-B-cell maturation antigen (BCMA) x anti-CD3 BiTE^{®} (bispecific T cell engager) construct. In some embodiments, the drug delivery device may contain or be used with AMG 757 or another product containing a half-life extended (HLE) anti- delta-like ligand 3 (DLL3) x anti-CD3 BiTE^{®} (bispecific T cell engager) construct. In some embodiments, the drug delivery device may contain or be used with AMG 910 or another product containing a half-life extended (HLE) epithelial cell tight junction protein claudin 18.2 x CD3 BiTE^{®} (bispecific T cell engager) construct.

Although the drug delivery devices, assemblies, components, and subsystems have been described in terms of exemplary embodiments, they are not limited thereto. The detailed description is to be construed as exemplary only and does not describe every possible embodiment of the present disclosure. Numerous alternative embodiments could be implemented, using either current technology or technology developed after the filing date of this patent that would still fall within the scope of the claims defining the invention(s) disclosed herein.

Those skilled in the art will recognize that a wide variety of modifications, alterations, and combinations can be made with respect to the above described embodiments without departing from the scope of the appended claims, and that such modifications, alterations, and combinations are to be viewed as being within the scope of the invention.

## Claims

1. A drug delivery device (10) comprising:
a housing (12) defining a shell and an inner volume (12a);
a container (30) at least partially disposed within the housing (12), the container (30) having an inner volume (33) to contain a medicament (38);
an activation mechanism (20) at least partially disposed within the housing (12), the activation mechanism (20) adapted to exert a force to urge the medicament (38) out the container (30);
a needle insertion mechanism (100) at least partially disposed within the housing (12), the needle insertion mechanism (100) including:
an actuation assembly (130) adapted to insert a needle (102) and/or a cannula (104) to deliver the medicament (38),
a valve assembly (160),
a needle yoke (110) operably coupled with the actuation assembly (130), the needle yoke (110) including a needle coupling portion (111) to receive a portion of the needle (102), and
a cannula yoke (120) operably coupled with the actuation assembly (130), the cannula yoke (120) including a cannula coupling portion (122) to receive a portion of the cannula;
a fluid flow connection (18) coupled with the container (30) and the needle insertion mechanism (100), the fluid flow connection (18) adapted to allow the medicament (38) to flow from the container (30) to the needle insertion mechanism (100);
**characterized in that** the actuation assembly (130) is configured to repeatedly move the needle (102) between at least a valve open position and a valve closed position to selectively allow and restrict the medicament (38) to flow through the needle (102) and/or the cannula (104) to support multiple dosings.

2. The drug delivery device (10) of claim 1, wherein the actuation assembly (130) comprises a scotch yoke assembly adapted to engage the needle yoke (110) and/or the cannula yoke (120), the scotch yoke assembly including a first spindle (132) operably coupled with the needle yoke (110) and the cannula yoke (120), the first spindle (132) adapted to, in response to a needle insertion input, move at least one of the needle (102) or the cannula (104) to an extended position.

3. The drug delivery device (10) of claim 2, wherein the first spindle (132) comprises a needle insertion mechanism (100) engagement portion comprising a drive pin (134), the needle insertion mechanism (100) engagement portion adapted to engage a portion of the needle yoke (110) and/or the cannula yoke (120), wherein upon rotating the first spindle (132), the needle insertion mechanism (100) engagement portion urges the needle (102) and/or the cannula (104) to the extended position.

4. The drug delivery device (10) of claim 2 or 3, wherein the valve assembly (160) comprises a second spindle (162) operably coupled with the needle yoke (110), the second spindle (162) adapted to, in response to a valve input, move the needle yoke (110) between the valve open position and the valve closed position.

5. The drug delivery device (10) of claim 4, wherein the second spindle (162) comprises a valve engagement portion comprising a track (164), the valve engagement portion adapted to receive a portion of the needle yoke (110), wherein upon rotating the second spindle (162), the valve engagement portion urges the needle (102) between the valve open position and the valve closed position.

6. The drug delivery device (10) of claim 4, wherein the second spindle (162) comprises a valve engagement portion comprising a ramped surface (264), the valve engagement portion adapted to receive a portion of the needle yoke (110), wherein upon rotating the second spindle (162), the valve engagement portion urges the needle (102) between the valve open position and the valve closed position.

7. The drug delivery device (10) of claim 2 or 3, wherein the valve assembly (160) comprises a second spindle (162) operably coupled with the first spindle (132), the second spindle (162) adapted to, in response to a valve input, urge the first spindle (132) to move the needle yoke (110) between the valve open position and the valve closed position,
optionally wherein the second spindle (162) comprises a second spindle gear member adapted to engage a first spindle gear member (336) operably coupled with the first spindle (132), wherein upon rotating the second spindle (162), the second spindle gear member urges the first spindle gear member (336), thereby rotating the first spindle (132) to urge the needle (102) between the valve open position and the valve closed position.

8. The drug delivery device (10) of claim 2 or 3, wherein the valve assembly (160) comprises a rack and pinion assembly operably coupled with the first spindle (132), the rack and pinion assembly adapted to, in response to a valve input, urge the first spindle (132) to move the needle yoke (110) between the valve open position and the valve closed position,
optionally wherein the rack and pinion assembly comprises a pinion (436) operably coupled with the first spindle (132) and an actuation rack being movable in a linear direction, the valve actuation rack having a plurality of stops formed thereon to engage the pinion (436), wherein upon linearly moving the actuation rack, at least one of the plurality of stops engages the pinion (436) to cause the first spindle (132) to rotate and urge the needle (102) between the valve open position and the valve closed position.

9. The drug delivery device (10) of claim 2 or 3, wherein the valve assembly (160) comprises a bi-stable spring assembly (560) operably coupled with the needle yoke (110), the bi-stable spring assembly (560) adapted to, in response to a valve input, move the needle yoke (110) between the valve open position and the valve closed position,
optionally wherein the bi-stable spring assembly (560) comprises:
a bi-stable spring operably coupled with the needle yoke (110) and being movable between a first position and a second position; and
at least one pivotable body (564) being operably coupled with the bi-stable spring and the needle yoke (110);
wherein upon pivoting the pivotable body (564), the pivotable body (564) urges the bi-stable spring between the first position and the second position, thereby moving the needle (102) between the valve open position and the valve closed position.

10. The drug delivery device (10) of claim 2 or 3, wherein the first spindle (132) is further adapted to, in response to a valve input, move the needle yoke (110) between the valve open position and the valve closed position,
optionally wherein the first spindle (132) comprises either:
a) at least one drive pin (134) adapted to engage the needle yoke (110) to move at least one of the needle or the cannula (104) to the extended position and a cam track adapted to engage the needle yoke (110) to move the needle yoke (110) between the valve open and the valve closed position; or
b) at least one drive pin (134) adapted to engage the needle yoke (110) to move at least one of the needle (102) or the cannula (104) to the extended position, the valve open, and valve closed positions.

11. The drug delivery device (10) of claim 2 or 3, wherein the valve assembly (160) comprises a plunger displacement timing assembly (1060) operably coupled with the needle yoke (110), the plunger displacement timing assembly (1060) adapted to, in response to a valve input, move the needle yoke (110) between the valve open position and the valve closed position,
optionally wherein the plunger displacement timing assembly (1060) comprises:
a plunger (1086) including a tether member (1088) operably coupled therewith; and
a rotatable timing wheel (1082) operably coupled with the tether member (1088) and the needle yoke (110);
wherein upon advancement of the plunger (1086), the tether member (1088) causes the rotatable timing wheel (1082) to rotate, thereby moving the needle (102) between the valve open position and the valve closed position.

12. The drug delivery device (10) of claim 1, wherein the actuation assembly (130) comprises a linear slide assembly (1160) adapted to slidingly engage the needle yoke (110) and the cannula yoke (120).

13. The drug delivery device (10) of claim 12, wherein the linear slide assembly (1160) comprises:
a linear slide member operably coupled with the needle yoke (110), the linear slide member adapted to, in response to a needle insertion input, move at least one of the needle (102) or the cannula (104) to an extended position; and
a valve actuation cam (1164) adapted to, in response to a valve input, urge the linear slide member to move the needle yoke (110) between the valve open position and the valve closed position.

14. The drug delivery device (10) of claim 13, wherein the linear slide member comprises a needle insertion mechanism engagement portion comprising a sliding track (1134), the needle insertion mechanism engagement portion adapted to receive a portion of the needle yoke (110), wherein upon linearly urging the linear slide member, the needle insertion mechanism engagement portion urges the needle (102) and/or the cannula (104) to an extended position.

15. The drug delivery device (10) of claim 14, wherein the valve actuation cam (1164) is operably coupled with the linear slide member, wherein upon rotating the valve actuation cam (1164), the cam urges the linear slide member, thereby causing the needle insertion mechanism engagement portion to urge the needle (102) between the valve open position and the valve closed position.

## Patentansprüche

1. Arzneimittelabgabevorrichtung (10), die umfasst:
ein Gehäuse (12), das eine Außenhülle und einen Innenraum (12a) bildet;
einen Behälter (30), der mindestens teilweise innerhalb des Gehäuses (12) angeordnet ist, wobei der Behälter (30) einen Innenraum (33) enthält, der ein Arzneimittel (38) enthält;
einen Auslösemechanismus (20), der mindestens teilweise innerhalb des Gehäuses (12) angeordnet ist, wobei der Auslösemechanismus (20) dafür ausgelegt ist, eine Kraft auszuüben, um das Arzneimittel (38) aus dem Behälter (30) herauszudrücken;
einen Nadel-Einführmechanismus (100), der mindestens teilweise innerhalb des Gehäuses (12) angeordnet ist, wobei der Nadel-Einführmechanismus (100) enthält:
eine Betätigungsanordnung (130), die dafür ausgelegt ist, eine Nadel (102) und/oder eine Kanüle (104) einzuführen, um das Arzneimittel (38) abzugeben,
eine Ventilanordnung (160),
ein Nadeljoch (110), das funktionsmäßig mit der Betätigungsanordnung (130) gekoppelt ist, wobei das Nadeljoch (110) einen Nadelkupplungsabschnitt (111) enthält, der einen Abschnitt der Nadel (102) aufnimmt, und
ein Kanülenjoch (120), das funktionsmäßig mit der Betätigungsanordnung (130) gekoppelt ist, wobei das Kanülenjoch (120) einen Kanülenkopplungsabschnitt (122) enthält, der einen Abschnitt der Kanüle aufnimmt;
eine Fluidströmungsverbindung (18), die mit dem Behälter (30) und dem Nadel-Einführmechanismus (100) gekoppelt ist, wobei die Fluidströmungsverbindung (18) so ausgelegt ist, dass sie den Fluss des Arzneimittels (38) vom Behälter (30) zum Nadel-Einführmechanismus (100) ermöglicht;
**dadurch gekennzeichnet, dass** die Betätigungsanordnung (130) so konfiguriert ist, dass sie die Nadel (102) wiederholt zwischen mindestens einer Ventil-Offenstellung und einer Ventil-Geschlossenstellung bewegt, um den Fluss des Arzneimittels (38) durch die Nadel (102) und/oder die Kanüle (104) optional zuzulassen oder zu unterbinden, um mehrere Dosierungen zu ermöglichen.

2. Arzneimittelabgabevorrichtung (10) nach Anspruch 1, wobei die Betätigungsanordnung (130) eine Scotch-Yoke-Anordnung umfasst, die dafür ausgelegt ist, mit dem Nadeljoch (110) und/oder dem Kanülenjoch (120) in Eingriff zu kommen, wobei die Scotch-Yoke-Anordnung eine erste Spindel (132) enthält, die funktionsfähig mit dem Nadeljoch (110) und dem Kanülenjoch (120) gekoppelt ist, wobei die erste Spindel (132) so ausgelegt ist, dass sie als Reaktion auf einen Befehl zum Einführen der Nadel mindestens entweder die Nadel (102) oder die Kanüle (104) in eine ausgefahrene Position bewegt.

3. Arzneimittelabgabevorrichtung (10) nach Anspruch 2, wobei die erste Spindel (132) einen Eingriffsabschnitt des Nadel-Einführmechanismus (100) umfasst, der einen Antriebsstift (134) umfasst, wobei der Eingriffsabschnitt des Nadel-Einführmechanismus (100) so ausgelegt ist, dass er mit einem Abschnitt des Nadeljochs (110) und/oder des Kanülenjochs (120) in Eingriff kommt, wobei bei Drehung der ersten Spindel (132) der Eingriffsabschnitt des Nadel-Einführmechanismus (100) die Nadel (102) und/oder die Kanüle (104) in die ausgefahrene Position drückt.

4. Arzneimittelabgabevorrichtung (10) nach Anspruch 2 oder 3, wobei die Ventilanordnung (160) eine zweite Spindel (162) umfasst, die funktionsmäßig mit dem Nadeljoch (110) gekoppelt ist, wobei die zweite Spindel (162) so ausgelegt ist, dass sie als Reaktion auf einen Ventileingabeimpuls das Nadeljoch (110) zwischen der Ventil-Offenstellung und der Ventil-Geschlossenstellung bewegt.

5. Arzneimittelabgabevorrichtung (10) nach Anspruch 4, wobei die zweite Spindel (162) einen Ventileingriffsabschnitt umfasst, der eine Führung (164) aufweist, wobei der Ventileingriffsabschnitt dafür ausgelegt ist, einen Abschnitt des Nadeljochs (110) aufzunehmen, wobei bei Drehung der zweiten Spindel (162) der Ventileingriffsabschnitt die Nadel (102) zwischen der Ventil-Öffnungsstellung und der Ventil-Geschlossenstellung drückt.

6. Arzneimittelabgabevorrichtung (10) nach Anspruch 4, wobei die zweite Spindel (162) einen Ventileingriffsabschnitt umfasst, der eine rampenförmige Fläche (264) aufweist, wobei der Ventileingriffsabschnitt so ausgelegt ist, dass er einen Abschnitt des Nadeljochs (110) aufnimmt, wobei bei Drehung der zweiten Spindel (162) der Ventileingriffsabschnitt die Nadel (102) zwischen der Ventil-Öffnungsstellung und der Ventil-Geschlossenstellung drückt.

7. Arzneimittelabgabevorrichtung (10) nach Anspruch 2 oder 3, wobei die Ventilanordnung (160) eine zweite Spindel (162) umfasst, die funktionsmäßig mit der ersten Spindel (132) gekoppelt ist, wobei die zweite Spindel (162) so ausgelegt ist, dass sie als Reaktion auf einen Ventileingabeimpuls die erste Spindel (132) dazu veranlasst, das Nadeljoch (110) zwischen der Ventil-Offenstellung und der Ventil-Geschlossenstellung zu bewegen,
wobei die zweite Spindel (162) optional ein zweites Spindelzahnradelement umfasst, das so ausgelegt ist, dass es mit einem ersten Spindelzahnradelement (336) in Eingriff kommt, das funktionsmäßig mit der ersten Spindel (132) gekoppelt ist, wobei bei Drehung der zweiten Spindel (162) das zweite Spindelzahnradelement das erste Spindelzahnradelement (336) antreibt, wodurch die erste Spindel (132) gedreht wird, um die Nadel (102) zwischen der Ventil-Offenstellung und der Ventil-Geschlossenstellung zu bewegen.

8. Arzneimittelabgabevorrichtung (10) nach Anspruch 2 oder 3, wobei die Ventilanordnung (160) eine Zahnstangen- und Ritzelanordnung umfasst, die funktionsmäßig mit der ersten Spindel (132) gekoppelt ist, wobei die Zahnstangen- und Ritzelanordnung so ausgelegt ist, dass sie als Reaktion auf einen Ventileingabeimpuls die erste Spindel (132) dazu veranlasst, das Nadeljoch (110) zwischen der Ventil-Offenstellung und der Ventil-Geschlossenstellung zu bewegen,
wobei die Zahnstangen- und Ritzelanordnung optional ein Ritzel (436), das funktionsmäßig mit der ersten Spindel (132) gekoppelt ist, und eine in linearer Richtung bewegliche Betätigungszahnstange umfasst, wobei die Ventilbetätigungszahnstange eine Vielzahl von daran gebildeten Anschlägen aufweist, um mit dem Ritzel (436) in Eingriff zu kommen, wobei bei linearer Bewegung der Betätigungszahnstange mindestens einer der Vielzahl von Anschlägen mit dem Ritzel (436) in Eingriff kommt, um die erste Spindel (132) zur Drehung zu veranlassen und die Nadel (102) zwischen der Ventil-Offenstellung und der Ventil-Geschlossenstellung zu bewegen.

9. Arzneimittelabgabevorrichtung (10) nach Anspruch 2 oder 3, wobei die Ventilanordnung (160) eine bistabile Federanordnung (560) umfasst, die funktionsmäßig mit dem Nadeljoch (110) gekoppelt ist, wobei die bistabile Federanordnung (560) so ausgelegt ist, dass sie als Reaktion auf einen Ventileingabeimpuls das Nadeljoch (110) zwischen der Ventil-Offenstellung und der Ventil-Geschlossenstellung bewegt,
optional wobei die bistabile Federanordnung (560) umfasst:
eine bistabile Feder, die funktionsmäßig mit dem Nadeljoch (110) gekoppelt ist und zwischen einer ersten Position und einer zweiten Position bewegbar ist; und
mindestens einen schwenkbaren Körper (564), der funktionsmäßig mit der bistabilen Feder und dem Nadeljoch (110) gekoppelt ist;
wobei beim Schwenken des schwenkbaren Körpers (564) der schwenkbare Körper (564) die bistabile Feder zwischen der ersten Position und der zweiten Position vorspannt, wodurch die Nadel (102) zwischen der Ventil-Offenstellung und der Ventil-Geschlossenstellung bewegt wird.

10. Arzneimittelabgabevorrichtung (10) nach Anspruch 2 oder 3, wobei die erste Spindel (132) ferner so ausgelegt ist, dass sie als Reaktion auf einen Ventileingabeimpuls das Nadeljoch (110) zwischen der Ventil-Offenstellung und der Ventil-Geschlossenstellung bewegt,
optional wobei die erste Spindel (132) umfasst:
a) mindestens einen Antriebsstift (134), der so ausgelegt ist, dass er in das Nadeljoch (110) eingreift, um mindestens entweder die Nadel oder die Kanüle (104) in die ausgefahrene Position zu bewegen, und eine Nockenbahn, die so ausgelegt ist, dass sie in das Nadeljoch (110) eingreift, um das Nadeljoch (110) zwischen der Ventil-Offen- und der Ventil-Geschlossenstellung zu bewegen; oder
b) mindestens einen Antriebsstift (134), der so ausgelegt ist, dass er mit dem Nadeljoch (110) in Eingriff kommt, um entweder die Nadel (102) oder die Kanüle (104) in die ausgefahrene Position sowie in die Ventil-offen- und Ventil-Geschlossenstellung zu bewegen.

11. Arzneimittelabgabevorrichtung (10) nach Anspruch 2 oder 3, wobei die Ventilanordnung (160) eine Kolbenverschiebungs-Zeitsteuerungsanordnung (1060) umfasst, die funktionsmäßig mit dem Nadeljoch (110) gekoppelt ist, wobei die Kolbenverschiebungs-Zeitsteuerungsanordnung (1060) so ausgelegt ist, dass sie als Reaktion auf einen Ventileingabeimpuls das Nadeljoch (110) zwischen der Ventil-Offenstellung und der Ventil-Geschlossenstellung bewegt,
optional wobei die Kolbenverschiebungs-Zeitsteuerungsanordnung (1060) umfasst:
einen Kolben (1086), der ein damit funktionsmäßig gekoppeltes Verbindungselement (1088) enthält; und
ein drehbares Steuerrad (1082), das funktionsmäßig mit dem Verbindungselement (1088) und dem Nadeljoch (110) gekoppelt ist;
wobei bei Vorwärtsbewegung des Kolbens (1086) das Verbindungselement (1088) bewirkt, dass sich das drehbare Steuerrad (1082) dreht, wodurch die Nadel (102) zwischen der Ventil-Offenstellung und der Ventil-Geschlossenstellung bewegt wird.

12. Arzneimittelabgabevorrichtung (10) nach Anspruch 1, wobei die Betätigungsanordnung (130) eine lineare Gleitanordnung (1160) umfasst, die dafür ausgelegt ist, gleitend mit dem Nadeljoch (110) und dem Kanülenjoch (120) in Eingriff zu kommen.

13. Arzneimittelabgabevorrichtung (10) nach Anspruch 12, wobei die lineare Gleitanordnung (1160) umfasst:
ein lineares Gleitelement, das funktionsfähig mit dem Nadeljoch (110) gekoppelt ist, wobei das lineare Gleitelement so ausgelegt ist, dass es als Reaktion auf einen Befehl zum Einführen der Nadel mindestens entweder die Nadel (102) oder die Kanüle (104) in eine ausgefahrene Position bewegt; und
eine Ventilbetätigungsnocke (1164), die so ausgelegt ist, dass sie als Reaktion auf einen Ventileingabeimpuls das lineare Gleitelement dazu veranlasst, das Nadeljoch (110) zwischen der Ventil-Offenstellung und der Ventil-Geschlossenstellung zu bewegen.

14. Arzneimittelabgabevorrichtung (10) nach Anspruch 13, wobei das lineare Gleitelement einen Eingriffsabschnitt für den Nadel-Einführmechanismus umfasst, der eine Gleitschiene (1134) umfasst, wobei der Eingriffsabschnitt für den Nadel-Einführmechanismus dafür ausgelegt ist, einen Abschnitt des Nadeljochs (110) aufzunehmen, wobei der Eingriffsabschnitt für den Nadel-Einführmechanismus bei linearer Betätigung des linearen Gleitelements die Nadel (102) und/oder die Kanüle (104) in eine ausgefahrene Position drückt.

15. Arzneimittelabgabevorrichtung (10) nach Anspruch 14, wobei die Ventilbetätigungsnocke (1164) funktionsmäßig mit dem linearen Gleitelement gekoppelt ist, wobei die Nocke bei Drehung der Ventilbetätigungsnocke (1164) das lineare Gleitelement drückt, wodurch der Eingriffsabschnitt für den Nadel-Einführmechanismus die Nadel (102) zwischen der Ventil-Offenstellung und der Ventil-Geschlossenstellung drückt.

## Revendications

1. Dispositif d'administration de médicament (10), comprenant :
un boîtier (12) définissant une enveloppe et un volume intérieur (12a) ;
un contenant (30) au moins partiellement disposé à l'intérieur du boîtier (12), le contenant (30) ayant un volume intérieur (33) pour contenir un médicament (38) ;
un mécanisme d'activation (20) au moins partiellement disposé à l'intérieur du boîtier (12), le mécanisme d'activation (20) étant adapté pour exercer une force pour pousser le médicament (38) hors du contenant (30) ;
un mécanisme d'insertion d'aiguille (100) au moins partiellement disposé à l'intérieur du boîtier (12), le mécanisme d'insertion d'aiguille (100) incluant :
un ensemble d'actionnement (130) adapté pour insérer une aiguille (102) et/ou une canule (104) pour administrer le médicament (38),
un ensemble à soupape (160),
un étrier d'aiguille (110) couplé opérationnellement à l'ensemble d'actionnement (130), l'étrier d'aiguille (110) incluant une partie de couplage d'aiguille (111) destinée à recevoir une partie de l'aiguille (102), et
un étrier de canule (120) couplé opérationnellement à l'ensemble d'actionnement (130), l'étrier de canule (120) incluant une partie de couplage de canule (122) destinée à recevoir une partie de la canule ;
un raccord d'écoulement de fluide (18) couplé au contenant (30) et au mécanisme d'insertion d'aiguille (100), le raccord d'écoulement de fluide (18) étant adapté pour permettre au médicament (38) de s'écouler depuis le contenant (30) jusqu'au mécanisme d'insertion d'aiguille (100) ;
**caractérisé en ce que** l'ensemble d'actionnement (130) est configuré pour mettre en mouvement à plusieurs reprises l'aiguille (102) entre au moins une position ouverte de soupape et une position fermée de soupape pour sélectivement permettre au médicament (38), et empêcher celui-ci, de s'écouler à travers l'aiguille (102) et/ou la canule (104) pour prendre en charge de multiples dosages.

2. Dispositif d'administration de médicament (10) de la revendication 1, dans lequel l'ensemble d'actionnement (130) comprend un ensemble à étrier à bielle-manivelle adapté pour entrer en prise avec l'étrier d'aiguille (110) et/ou l'étrier de canule (120), l'ensemble à étrier à bielle-manivelle incluant une première broche (132) couplée opérationnellement à l'étrier d'aiguille (110) et à l'étrier de canule (120), la première broche (132) étant adaptée pour, en réponse à une entrée d'insertion d'aiguille, mettre en mouvement au moins une de l'aiguille (102) ou de la canule (104) jusqu'à une position étendue.

3. Dispositif d'administration de médicament (10) de la revendication 2, dans lequel la première broche (132) comprend une partie d'entrée en prise du mécanisme d'insertion d'aiguille (100) comprenant une tige d'entraînement (134), la partie d'entrée en prise du mécanisme d'insertion d'aiguille (100) étant adaptée pour entrer en prise avec une partie de l'étrier d'aiguille (110) et/ou de l'étrier de canule (120), dans lequel, lors de la mise en rotation de la première broche (132), la partie d'entrée en prise du mécanisme d'insertion d'aiguille (100) pousse l'aiguille (102) et/ou la canule (104) jusqu'à la position étendue.

4. Dispositif d'administration de médicament (10) de la revendication 2 ou 3, dans lequel l'ensemble à soupape (160) comprend une seconde broche (162) couplée opérationnellement à l'étrier d'aiguille (110), la seconde broche (162) étant adaptée pour, en réponse à une entrée de soupape, mettre en mouvement l'étrier d'aiguille (110) entre la position ouverte de soupape et la position fermée de soupape.

5. Dispositif d'administration de médicament (10) de la revendication 4, dans lequel la seconde broche (162) comprend une partie d'entrée en prise de soupape comprenant une voie (164), la partie d'entrée en prise de soupape étant adaptée pour recevoir une partie de l'étrier d'aiguille (110), dans lequel, lors de la mise en rotation de la seconde broche (162), la partie d'entrée en prise de soupape pousse l'aiguille (102) entre la position ouverte de soupape et la position fermée de soupape.

6. Dispositif d'administration de médicament (10) de la revendication 4, dans lequel la seconde broche (162) comprend une partie d'entrée en prise de soupape comprenant une surface en rampe (264), la partie d'entrée en prise de soupape étant adaptée pour recevoir une partie de l'étrier d'aiguille (110), dans lequel, lors de la mise en rotation de la seconde broche (162), la partie d'entrée en prise de soupape pousse l'aiguille (102) entre la position ouverte de soupape et la position fermée de soupape.

7. Dispositif d'administration de médicament (10) de la revendication 2 ou 3, dans lequel l'ensemble à soupape (160) comprend une seconde broche (162) couplée opérationnellement à la première broche (132), la seconde broche (162) étant adaptée pour, en réponse à une entrée de soupape, pousser la première broche (132) pour mettre en mouvement l'étrier d'aiguille (110) entre la position ouverte de soupape et la position fermée de soupape,
facultativement dans lequel la seconde broche (162) comprend un élément d'engrenage de seconde broche adapté pour entrer en prise avec un élément d'engrenage de première broche (336) couplé opérationnellement à la première broche (132), dans lequel, lors de la mise en rotation de la seconde broche (162), l'élément d'engrenage de seconde broche pousse l'élément d'engrenage de première broche (336), ainsi mettant en rotation la première broche (132) pour pousser l'aiguille (102) entre la position ouverte de soupape et la position fermée de soupape.

8. Dispositif d'administration de médicament (10) de la revendication 2 ou 3, dans lequel l'ensemble à soupape (160) comprend un ensemble à crémaillère et à pignon couplé opérationnellement à la première broche (132), l'ensemble à crémaillère et à pignon étant adapté pour, en réponse à une entrée de soupape, pousser la première broche (132) pour mettre en mouvement l'étrier d'aiguille (110) entre la position ouverte de soupape et la position fermée de soupape,
facultativement dans lequel l'ensemble à crémaillère et à pignon comprend un pignon (436) couplé opérationnellement à la première broche (132) et une crémaillère d'actionnement pouvant être mis en mouvement dans une direction linéaire, la crémaillère d'actionnement de soupape ayant une pluralité d'arrêts formés sur celle-ci pour entrer en prise avec le pignon (436), dans lequel, lors de la mise en mouvement linéaire de la crémaillère d'actionnement, au moins un de la pluralité d'arrêts entre en prise avec le pignon (436) pour amener la première broche (132) à entrer en rotation et pousser l'aiguille (102) entre la position ouverte de soupape et la position fermée de soupape.

9. Dispositif d'administration de médicament (10) de la revendication 2 ou 3, dans lequel l'ensemble à soupape (160) comprend un ensemble à ressort bistable (560) couplé opérationnellement à l'étrier d'aiguille (110), l'ensemble à ressort bistable (560) étant adapté pour, en réponse à une entrée de soupape, mettre en mouvement l'étrier d'aiguille (110) entre la position ouverte de soupape et la position fermée de soupape,
facultativement dans lequel l'ensemble à ressort bistable (560) comprend :
un ressort bistable couplé opérationnellement à l'étrier d'aiguille (110) et pouvant être mis en mouvement entre une première position et une seconde position ; et
au moins un corps pouvant être mis en pivotement (564) couplé opérationnellement au ressort bistable et à l'étrier d'aiguille (110) ;
dans lequel, lors de la mise en pivotement du corps pouvant être mis en pivotement (564), le corps pouvant être mis en pivotement (564) pousse le ressort bistable entre la première position et la seconde position, ainsi mettant en mouvement l'aiguille (102) entre la position ouverte de soupape et la position fermée de soupape.

10. Dispositif d'administration de médicament (10) de la revendication 2 ou 3, dans lequel la première broche (132) est en outre adaptée pour, en réponse à une entrée de soupape, mettre en mouvement l'étrier d'aiguille (110) entre la position ouverte de soupape et la position fermée de soupape,
facultativement dans lequel la première broche (132) comprend soit :
a) au moins une tige d'entraînement (134) adaptée pour entrer en prise avec l'étrier d'aiguille (110) pour mettre en mouvement au moins une de l'aiguille ou de la canule (104) jusqu'à la position étendue et une voie de came adaptée pour entrer en prise avec l'étrier d'aiguille (110) pour mettre en mouvement l'étrier d'aiguille (110) entre la position ouverte de soupape et la position fermée de soupape ; ou
b) au moins une tige d'entraînement (134) adaptée pour entrer en prise avec l'étrier d'aiguille (110) pour mettre en mouvement au moins une de l'aiguille (102) ou de la canule (104) jusqu'à la position étendue, les positions ouverte de soupape, et fermée de soupape.

11. Dispositif d'administration de médicament (10) de la revendication 2 ou 3, dans lequel l'ensemble à soupape (160) comprend un ensemble de synchronisation de déplacement de piston plongeur (1060) couplé opérationnellement à l'étrier d'aiguille (110), l'ensemble de synchronisation de déplacement de piston plongeur (1060) étant adapté pour, en réponse à une entrée de soupape, mettre en mouvement l'étrier d'aiguille (110) entre la position ouverte de soupape et la position fermée de soupape,
facultativement dans lequel l'ensemble de synchronisation de déplacement de piston plongeur (1060) comprend :
un piston plongeur (1086) incluant un élément d'attache (1088) couplé opérationnellement à celui-ci ; et
une roue de synchronisation pouvant être mise en rotation (1082) couplée opérationnellement à l'élément d'attache (1088) et à l'étrier d'aiguille (110) ;
dans lequel, lors de l'avance du piston plongeur (1086), l'élément d'attache (1088) amène la roue de synchronisation pouvant être mise en rotation (1082) à être mise en rotation, ainsi mettant en mouvement l'aiguille (102) entre la position ouverte de soupape et la position fermée de soupape.

12. Dispositif d'administration de médicament (10) de la revendication 1, dans lequel l'ensemble d'actionnement (130) comprend un ensemble à coulisseau linéaire (1160) adapté pour entrer en prise de façon coulissante avec l'étrier d'aiguille (110) et l'étrier de canule (120).

13. Dispositif d'administration de médicament (10) de la revendication 12, dans lequel l'ensemble à coulisseau linéaire (1160) comprend :
un élément coulisseau linéaire couplé opérationnellement à l'étrier d'aiguille (110), l'élément coulisseau linéaire étant adapté pour, en réponse à une entrée d'insertion d'aiguille, mettre en mouvement au moins un de l'aiguille (102) ou de la canule (104) jusqu'à une position étendue ; et
une came d'actionnement de soupape (1164) adaptée pour, en réponse à une entrée de soupape, pousser l'élément coulisseau linéaire pour mettre en mouvement l'étrier d'aiguille (110) entre la position ouverte de soupape et la position fermée de soupape.

14. Dispositif d'administration de médicament (10) de la revendication 13, dans lequel l'élément coulisseau linéaire comprend une partie d'entrée en prise de mécanisme d'insertion d'aiguille comprenant une voie de coulissement (1134), la partie d'entrée en prise de mécanisme d'insertion d'aiguille étant adaptée pour recevoir une partie de l'étrier d'aiguille (110), dans lequel, lors de la poussée linéaire de l'élément coulisseau linéaire, la partie d'entrée en prise de mécanisme d'insertion d'aiguille pousse l'aiguille (102) et/ou la canule (104) jusqu'à une position étendue.

15. Dispositif d'administration de médicament (10) de la revendication 14, dans lequel la came d'actionnement de soupape (1164) est couplée opérationnellement à l'élément coulisseau linéaire, dans lequel, lors de la mise en rotation de la came d'actionnement de soupape (1164), la came pousse l'élément coulisseau linéaire, ainsi amenant la partie d'entrée en prise de mécanisme d'insertion d'aiguille à pousser l'aiguille (102) entre la position ouverte de soupape et la position fermée de soupape.
